(19) Europäisches Patentamt
European Patent Office
Office européen des brevets

(11) EP 4 420 713 A2

(12) EUROPEAN PATENT APPLICATION

(43) Date of publication:
**28.08.2024 Bulletin 2024/35**

(21) Application number: **24172862.5**

(22) Date of filing: **21.01.2021**

(51) International Patent Classification (IPC):
***A61N 1/00*** *(2006.01)*

(52) Cooperative Patent Classification (CPC):
**A61N 1/36103; A61K 9/0024; A61K 47/10; A61K 47/34; A61N 1/36146**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.01.2020 US 202062963992 P**
**06.04.2020 US 202063005890 P**

(62) Document number(s) of the earlier application(s) in accordance with Art. 76 EPC:
**21744391.0 / 4 093 484**

(71) Applicant: **The Penn State Research Foundation University Park, PA 16802 (US)**

(72) Inventors:
- **MANTO, Kristen**
  **Hummelstown, 17036 (US)**
- **ELFAR, John**
  **Hummelstown, 17036 (US)**
- **GUDDADARANGAIAH, Jagadeeshaprasad Mashanipalya**
  **University Park, 16802 (US)**
- **GOVINDAPPA, Prem Kumar**
  **University Park, 16802 (US)**

(74) Representative: **Fish & Richardson P.C.**
**Highlight Business Towers**
**Mies-van-der-Rohe-Straße 8**
**80807 München (DE)**

Remarks:
•This application was filed on 26-04-2024 as a divisional application to the application mentioned under INID code 62.
•Claims filed after the date of filing of the application (Rule 68(4) EPC).

# (54) METHODS AND MATERIALS FOR TREATING NERVE INJURY AND/OR PROMOTING WOUND HEALING

(57) This document relates to methods and materials for treating nerve injuries. For example, thermoresponsive compositions containing 4-aminopyridine (4-AP) and/or one or more derivatives of 4-AP as well as methods for using such thermoresponsive compositions as a delivery system for 4-AP and/or one or more derivatives of 4-AP (*e.g.,* to treat nerve injury) are provided. This document also provides methods and materials for treating a wound (*e.g.,* a skin wound). For example, compositions containing 4-AP and/or one or more derivatives of 4-AP can be administered (*e.g.*, systemically administered) to a mammal having a wound (*e.g.,* a skin wound) to treat the wound (*e.g.,* to promote wound healing).

EP 4 420 713 A2

## Description

### CROSS-REFERENCE TO RELATED APPLICATIONS

This application claims the benefit of U.S. Patent Application Serial No. 62/963,992, filed on January 21, 2020, and claims the benefit of U.S. Patent Application Serial No. 63/005,890, filed on April 6, 2020.

### STATEMENT REGARDING FEDERAL FUNDING

This invention was made with government support under Grant No. W81XWH-16-1-0725 awarded by the U. S. Army/MRMC. The Government has certain rights in the invention.

### BACKGROUND

*1. Technical Field*

This document relates to methods and materials for treating a nerve injury. For example, this document provides thermoresponsive compositions containing 4-aminopyridine (4-AP) and/or one or more derivatives of 4-AP and methods for using such thermoresponsive compositions as a delivery system for 4-AP and/or one or more derivatives of 4-AP (*e.g.,* to treat a nerve injury). This document also provides methods and materials for treating a wound (*e.g.,* a skin wound). For example, compositions containing 4-AP and/or one or more derivatives of 4-AP can be administered (*e.g.,* systemically administered) to a mammal having a wound to treat the wound (*e.g.,* to promote wound healing).

*2. Background Information*

Traumatic peripheral nerve injury (TPNI) represents a major medical problem occurring in approximately 3% of all trauma patients. A TPNI can result in life-long disability, such as loss of motor and/or sensory function, and pain. TPNI is extremely prevalent in the military setting as in combat injuries such as gunshot wounds and blast trauma. TPNI can also a result of nerve crush, stretching, laceration, and acute nerve compression. To date, there are no effective treatment options beyond primary surgical repair which in itself presents various challenges such as a short timeframe to successfully intervene, rare outcomes of complete functional recovery, and costs of surgery and long-term follow-up care. The presence or absence of axonal continuity plays a pivotal role in deciding treatment strategy for TPNI and improving functional recovery. Nerve injuries like laceration have a complete loss of axonal continuity while axonal continuity is retained in crush injuries. Advanced electro-diagnostics and imaging studies are routinely used as primary ways to evaluate axonal continuity. However, electro-diagnostics and imaging studies do not provide any diagnosis within weeks of injury, and thus ‘watchful waiting’ of weeks or months increases chances of poor functional recovery and drastic muscle loss due to TPNI. On the contrary, if the early surgical exploration of nerves was employed to decide axonal continuity, it might result in poor functional recovery in case of crushed nerves. Thus, there is an unmet need for an early diagnostic test/tool to distinguish axonal continuity in the nerves.

### SUMMARY

This document provides methods and materials for assessing and/or treating a nerve injury. For example, this document provides thermoresponsive compositions (*e.g.*, thermoresponsive polymer compositions) containing 4-AP and/or one or more derivatives of 4-AP In some cases, a thermoresponsive composition described herein can be used to assess axonal continuity of an injured nerve (*e.g.,* an injured nerve within a mammal). For example, a thermoresponsive composition described herein can be delivered onto, into, around, and/or near an injured nerve (*e.g.,* a nerve injury site) within a mammal to assess axonal continuity of the injured nerve. In some cases, a thermoresponsive composition described herein can be used as a delivery system for 4-AP and/or one or more derivatives of 4-AP. For example, a thermoresponsive composition described herein can be delivered onto, into, around, and/or near an injured nerve (*e.g.,* a nerve injury site) within a mammal to treat the nerve injury.

As demonstrated herein, 4-AP can be formulated into a thermoresponsive composition containing block poly lactic-co-glycolic acid (PLGA) (*e.g.,* a copolymer of poly lactic acid (PLA) and poly glycolic acid (PGA) such as poly(lactide-co-glycolide)-b-poly(ethylene glycol)-b-poly(lactide-co-glycolide)) and polyethylene glycol (PEG) that is a liquid at room temperature and undergoes a phase transition to form a gel at body temperature (*e.g.*, at about 37°C for a human). Such a thermoresponsive composition can be injected as a liquid onto, into, around, and/or near an injured nerve and can form a hydrogel *in situ* for controlled-release of 4-AP at the injury site to promote remylination, increase axonal area, and/or increase nerve conduction velocity in the injured nerve. Also as demonstrated herein, local administration of a

thermoresponsive (4-AP)-PLGA-PEG composition can restore muscle responses to electrical stimulation within 30 minutes of administration when there is a nerve continuity.

Having the ability to determine the presence or absence of axonal continuity in nerves can allow a physician to more efficiently and/or more accurately assess and/or treat a patient suffering from a TPNI. For example, confirming the presence or absence of axonal continuity in an injured nerve can allow immediate treatment thus maximizing the patient's chances of functional recover, and can avoid potentially damaging surgical explorations. Further, having the ability to deliver 4-AP and/or one or more derivatives of 4-AP directly to a nerve injury site using a temperature-sensitive, controlled-release formulation provides a unique and unrealized opportunity to treat a nerve injury. Local controlled delivery of 4-AP and/or one or more derivatives of 4-AP avoids the potential adverse side effects seen with systemic 4-AP dosing in humans such as seizures. Moreover, local controlled delivery of 4-AP and/or one or more derivatives of 4-AP provides a non-invasive treatment option that can bypass the need for unnecessary surgical intervention and/or daily dosing.

In general, one aspect of this document features thermoresponsive compositions containing: a) a thermoresponsive polymer, and b) 4-AP and/or one or more derivatives of 4-AP. The thermoresponsive composition can be a liquid when below a physiological temperature of a mammal and can be a gel when at or above the physiological temperature of the mammal. The thermoresponsive composition can be a liquid at about 10°C to about 32°C. The thermoresponsive composition can be a gel at about 32°C to about 37°C. The thermoresponsive polymer can be a copolymer. The copolymer can include PLGA and PEG. The PLGA can have a molecular weight of about 200 to about 1900. The PEG can have a molecular weight of about 1500 to about 3000. The copolymer can include from about 3:5 PLGA:PEG to about 9:1 of PLGA:PEG. The thermoresponsive composition can include 4-AP. The thermoresponsive composition can include a derivative of 4-AP. The derivative of 4-AP can be 3,4-diaminopyridine, 3-hydroxy-4-aminopyridine, N-(4-pyridyl)-t-butyl carbamate, N-(4-pyridyl) ethyl carbamate, N-(4-pyridyl) methyl carbamate, or N-(4-pyridyl) isopropyl carbamate. The thermoresponsive composition can include from about 10 nM to about 1 μM of the 4-AP and/or the derivative of 4-AP.

In another aspect, this document features methods for treating a nerve injury within a mammal. The methods can include, or consist essentially of, administering a thermoresponsive composition including a) a thermoresponsive polymer, and b) 4-AP and/or one or more derivatives of 4-AP onto, into, around, and/or near a nerve injury within a mammal. The mammal can be a human. The nerve injury can be a crush injury. The nerve injury can be in a sciatic nerve. The thermoresponsive composition can be a liquid when below a physiological temperature of the mammal and can be a gel when at or above the physiological temperature of the mammal such that the thermoresponsive composition, once administered onto, into, around, and/or near the nerve injury can form a gel *in situ* within the mammal. The gel can release about 0.5 mg/kg to about 10 mg/kg of the 4-AP and/or one or more derivatives of 4-AP. The gel can release the 4-AP and/or one or more derivatives of 4-AP for about 60 seconds to about 4 weeks.

In another aspect, this document features methods for assessing a nerve injury within a mammal. The methods can include, or consist essentially of, (a) administering a thermoresponsive composition including a thermoresponsive polymer and 4-AP and/or one or more derivatives of 4-AP onto, into, around, and/or near a nerve injury within a mammal; (b) administering an electrical stimulation onto, into, around, and/or near the nerve injury within the mammal; (d) detecting the presence of absence of nerve conduction of the nerve injury; (d) classifying the nerve injury as having axonal continuity based at least in part on the presence of the nerve conduction; and (e) classifying the nerve injury as lacking axonal continuity based at least in part on the absence of the nerve conduction. The mammal can be a human. The nerve injury can be in a motor nerve.

This document also provides methods and materials for treating a wound (*e.g.,* a skin wound or an internal wound). For example, compositions containing 4-AP and/or one or more derivatives of 4-AP can be administered (*e.g.*, systemically administered) to a mammal having a wound to treat the wound (*e.g.,* to promote wound healing). As demonstrated herein, systemic delivery of 4-AP can accelerate wound healing in a standard rodent model of wound healing. For example, systemic delivery of 4-AP can result in at least 10 percent (*e.g.,* at least a 20 percent) wound area reduction in three days and at least 50 percent (*e.g.,* at least 60 or 70 percent) reduction in wound area by seven days as compared to control animals not receiving 4-AP.

In one aspect, this document features methods for treating a wound within a mammal. The methods can include, or consist essentially of, administering a composition including 4-AP or one or more derivatives of 4-AP to a mammal having a wound. The mammal can be a human. The wound can be a cutaneous wound. The wound can be an after-shave wound, an abrasion wound, a diabetic wound, a bed sore, a surgical wound, an anastamotic leak, a tendon gap, a muscle defect, a multi-tissue disruption, and an ulceration. The administration can be a systemic administration. The systemic administration can include an intravenous injection.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference

in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

## DESCRIPTION OF THE DRAWINGS

Figure 1 contains graphs showing time-dependent UPLC/MS/MS measurements of serum 4-AP level in rats before and after treatment with 150 μg/kg 4-AP. Red dotted line denotes the human tolerable limit of serum 4-AP (100 ng/ml). Graph is mean ± SEM, *P< 0.05, **P< 0.01, ***P< 0.001 by one-way ANOVA followed by Tukey's multiple comparisons post-hoc test.

Figure 2 contains images showing an exemplary experimental set-up for *in situ* muscle force measurement. Stimulating electrode is placed under the sciatic nerve equipped with a force transducer (FT10, Grass Instrument) and Triceps surae muscle tension is measure by stimulating the sciatic nerve.

Figures 3A - 3H contain graphs showing representative muscle tension tracings of Triceps surae muscles before and after crush injury with or without systemic 4-AP (150μg/kg, intraperitoneal (i.p.)) (Figure 3A) and local 4-AP thermogel (Figure 3B) treatments, n=3. Figure 3C and Figure 3D show the muscle tension as % increase over crush after systemic and local 4-AP treatments, respectively. Bar graphs showing AUC (Figure 3E and Figure 3F) and peak muscle tension (Figure 3G and Figure 3H) before and after systemic or local 4-AP treatment, respectively. Figures E and G are for systemic, and figures F and H are for local 4-AP treatments. Data are represented as mean ± SEM, *P< 0.05, **P< 0.01, ***P< 0.001 by one-way ANOVA followed by Tukey's multiple comparisons post-hoc test.

Figures 4A - 4B contain graphs showing representative muscle tension tracings of Triceps surae muscles after nerve cut injury and 4-AP (150 μg/kg) i.p. treatment (Figure 4A) and AUC derived from muscle tension experiments (Figure 4B), n = 3. Data are represented as mean ± SEM, ***P< 0.001 by one-way ANOVA followed by Tukey's multiple comparisons post-hoc test.

Figures 5A - 5H contain graphs showing representative muscle tension tracings of Triceps surae muscles 3 days after crush injury with or without systemic 4-AP (150 μg/kg, i.p.) (Figure 5A) and local 4-AP thermogel (Figure 5B) treatments, n=3. Figure 5C and Figure 5D show the muscle tension as % increase over crush after systemic and local 4-AP treatments, respectively. Bar graphs showing AUC (Figure 5E, Figure 5F) and peak muscle tension (Figure 5G, Figure 5H) before and after systemic or local 4-AP treatment, respectively. Figure 5E and Figure 5G are for systemic, and Figure 5F and Figure 5H are for local 4-AP treatments. Data are represented as mean ± SEM, *P < 0.05 by one-way ANOVA followed by Tukey's multiple comparisons post-hoc test.

Figure 6 contains images showing *in-vitro* thermogelation. At an ambient temperature of less than 25°C, both polymers are dissolved in solution and the solution can be loaded with drug. Upon warming from less than 25°C to about 30°C a thermogel appearance starts to form. Above 30°C, a high viscosity gel forms.

Figure 7 contains graphs showing that 4-AP can be incorporated in PLGA-PEG.

Figure 8 is a line graph plotting cumulative release of 4-AP from PLGA-PEG thermogel at varying drug concentrations.

Figure 9 contains graphs showing that gelation temperature increases with 4-AP concentration.

Figure 10 contains graphs showing that gelation time increases with 4-AP concentration.

Figure 11 contains graphs showing that solution-gel transition is reversible.

Figure 12 contains images showing that *in vivo* (4-AP)-PLGA-PEG (4-AP)-PLGA-PEG remains on the injury site and degrades for about 21 days.

Figure 13 contains graphs showing that serum 4-AP levels never exceed the human tolerable limit.

Figure 14 contains graphs showing that (4-AP)-PLGA-PEG enhances motor and sensory function after moderate sciatic nerve crush injury. Systemic 4-AP treatment was administered daily for 28 days. Thermogel was placed on the crush site immediately after injury.

Figures 15A - 15F contain images of a full excision wounding procedure. (Figure15A - Figure 15D) Depilation of mouse dorsal skin hair using mechanical shave clipper and hair removed by applying commercially available Nair cream. (Figure 15E and Figure 15F) Creation of full 5 mm diameter excision wound by using biopsy punch on the mouse dorsal skin.

Figures 16A - 16C contain images of wound closure at day 3 after administration of saline (control) or 4-AP. (Figure 16A) 5 mm diameter excision wound at 0 day. Representative wound closure photographs corresponding to saline (Figure 16B) and 4-AP (Figure 16C) treated mice are shown. The diameter/width of the wound area at day 3 was 5 mm for saline treated mice and 4.0 mm for 4-AP treated mice.

Figures 17A - 17B contain images of wound closure at day 7 after administration of saline (control) or 4-AP. Representative wound closure photographs corresponding to saline (Figure 17A) and 4-AP (Figure 17B) treated mice are shown. The diameter/width of the wound area at day 7 was 2.7 mm for saline treated mice and 1.5 mm for 4-AP treated mice.

Figures 18A - 18B contain images of wound closure at day 10 after administration of saline (control) or 4-AP. Representative wound closure photographs corresponding to saline and 4-AP treated mice shown. The diameter/width of the wound area at day 10 was 1.0 for saline treated mice and 0.3 mm for 4-AP treated mice.

Figure 19 shows that 4-AP accelerates wound healing. Wound closure rates were calculated using image J analysis for the wound area at days 3, 7, and 10 after administration of saline (control) or 4-AP to mouse dorsal skin excision wounds.

Figures 20A - 20C. Schematic representation of a (4-AP)-loaded thermogel as a local controlled-release delivery system. (Figure 20A) Chemical structures of 4-AP and PLGA-PEG, respectively. (Figure 20B) Amphiphilic polyester-PEG-polyester triblock copolymers self-assemble into micelles in aqueous solution at room temperature and form a solid gel via crosslinks at higher temperature. (Figure 20C) 4-AP can be incorporated into the triblock copolymer at room temperature and injected into an animal in liquid phase. Once the formulation reaches body temperature, it forms an *in-situ* hydrogel for controlled-release of 4-AP.

Figure 21. $^1$H NMR spectra of PLGA-PEG, 4-AP, and (4-AP)-PLGA-PEG. The chemical shifts of the mixture showed significant change at both aromatic protons of 4-AP from $\delta$8.02 to $\delta$7.94 and $\delta$6.69 to $\delta$6.83, respectively, when mixed with PLGA-PEG. In addition, the PLGA-PEG aliphatic region was changed from $\delta$1.31 to $\delta$1.29 after mixing.

Figures 22A - 22D. Rheological investigation of copolymer aqueous solutions. (Figure 22A) Gelation temperatures of PLGA-PEG and 2$\mu$g/$\mu$L (4-AP)-PLGA-PEG were 31.3°C and 32°C, respectively. (Figure 22B) Gelation temperature increased with increasing 4-AP concentration. Figure 22C) Solution to gel (sol-gel) transition occurred in 19 seconds. (Figure 22D) Sol-gel transition was fully reversible and occurred in 1228 seconds.

Figure 23. Cumulative *in-vitro* release of 4-AP from PLGA-PEG in PBS (pH 7.4) at 37°C. PLGA-PEG carriers exhibited a burst release of 4-AP within one day followed by release of a low maintenance dose for approximately 28 days. Cumulative release was proportional to total loaded amount of 4-AP.

Figures 24A - 24B. Pharmacokinetic study of 4-AP serum levels in mice after (4-AP)-PLGA-PEG administration. (Figure 24A) 4-AP serum levels peaked one hour after administration and never exceeded the human tolerable limit of 100 ng/mL. (Figure 24B) 4-AP serum levels were nearly undetectable by Day 21.

Figures 25A - 25D. (4-AP)-PLGA-PEG injection on the mouse sciatic nerve using small animal ultrasonography. (Figure 25A) Longitudinal visualization of the sciatic nerve using the Vevo 3100 40 MHz ultrasound probe. (Figure 25B) Identification of the 20 G needle with the nerve. (Figure 25C) Positioning the needle over the sciatic nerve pre-injection. (Figure 25D) Visualization of (4-AP)-PLGA-PEG on the sciatic nerve post-injection.

Figure 26. *In vivo* biodegradation study of (4-AP)-PLGA-PEG on the sciatic nerve. Upon administration on the sciatic nerve, the (4-AP)-PLGA-PEG turns opaque indicating thermogelation. The gel remained directly on the sciatic nerve injury site for over 21 days, although its overall mass decreased due to controlled polymeric degradation.

Figures 27A - 27C. Effects of (4-AP)-PLGA-PEG on motor and sensory functional outcomes. (Figure 27A) (4-AP)-PLGA-PEG improved post-injury functional recovery (SFI) on days 1, 3, 7, and 14 (*$p < 0.05$) compared to saline, PLGA-PEG, and systemic 4-AP groups. (Figure 27B) (4-AP)-PLGA-PEG improved grip strength on days 3, 7, 14, 21, and 28 post-injury compared to all other treatment groups (*$p < 0.05$). (Figure 27C) (4-AP)-PLGA-PEG significantly improved withdrawal reflex (percent response to filament) as compared to the saline group (*$p < 0.05$) on post-injury days 1, 3, 7, 14 and 21.

Figures 28A - 28C. (4-AP)-PLGA-PEG enhanced nerve remyelination after traumatic sciatic nerve crush injury. (Figure 28A) Immunofluorescence of NF-H and MPZ in (4-AP)-PLGA-PEG-treated nerves compared to saline-treated nerves on post-injury day 28. (Figures 28B - 28C) (4-AP)-PLGA-PEG treated nerves had significantly greater fluorescence intensities of NF-H (Figure 28B) and MPZ (Figure 28C), two major myelination markers, compared to saline 28 days post-injury.

## DETAILED DESCRIPTION

This document provides methods and materials for assessing and/or treating nerve injuries. For example, this document provides thermoresponsive compositions (*e.g.*, thermoresponsive polymer compositions) containing 4-AP and/or one or more derivatives of 4-AP. In some cases, a thermoresponsive composition described herein can be used as a delivery system for 4-AP and/or one or more derivatives of 4-AP. For example, a thermoresponsive composition described herein (*e.g.,* a thermoresponsive polymer composition containing 4-AP and/or one or more derivatives of 4-AP) can be delivered onto, into, around, and/or near an injured nerve (*e.g.,* a nerve injury site) within a mammal to assess axonal continuity of the injured nerve. For example, a thermoresponsive composition described herein (*e.g.,* a thermoresponsive polymer composition containing 4-AP and/or one or more derivatives of 4-AP) can be delivered onto, into, around, and/or near an injured nerve (*e.g.,* a nerve injury site) within a mammal to treat the nerve injury. In another

aspect, this document provides compositions containing 4-AP and/or one or more derivatives of 4-AP and methods for using such compositions. For example, compositions containing 4-AP and/or one or more derivatives of 4-AP can be administered (*e.g.*, systemically administered) to a mammal having a wound (*e.g.,* a skin wound or an internal wound) to treat the wound (*e.g.,* to promote wound healing).

A thermoresponsive composition described herein (*e.g.,* a thermoresponsive polymer composition containing 4-AP and/or one or more derivatives of 4-AP) can be a three-dimensional network of polymeric chains that can change phases (*e.g.*, from a liquid phase to a gel phase or *vice versa)* as the temperature changes. In some cases, a thermoresponsive composition can be a liquid at a lower temperature such as an ambient temperature (*e.g.*, at room temperature such as about 22°C) and can transition to a gel at a higher temperature such as a physiological temperature (*e.g.*, at body temperature such as 37°C). For example, a thermoresponsive composition can be a liquid at a temperature of from about 10°C to about 32°C (*e.g.,* from about 10°C to about 30°C, from about 10°C to about 25°C, from about 10°C to about 20°C, from about 15°C to about 32°C, from about 18°C to about 32°C, from about 20°C to about 32°C, from about 12°C to about 30°C, from about 15°C to about 25°C, or from about 20°C to about 22°C) and can be a gel at a temperature of from about 32°C to about 37°C (*e.g.,* from about 33°C to about 37°C, from about 34°C to about 37°C, from about 35°C to about 37°C, from about 32°C to about 36°C, from about 32°C to about 35°C, from about 32°C to about 34°C, or from about 33°C to about 35°C). In some cases, the phase transition of a thermoresponsive composition described herein can be reversible. In some cases, the phase transition of a thermoresponsive composition described herein can be irreversible. The temperature at or below which a thermoresponsive composition can be maintained in a liquid phase can be referred to as the lower critical solution temperature (LCST). A thermoresponsive composition described herein *(e.g.,* a thermoresponsive polymer composition containing 4-AP and/or one or more derivatives of 4-AP) can have any appropriate LCST. In some cases, a LCST of a thermoresponsive composition described herein can be an ambient temperature (*e.g.*, room temperature). For example, a LCST of a thermoresponsive composition described herein can be from about 3°C to about 32°C (*e.g.,* from about 3°C to about 28°C, from about 3°C to about 25°C, from about 3°C to about 22°C, from about 3°C to about 20°C, from about 3°C to about 15°C, from about 3°C to about 10°C, from about 3°C to about 5°C, from about 5°C to about 32°C, from about 10°C to about 32°C, from about 15°C to about 32°C, from about 20°C to about 32°C, from about 22°C to about 32°C, from about 25°C to about 32°C, from about 28°C to about 32°C, from about 5°C to about 25°C, from about 15°C to about 20°C, from about 5°C to about 10°C, from about 10°C to about 15°C, from about 15°C to about 20°C, from about 20°C to about 25°C, or from about 25°C to about 30°C). The temperature at which a thermoresponsive composition can change phases (*e.g.,* from a liquid phase to a gel phase or *vice versa*) can be referred to as the transition temperature. A thermoresponsive composition described herein (*e.g.,* a thermoresponsive polymer composition containing 4-AP and/or one or more derivatives of 4-AP) can have any appropriate transition temperature. In some cases, a transition temperature of a thermoresponsive composition described herein can be a physiological temperature (*e.g.*, body temperature of a mammal). For example, a transition temperature of a thermoresponsive composition described herein can be from about 32°C to about 38°C (*e.g.,* at about 32°C, at about 33°C, at about 34°C, at about 35°C, at about 36°C, at about 37°C, at about 38°C). The LCST and/or the transition temperature of a thermoresponsive composition described herein can be affected by many structural parameters of the thermoresponsive composition such as the hydrophobic content, architecture of the thermoresponsive composition, molar mass of the thermoresponsive composition, the relative ratio of hydrophobic to hydrophilic components in the thermoresponsive composition, and any combinations thereof.

In some cases, a thermoresponsive composition described herein (*e.g.,* a thermoresponsive polymer composition containing 4-AP and/or one or more derivatives of 4-AP) can be biodegradable. For example, a thermoresponsive composition described herein can be decomposed by a living organism (*e.g.*, by a biological process).

In some cases, a thermoresponsive composition described herein (*e.g.,* a thermoresponsive polymer composition containing 4-AP and/or one or more derivatives of 4-AP) can be non-biodegradable.

In some cases, a thermoresponsive composition described herein (*e.g.,* a thermoresponsive polymer composition containing 4-AP and/or one or more derivatives of 4-AP) can be biocompatible. For example, a thermoresponsive composition described herein can produce minimal to no adverse effects (*e.g.*, toxicity, irritation, allergies, and/or rejection) when administered to a mammal (*e.g.,* a human).

A thermoresponsive composition described herein (*e.g.,* a thermoresponsive polymer composition containing 4-AP and/or one or more derivatives of 4-AP) or a composition described herein (*e.g.,* a composition containing 4-AP and/or one or more derivatives of 4-AP) can include 4-AP and/or any appropriate derivative(s) of 4-AP. When a thermoresponsive composition described herein includes one or more derivatives of 4-AP, the derivative of 4-AP can be any 4-AP derivative of 4-AP. Examples of derivatives of 4-AP that can be included in a thermoresponsive composition described herein include, without limitation, 3,4-diaminopyridine, 3-hydroxy-4-aminopyridine, N-(4-pyridyl)-t-butyl carbamate, N-(4-pyridyl) ethyl carbamate, N-(4-pyridyl) methyl carbamate, and N-(4-pyridyl) isopropyl carbamate. In some cases, 4-AP and/or one or more derivatives of 4-AP can have a structure according to Formula I:

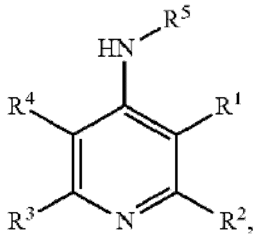

where R1, R2, R3, R4, and R5 are each independently selected from hydrogen, halogen, amine, hydroxyl, alkoxy, carboxyl, or C1 -C6 alkyl. For example, R1, R2, R3, R4, and R5 can all be hydrogen. In some cases, 4-AP or a derivative thereof can be a potassium channel blocker. In some cases, 4-AP or a derivative thereof can be a calcium channel agonist. In some cases, 4-AP or a derivative thereof can be electrically active. In some cases, 4-AP or a derivative thereof can be in the form of a free base. In some cases, or a derivative thereof can be in the form of a salt (*e.g.,* pharmaceutically acceptable salt). When or a derivative thereof is in the form of a salt, the salt can include any appropriate acid (*e.g.,* an organic acid or an inorganic acid). Examples of acids that can be used to form a salt with 4-AP or a derivative thereof include, without limitation, hydrochloric acid, hydrobromic acid, sulfuric acid, phosphoric acid, methanesulfonic acid, ethanesulfonic acid, malic acid, acetic acid, oxalic acid, tartaric acid, citric acid, lactic acid, fumaric acid, succinic acid, maleic acid, salicylic acid, benzoic acid, phenylacetic acid, and mandelic acid.

In some cases, 4-AP and/or one or more derivatives of 4-AP can be as described elsewhere (see, *e.g.,* U.S. Patent Application Publication No. 2018/0271847 and U.S. Patent No. 9,993,429).

A thermoresponsive composition described herein (*e.g.,* a thermoresponsive polymer composition containing 4-AP and/or one or more derivatives of 4-AP) or a composition described herein (*e.g.,* a composition containing 4-AP and/or one or more derivatives of 4-AP) can include any appropriate amount of 4-AP and/or one or more derivatives of 4-AP. In some cases, a thermoresponsive composition described herein can include from about 10 nM (0.01 μM) to about 1 μM (*e.g.,* from about 0.01 μM to about 0.9 μM, from about 0.01 μM to about 0.8 μM, from about 0.01 μM to about 0.7 μM, from about 0.01 μM to about 0.6 μM, from about 0.01 μM to about 0.5 μM, from about 0.01 μM to about 0.4 μM, from about 0.01 μM to about 0.3 μM, from about 0.01 μM to about 0.2 μM, from about 0.01 μM to about 0.1 μM, from about 0.1 μM to about 1 μM, from about 0.2 μM to about 1 μM, from about 0.3 μM to about 1 μM, from about 0.4 μM to about 1 μM, from about 0.5 μM to about 1 μM, from about 0.6 μM to about 1 μM, from about 0.7 μM to about 1 μM, from about 0.8 μM to about 1 μM, from about 0.9 μM to about 1 μM, from about 0.1 μM to about 0.9 μM, from about 0.2 μM to about 0.8 μM, from about 0.3 μM to about 0.7 μM, from about 0.4 μM to about 0.6 μM, from about 0.1 μM to about 0.3 μM, from about 0.3 μM to about 0.5 μM, from about 0.5 μM to about 0.7 μM, or from about 0.7 μM to about 0.9 μM) of 4-AP and/or one or more derivatives of 4-AP. In some cases, a thermoresponsive composition described herein can include from about 5 mg to about 100 mg (*e.g.,* from about 5 mg to about 90 mg, from about 5 mg to about 80 mg, from about 5 mg to about 70 mg, from about 5 mg to about 60 mg, from about 5 mg to about 50 mg, from about 5 mg to about 40 mg, from about 5 mg to about 30 mg, from about 5 mg to about 20 mg, from about 5 mg to about 10 mg, from about 15 mg to about 100 mg, from about 25 mg to about 100 mg, from about 35 mg to about 100 mg, from about 45 mg to about 100 mg, from about 55 mg to about 100 mg, from about 65 mg to about 100 mg, from about 75 mg to about 100 mg, from about 85 mg to about 100 mg, from about 95 mg to about 100 mg, from about 10 mg to about 90 mg, from about 20 mg to about 80 mg, from about 30 mg to about 70 mg, from about 40 mg to about 60 mg, from about 10 mg to about 30 mg, from about 30 mg to about 50 mg, from about 50 mg to about 70 mg, or from about 70 mg to about 90 mg) of 4-AP and/or one or more derivatives of 4-AP. In some cases, a thermoresponsive composition described herein can include from about 0.01% to about 99% (*e.g.,* from about 0.01% to about 90%, from about 0.01% to about 80%, from about 0.01% to about 70%, from about 0.01% to about 60%, from about 0.01% to about 50%, from about 0.01% to about 40%, from about 0.01% to about 30%, from about 0.01% to about 20%, from about 0.01% to about 10%, from about 0.01% to about 5%, from about 0.01% to about 1%, from about 1% to about 99%, from about 5% to about 99%, from about 10% to about 99%, from about 20% to about 99%, from about 30% to about 99%, from about 40% to about 99%, from about 50% to about 99%, from about 60% to about 99%, from about 70% to about 99%, from about 80% to about 99%, from about 90% to about 99%, from about 10% to about 90%, from about 20% to about 80%, from about 30% to about 70%, from about 40% to about 60%, from about 10% to about 30%, from about 30% to about 50%, from about 50% to about 70%, or from about 70% to about 90%) of 4-AP and/or one or more derivatives of 4-AP.

A thermoresponsive composition described herein (*e.g.,* a thermoresponsive polymer composition containing 4-AP and/or one or more derivatives of 4-AP) can include any appropriate polymer(s). A "polymer" is a molecule of repeating structural units (*e.g.,* monomers) formed via a chemical reaction, *i.e.,* polymerization. Examples of polymers that can be included in a thermoresponsive composition described herein include, without limitation, PLGA, PEG, methoxypoly(ethylene glycol) (mPEG), poly(L-lactide-co-glycolide) (PLLGA), polycaprolactone (PCL), poly(lactide-co-caprolactone) (PLCL), poly-DL-lactide (PDLLA), polylactic acid (PLA), and any combinations thereof. A polymer can have any

appropriate molecular weight (MW; *e.g.,* an average MW). For example, a polymer that can be included in a thermoresponsive composition described herein can have a molecular weight of from about 200 Daltons (Da) to about 3000 Da. For example, when a polymer is a PLGA, the PLGA can have a MW (*e.g.,* an average MW) of from about 200 Da to about 1900 Da (*e.g.,* from about 200 Da to about 1700 Da, from about 200 Da to about 1500 Da, from about 200 Da to about 1300 Da, from about 200 Da to about 1000 Da, from about 200 Da to about 700 Da, from about 200 Da to about 500 Da, from about 400 Da to about 1900 Da, from about 750 Da to about 1900 Da, from about 1000 Da to about 1900 Da, from about 1200 Da to about 1900 Da, from about 1500 Da to about 1900 Da, from about 1700 Da to about 1900 Da, from about 500 Da to about 1700 Da, or from about 1000 Da to about 1500 Da). In some cases, when a polymer is a PLGA, the PLGA can have a MW of about 1500 Da (PLGA 1500). In some cases, when a polymer is a PLGA, the PLGA can have a MW of about 1700 Da (PLGA 1700)). For example, when a polymer is a PEG, the PEG can have a MW (*e.g.,* an average MW) of from about 1500 Da to about 3000 Da (*e.g.,* from about 1500 Da to about 2500 Da, from about 1500 Da to about 2000 Da, from about 1500 Da to about 1700 Da, from about 1700 Da to about 3000 Da, from about 2000 Da to about 3000 Da, from about 2500 Da to about 3000 Da, from about 1700 Da to about 2500 Da, from about 1900 Da to about 2200 Da, from about 1700 Da to about 1900 Da, from about 1900 Da to about 2100 Da, or from about 2100 Da to about 2500 Da). In some cases, when a polymer is a PEG, the PEG can have a MW of about 1500 Da (PEG 1500). Unless otherwise specified, polymer MWs provided herein are weight average MW. In some cases, a polymer can be a copolymer (*e.g.,* can be formed from polymerization of two or more different monomers). When a polymer is a copolymer, the copolymer can be any type of copolymer (*e.g.,* a linear copolymer or a branched copolymer). Examples of types of copolymers that can be used as described herein include, without limitation, alternating copolymers, statistical copolymers, gradient copolymers, block copolymers, and grafted copolymers. A polymer can be natural polymer or a synthetic polymer. In some cases, a polymer can be a biodegradable polymer. In some cases, a polymer can be a biocompatible polymer. In some cases, a polymer included in a thermoresponsive composition described herein can include one or more functional groups (*e.g.,* hydrophilic functional groups) in its polymeric structures. Examples of functional groups that can be included in a polymer of a thermoresponsive composition described herein include, without limitation, such as amino groups (*e.g.,* $NH_2$), hydroxyl groups (*e.g.,* OH), amide groups (*e.g.* CONH- and $CONH_2$), and sulfate groups (*e.g.,* $-SO_3H$).

In some cases, a thermoresponsive composition described herein (*e.g.,* a thermoresponsive polymer composition containing 4-AP and/or one or more derivatives of 4-AP) can be a copolymer. A copolymer can include any appropriate ratio of a first polymer to a second polymer. For example, when a thermoresponsive composition described herein includes a PLGA copolymer, the PLGA can include from about 1:1 LA: GA to about 15:1 of LA:GA (*e.g.,* from about 1:1 to about 14:1, from about 1:1 to about 13:1, from about 1:1 to about 12:1, from about 1:1 to about 10:1, from about 1:1 to about 5:1, from about 1:1 to about 5:1, from about 5:1 to about 15:1, from about 8:1 to about 15:1, from about 10:1 to about 15:1, from about 13:1 to about 15:1, from about 3:1 to about 5:1, from about 5:1 to about 10:1, or from about 10:1 to about 13:1 of LA:GA). In some cases, when a thermoresponsive composition described herein includes a PLGA copolymer, the PLGA can include about 15:1 LA:GA. For example, when a thermoresponsive composition described herein includes a PLGA copolymer, the PLGA can include from about 50% / 50% LA/GA to about 94% / 6% LA/GA (*e.g.,* in weight percent). In some cases, when a thermoresponsive composition described herein includes a PLGA copolymer, the PLGA can include about 94% / 6% LA/GA (*e.g.,* in weight percent). For example, when a thermoresponsive composition described herein includes a PLGA-PEG copolymer (*e.g.,* a PLGA-PEG block copolymer), the thermoresponsive composition described herein can include from about 3:5 PLGA:PEG to about 9:1 of PLGA:PEG (*e.g.,* from about 3:5 PLGA:PEG to about 8:1 of PLGA:PEG, from about 3:5 PLGA:PEG to about 7:1 of PLGA:PEG, from about 3:5 PLGA:PEG to about 6:1 of PLGA:PEG, from about 3:5 PLGA:PEG to about 5:1 of PLGA:PEG, from about 3:5 PLGA:PEG to about 4:1 of PLGA:PEG, from about 3:5 PLGA:PEG to about 3:1 of PLGA:PEG, from about 3:5 PLGA:PEG to about 2:1 of PLGA:PEG, from about 3:5 PLGA:PEGto about 1:1 of PLGA:PEG, from about 4:5 PLGA:PEG to about 9:1 of PLGA:PEG, from about 1:1 PLGA:PEG to about 9:1 of PLGA:PEG, from about 2:1 PLGA:PEG to about 9:1 of PLGA:PEG, from about 3:1 PLGA:PEG to about 9:1 of PLGA:PEG, from about 4:1 PLGA:PEG to about 9:1 of PLGA:PEG, from about 5:1 PLGA:PEG to about 9:1 of PLGA:PEG, from about 6:1 PLGA:PEG to about 9:1 of PLGA:PEG, from about 7:1 PLGA:PEG to about 9:1 of PLGA:PEG, from about 8:1 PLGA:PEG to about 9:1 of PLGA:PEG, from about 4:5 PLGA:PEG to about 8:1 of PLGA:PEG, from about 1:1 PLGA:PEG to about 7:1 of PLGA:PEG, from about 2:1 PLGA:PEG to about 6:1 of PLGA:PEG, from about 3:1 PLGA:PEG to about 5:1 of PLGA:PEG, from about 1:1 PLGA:PEG to about 3:1 of PLGA:PEG, from about 3:1 PLGA:PEG to about 5:1 of PLGA:PEG, or from about 5:1 PLGA:PEG to about 7:1 of PLGA:PEG). For example, when a thermoresponsive composition described herein includes a PLGA-PEG copolymer (*e.g.,* a PLGA-PEG block copolymer), the thermoresponsive composition described herein can include from about 37.5% / 62.5% PLGA/PEG (e.g., in weight percent) to about 90% / 10% of PLGA/PEG (*e.g.,* from about 37.5% / 62.5% PLGA/PEG to about 80% / 20% of PLGA/PEG, from about 37.5% / 62.5% PLGA/PEG to about 70% / 30% of PLGA/PEG, from about 37.5% / 62.5% PLGA/PEG to about 60% / 40% of PLGA/PEG, from about 37.5% / 62.5% PLGA/PEG to about 50% / 50% of PLGA/PEG, from about 37.5% / 62.5% PLGA/PEG to about 40% / 60% of PLGA/PEG, from about 40% / 60% PLGA/PEG to about 50% / 50% of PLGA/PEG, from about 60% / 40% PLGA/PEG to about 90%

/ 10% of PLGA/PEG, from about 70% / 30% PLGA/PEG to about 90% / 10% of PLGA/PEG, from about 80% / 20% PLGA/PEG to about 90% / 10% of PLGA/PEG, from about 40% / 60% PLGA/PEG to about 80% / 20% of PLGA/PEG, or from about 50% / 50% PLGA/PEG to about 70% / 30% of PLGA/PEG).

In some cases, polymers within a thermoresponsive composition described herein (*e.g.,* a thermoresponsive polymer composition containing 4-AP and/or one or more derivatives of 4-AP) can be crosslinked or can be such that they are not crosslinked.

In some cases, a thermoresponsive composition described herein (*e.g.,* a thermoresponsive polymer composition containing 4-AP and/or one or more derivatives of 4-AP) can include 4-AP and a PLGA-PEG copolymer (*e.g.,* a PLGA-PEG block copolymer including PLGA-PEG-PLGA such as PLGA-PEG-PLGA 1700-1500-1700 Da). For example, a thermoresponsive composition described herein can include from about 10 nM 4-AP to about 1 μM 4-AP, can include from about 37.5% to about 90% PLGA, and can include from about 10% to about 62.5% PEG.

In some cases, a thermoresponsive composition described herein (*e.g.,* a thermoresponsive polymer composition containing 4-AP and/or one or more derivatives of 4-AP) or a composition described herein (*e.g.,* a composition containing 4-AP and/or one or more derivatives of 4-AP) can include one or more pharmaceutically acceptable carriers (additives), excipients, and/or diluents. Examples of pharmaceutically acceptable carriers, excipients, and diluents that can be used in a composition described herein include, without limitation, phosphate-buffered saline, sucrose, lactose, starch (*e.g.,* starch glycolate), cellulose, cellulose derivatives (e.g., modified celluloses such as microcrystalline cellulose and cellulose ethers like hydroxypropyl cellulose (HPC) and cellulose ether hydroxypropyl methylcellulose (HPMC)), xylitol, sorbitol, mannitol, gelatin, polymers (*e.g.,* polyvinylpyrrolidone (PVP), crosslinked polyvinylpyrrolidone (crospovidone), carboxymethyl cellulose, polyethylene-polyoxypropylene-block polymers, and crosslinked sodium carboxymethyl cellulose (croscarmellose sodium)), titanium oxide, azo dyes, silica gel, fumed silica, talc, magnesium carbonate, vegetable stearin, magnesium stearate, aluminum stearate, stearic acid, antioxidants (*e.g.,* vitamin A, vitamin E, vitamin C, retinyl palmitate, and selenium), citric acid, sodium citrate, parabens (*e.g.,* methyl paraben and propyl paraben), petrolatum, dimethyl sulfoxide, mineral oil, serum proteins (*e.g.,* human serum albumin), glycine, sorbic acid, potassium sorbate, water, salts or electrolytes (*e.g.,* saline, protamine sulfate, disodium hydrogen phosphate, potassium hydrogen phosphate, sodium chloride, and zinc salts), colloidal silica, magnesium trisilicate, polyacrylates, waxes, wool fat, and lecithin.

In some cases, a thermoresponsive composition described herein (*e.g.,* a thermoresponsive polymer composition containing 4-AP and/or one or more derivatives of 4-AP) can be formulated as a delivery system for 4-AP and/or one or more derivatives of 4-AP (*e.g.,* can be formulated to release 4-AP and/or one or more derivatives of 4-AP from the thermoresponsive composition). For example, a thermoresponsive composition described herein can be formulated to release 4-AP and/or one or more derivatives of 4-AP from the thermoresponsive composition when the thermoresponsive composition is in its gel phase. For example, a thermoresponsive composition described herein can be formulated as a controlled-release delivery system for 4-AP and/or one or more derivatives of 4-AP from the thermoresponsive composition. Examples of types of controlled-release delivery that a thermoresponsive composition described herein can be formulated for include, without limitation, burst release, slow release, delayed release, and sustained release. When a thermoresponsive composition is formulated for a burst release of 4-AP and/or one or more derivatives of 4-AP from the gel phase of the thermoresponsive composition, the burst release can release the 4-AP and/or one or more derivatives of 4-AP present in the thermoresponsive composition from about 0.5 hours to about 24 hours (*e.g.,* about 1 hour) after administering the thermoresponsive composition to a mammal (*e.g.,* after the thermoresponsive composition has formed a gel). When a thermoresponsive composition is formulated for a slow release of 4-AP and/or one or more derivatives of 4-AP from the gel phase of the thermoresponsive composition, the slow release can release the 4-AP and/or one or more derivatives of 4-AP present in the thermoresponsive composition for from about 60 seconds to about 4 weeks (*e.g.,* for from about 60 minutes to about 3 weeks) after administering the thermoresponsive composition to a mammal (*e.g.,* after the thermoresponsive composition has formed a gel). In some cases, a thermoresponsive composition described herein can be formulated to provide two or more types of controlled-release of 4-AP and/or one or more derivatives of 4-AP from the thermoresponsive composition in its gel phase. For example, a thermoresponsive composition described herein can be formulated to provide a burst release of 4-AP and/or one or more derivatives of 4-AP from the thermoresponsive composition in its gel phase followed by a slow release of any remaining 4-AP and/or one or more derivatives of 4-AP from the thermoresponsive composition in its gel phase.

This document also provides methods and materials for using a thermoresponsive composition described herein (*e.g.,* a thermoresponsive polymer composition containing 4-AP and/or one or more derivatives of 4-AP) or a composition described herein (*e.g.,* a composition containing 4-AP and/or one or more derivatives of 4-AP).

In some cases, a thermoresponsive composition described herein (*e.g.,* an effective amount of a thermoresponsive composition described herein) can be used for assessing an injured nerve (*e.g.,* an injured nerve within a mammal) for the presence or absence of axonal continuity. For example, a thermoresponsive composition described herein can be delivered onto, into, around, and/or near an injured nerve (*e.g.,* a nerve injury site) within a mammal to assess axonal continuity of the injured nerve. Any appropriate method can be used to assess axonal continuity of an

injured nerve. In some cases, a thermoresponsive composition described herein can be delivered onto, into, around, and/or near a nerve injury site within a mammal, an electrical stimulation can be delivered onto, into, around, and/or near nerve injury site within a mammal, and nerve conduction (*e.g.,* from the injured nerve) can be detected. For example, when an injured nerve is a motor nerve, an electrical stimulation can be delivered onto, into, around, and/or near the nerve injury site within a mammal, and muscle contraction (*e.g.,* from the injured motor nerve) can be detected. When the presence of nerve conduction is present, the nerve injury can be classified as having axonal continuity. When the presence of nerve conduction is absent, the nerve injury can be classified as lacking axonal continuity.

In some cases, a thermoresponsive composition described herein (*e.g.,* an effective amount of a thermoresponsive composition described herein) can used for treating a mammal in need thereof (*e.g.,* a human having a nerve injury). For example, a thermoresponsive composition described herein can be administered to a mammal having a nerve injury to treat the mammal.

In some cases, a thermoresponsive composition described herein (*e.g.,* a thermoresponsive polymer composition containing 4-AP and/or one or more derivatives of 4-AP) can be administered to a mammal (*e.g.,* a human) having a nerve injury to improve the function (*e.g.,* motor function and/or sensory function) of the injured nerve. For example, a thermoresponsive composition described herein can be administered to a mammal having a nerve injury to improve the function of the injured nerve by, for example, 10, 20, 30, 40, 50, 60, 70, 80, 90, 95, or more percent.

In some cases, a thermoresponsive composition described herein (*e.g.,* a thermoresponsive polymer composition containing 4-AP and/or one or more derivatives of 4-AP) can be administered to a mammal (*e.g.,* a human) having a nerve injury to reduce or eliminate one or more symptoms of the nerve injury (*e.g.,* pain, sensitivity, numbness, tingling, prickling, burning, problems with positional awareness, loss in grip strength, and/or difficulty walking). For example, a thermoresponsive composition described herein can be administered to a mammal having a nerve injury to reduce or eliminate one or more symptoms of a nerve injury by, for example, 10, 20, 30, 40, 50, 60, 70, 80, 90, 95, or more percent.

In some cases, a thermoresponsive composition described herein (*e.g.,* a thermoresponsive polymer composition containing 4-AP and/or one or more derivatives of 4-AP) can be administered to a mammal (*e.g.,* a human) having a nerve injury to promote myelination of the injured nerve. For example, a thermoresponsive composition described herein can be administered to a mammal having a nerve injury to increase the amount of myelin on the injured nerve by, for example, 10, 20, 30, 40, 50, 60, 70, 80, 90, 95, or more percent.

In some cases, a thermoresponsive composition described herein *(e.g.,* a thermoresponsive polymer composition containing 4-AP and/or one or more derivatives of 4-AP) can be administered to a mammal (*e.g.,* a human) having a nerve injury to increase the amount of expression of one or more myelin polypeptides (e.g., neurofilament-H (NF-H) polypeptides and/or myelin protein zero (MPZ) polypeptides). For example, a nerve treated with a thermoresponsive composition described herein can have at least 2 fold (e.g., at least about 2.5 fold, at least about 4 fold, at least about 5.5 fold, at least about 7.5 fold, or at least about 10 fold) more expression of one or more myelin polypeptides (e.g., as compared to a nerve not receiving 4-AP). For example, a nerve treated with a thermoresponsive composition described herein can have from about 2 fold to about 10 fold (e.g., from about 2 fold to about 9 fold, from about 2 fold to about 8 fold, from about 2 fold to about 7 fold, from about 2 fold to about 6 fold, from about 2 fold to about 5 fold, from about 2 fold to about 4 fold, from about 2 fold to about 3 fold, from about 3 fold to about 10 fold, from about 4 fold to about 10 fold, from about 5 fold to about 10 fold, from about 6 fold to about 10 fold, from about 7 fold to about 10 fold, from about 8 fold to about 10 fold, from about 9 fold to about 10 fold, from about 3 fold to about 9 fold, from about 4 fold to about 8 fold, from about 5 fold to about 7 fold, from about 2 fold to about 4 fold, from about 3 fold to about 5 fold, from about 4 fold to about 6 fold, from about 5 fold to about 7 fold, from about 6 fold to about 8 fold, or from about 7 fold to about 9 fold) more expression of one or more myelin polypeptides (e.g., as compared to a nerve not receiving 4-AP).

In some cases, a thermoresponsive composition described herein *(e.g.,* a thermoresponsive polymer composition containing 4-AP and/or one or more derivatives of 4-AP) can be administered to a mammal (*e.g.,* a human) having a nerve injury to increase the area (*e.g.,* the axonal area) of the injured nerve. For example, a thermoresponsive composition described herein can be administered to a mammal having a nerve injury to increase the area of the injured nerve by, for example, 10, 20, 30, 40, 50, 60, 70, 80, 90, 95, or more percent.

In some cases, a thermoresponsive composition described herein (*e.g.,* a thermoresponsive polymer composition containing 4-AP and/or one or more derivatives of 4-AP) can be administered to a mammal (*e.g.,* a human) having a nerve injury to increase the conduction velocity of the injured nerve. For example, a thermoresponsive composition described herein can be administered to a mammal having a nerve injury as described herein to increase the conduction velocity of the injured nerve by, for example, 10, 20, 30, 40, 50, 60, 70, 80, 90, 95, or more percent.

In some cases, a thermoresponsive composition described herein (*e.g.,* a thermoresponsive polymer composition containing 4-AP and/or one or more derivatives of 4-AP) can be administered to a mammal (*e.g.,* a human) having a nerve injury to reinnervate the site of the injured nerve. For example, a thermoresponsive composition described herein can be administered to a mammal having a nerve injury to improve the nerve supply to the site of the injured nerve by, for example, 10, 20, 30, 40, 50, 60, 70, 80, 90, 95, or more percent.

In some cases, a composition described herein (*e.g.,* a composition containing 4-AP and/or one or more

derivatives of 4-AP) can be administered to a mammal (*e.g.,* a human) having a wound (*e.g.,* a skin wound or an internal wound) to accelerate wound healing within the mammal. For example, a composition described herein can be administered to a mammal having a wound to accelerate wound healing within the mammal by, for example, 10, 20, 30, 40, 50, 60, 70, 80, 90, 95, or more percent.

In some cases, a composition described herein (*e.g.,* a composition containing 4-AP and/or one or more derivatives of 4-AP) can be administered to a mammal (*e.g.,* a human) having a wound (*e.g.,* a skin wound or an internal wound) to reduce or eliminate the breakdown of anastomosis within the mammal. For example, a composition described herein can be administered to a mammal having a wound to reduce the breakdown of anastomosis within the mammal by, for example, 10, 20, 30, 40, 50, 60, 70, 80, 90, 95, or more percent.

In some cases, a composition described herein (*e.g.,* a composition containing 4-AP and/or one or more derivatives of 4-AP) can be administered to a mammal (*e.g.,* a human) having a wound (*e.g.,* a skin wound or an internal wound) to accelerate the onset of an inflammatory phase of wound healing. For example, a composition described herein can be administered to a mammal having a wound to accelerate the onset of an inflammatory phase of wound healing within the mammal by, for example, 10, 20, 30, 40, 50, 60, 70, 80, 90, 95, or more percent.

In some cases, a composition described herein (*e.g.,* a composition containing 4-AP and/or one or more derivatives of 4-AP) can be administered to a mammal (*e.g.,* a human) having a wound (*e.g.,* a skin wound or an internal wound) to increase collagen deposition at the wound. For example, a composition described herein can be administered to a mammal having a wound to increase collagen deposition at the wound within the mammal by, for example, 10, 20, 30, 40, 50, 60, 70, 80, 90, 95, or more percent.

In some cases, a composition described herein (*e.g.,* a composition containing 4-AP and/or one or more derivatives of 4-AP) can be administered to a mammal (*e.g.,* a human) having a wound (*e.g.,* a skin wound or an internal wound) to increase re-epithelialization at the wound. For example, a composition described herein can be administered to a mammal having a wound to increase re-epithelialization at the wound within the mammal by, for example, 10, 20, 30, 40, 50, 60, 70, 80, 90, 95, or more percent.

In some cases, a composition described herein (*e.g.,* a composition containing 4-AP and/or one or more derivatives of 4-AP) can be administered to a mammal (*e.g.,* a human) having a wound (*e.g.,* a skin wound or an internal wound) to increase angiogenesis at the wound. For example, a composition described herein can be administered to a mammal having a wound to increase angiogenesis at the wound within the mammal by, for example, 10, 20, 30, 40, 50, 60, 70, 80, 90, 95, or more percent.

Any appropriate mammal having a nerve injury and/or having a wound (*e.g.,* a skin wound or an internal wound) can be assessed and/or treated as described herein (*e.g.,* by administering a thermoresponsive composition described herein). Examples of mammals that can have a nerve injury and/or a wound and that can be assessed and/or treated as described herein include, without limitation, humans, non-human primates such as monkeys, horses, bovine species, porcine species, dogs, cats, mice, rats, rabbits, and goats. In some cases, a human having a nerve injury can be assessed and/or treated as described herein. In some cases, a human having a wound (*e.g.,* a skin wound or an internal wound) can be treated as described herein.

A mammal having any type of nerve injury can be assessed and/or treated as described herein (*e.g.,* by administering a thermoresponsive composition described herein). Examples of types of nerve injuries that can be assessed and/or treated as described herein include, without limitation, peripheral nerve injuries, traumatic nerve injuries, crush injuries, stretched nerves, compressed nerves, pinched nerves, wounds (*e.g.,* skin wounds), somatic nerve injury in the gut, spinal nerve root injury, and cauda equina. In some cases, an injured nerve that can be treated as described herein is not a transected nerve. In some cases, an injured nerve that can be treated as described herein is not a severed nerve. In some cases, an injured nerve can be associated with a disease or disorder such as carpel tunnel syndrome, sciatica, a diabetic wound, ileus, or a pressure sore.

A nerve injury that can be assessed and/or treated as described herein (*e.g.,* by administering a thermoresponsive composition described herein) can affect any type of nerve. In some cases, an injured nerve that can be assessed and/or treated as described herein can be a sensory neuron. In some cases, an injured nerve that can be assessed and/or treated as described herein can be a motor neuron. In some cases, an injured nerve that can be assessed and/or treated as described herein can be a peripheral nerve (*e.g.,* a somatic nerve). In some cases, an injured nerve that can be assessed and/or treated as described herein can be in a nerve plexus. Examples of types of nerves that can be injured and can be assessed and/or treated as described herein include, without limitation, the sciatic nerve, ulnar nerve, radial nerve, median nerve, femoral nerve, a somatic nerve in the gut, a somatic nerve in the skin, and a spinal root nerve.

A nerve injury that can be assessed and/or treated as described herein (*e.g.,* by administering a thermoresponsive composition described herein) can affect a nerve in any location within a mammal. In some cases, an injured nerve that can be assessed and/or treated as described herein can be in the brain of a mammal. In some cases, an injured nerve that can be assessed and/or treated as described herein can be in the spinal cord of a mammal.

In some cases, methods described herein also can include identifying a mammal as having a nerve injury. Examples of methods for identifying a mammal as having a nerve injury include, without limitation, physical examinations,

electromagnetic fields, functional nerve electrical stimulation techniques, electromyography, nerve conduction studies, and magnetic resonance imaging. Once identified as having a nerve injury, a mammal can be administered or instructed to self-administer a thermoresponsive composition described herein (*e.g.,* a thermoresponsive polymer composition containing 4-AP and/or one or more derivatives of 4-AP).

A mammal having any type of wound can be treated as described herein (*e.g.,* by administering a composition described herein). A wound can affect any part of a mammal (*e.g.,* any part of a mammal's body). In some cases, a wound can be a cutaneous wound or skin wound. In some cases, a wound can be an internal wound (*e.g.,* a wound involving the abdomen, a muscle, and/or a vessel). In some cases, a wound can be a soft tissue wound (e.g., a rupture of a soft tissue). In some cases, a wound can include an anastomosis (e.g., an abdominal anastomosis or a vessel anastomosis). In some cases, a wound can include a nerve injury. In some cases, a wound can be a wound that does not include a nerve injury. Examples of wounds that can be treated as described herein include, without limitation, after-shave skin wounds, abrasion skin wounds, diabetic skin wounds, bed sores, surgical wounds, anastamotic leaks, tendon gaps, muscle defects, multi-tissue disruptions (e.g., tissue disruptions involving distinct layers), and ulcerations (e.g., internal and external ulcerations) such as scrotal ulcerations, buccal sores, and gastrointestinal tract ulcerations.

In some cases, the methods described herein can include identifying a mammal (*e.g.,* a human) as having a wound (*e.g.,* a skin wound or an internal wound). Any appropriate method can be used to identify a mammal as having a wound. For example, visual inspection, physical examinations (*e.g.,* for joint tenderness, swelling, redness, and flexibility), and/or imaging tests (*e.g.,* X-rays and magnetic resonance imaging (MRI), ultrasound, computed tomography (*e.g.,* computed tomography with or without angiography), radiographic angiography, and/or magnetic resonance angiography) can be used to identify mammals (*e.g.,* humans) as having a wound.

A thermoresponsive composition described herein (*e.g.,* a thermoresponsive polymer composition containing 4-AP and/or one or more derivatives of 4-AP) can be administered to a mammal in need thereof (*e.g.,* a mammal having a nerve injury) at any appropriate time. In some cases, a thermoresponsive composition described herein can be administered within two weeks of the mammal sustaining a nerve injury. For example, a thermoresponsive composition described herein can be administered within from about one hour to about two weeks of the mammal sustaining a nerve injury. In some cases, a thermoresponsive composition described herein can be administered within two weeks of the mammal being identified as having a nerve injury. For example, a thermoresponsive composition described herein can be administered immediately after injury to within about two weeks of the mammal being identified as having a nerve injury.

A composition described herein (*e.g.,* a composition containing 4-AP and/or one or more derivatives of 4-AP) can be administered to a mammal in need thereof *(e.g.,* a mammal having a wound such as a skin wound) at any appropriate time. In some cases, a composition described herein can be administered within two weeks of the mammal sustaining a wound (*e.g.,* a skin wound or an internal wound). For example, a composition described herein can be administered within from about one hour to about ten days of the mammal sustaining a wound. In some cases, a composition described herein can be administered within two weeks of the mammal being identified as having a wound. For example, a composition described herein can be administered immediately after sustaining a wound to within about two weeks of the mammal being identified as having a wound.

A thermoresponsive composition described herein (*e.g.,* a thermoresponsive polymer composition containing 4-AP and/or one or more derivatives of 4-AP) can be administered to a mammal in need thereof (*e.g.,* a mammal having a nerve injury) by any appropriate route. For example, a thermoresponsive composition described herein can be administered by injecting the thermoresponsive composition (*e.g.,* in a liquid phase) onto, into, around, and/or near an injured nerve (*e.g.,* a nerve injury site) within a mammal (*e.g.,* a human). In some cases, the injection can be applied directly onto an open injury site (*e.g.,* an open wound, and a surgical opening), such that the thermoresponsive composition can form a gel at a nerve injury site. In some cases, the injection can be into the vascular (*e.g.,* arterial) supply of a tissue having an injured nerve (*e.g.,* a nerve injury site) within a mammal (*e.g.,* a human) such that the thermoresponsive composition can travel to a nerve injury site via the vasculature in a liquid phase and can form a gel at a nerve injury site. In some cases, the injection can be an image-guided injection, where an imaging technique is used to visualize the placement of one or more injection needles. Imaging techniques that can be used in image-guided injection include, without limitation, ultrasound, radiography, X-ray, CT (*e.g.,* single-photon emission CT), fluoroscopy, positron emission tomography, and MRI.

A thermoresponsive composition described herein (*e.g.,* a thermoresponsive polymer composition containing 4-AP and/or one or more derivatives of 4-AP) can be administered to a mammal in need thereof (*e.g.,* a mammal having a nerve injury) locally or systemically. For example, a thermoresponsive composition described herein can be administered (*e.g.,* in a liquid phase) locally by an injection onto, into, around, and/or near a nerve (*e.g.,* an injured nerve) within a mammal (*e.g.,* a human). For example, a thermoresponsive composition described herein can be injected as a liquid onto, into, around, and/or near an injured nerve within a mammal, and can form a gel *in situ* (*e.g.,* to maintain close proximity between the thermoresponsive composition and the nerve injury site). When injecting a thermoresponsive composition described herein near an injured nerve, the thermoresponsive composition can be injected within from about 0 cm (*e.g.,* directly abutting an injured nerve) to about 200 cm (*e.g.,* from about 0 cm to about 175 cm, from about 0 cm

to about 150 cm, from about 0 cm to about 125 cm, from about 0 cm to about 100 cm, from about 0 cm to about 75 cm, from about 0 cm to about 50 cm, from about 0 cm to about 40 cm, from about 0 cm to about 30 cm, from about 0 cm to about 20 cm, from about 0 cm to about 10 cm, from about 1 cm to about 200 cm, from about 2 cm to about 200 cm, from about 5 cm to about 200 cm, from about 10 cm to about 200 cm, from about 25 cm to about 200 cm, from about 50 cm to about 200 cm, from about 75 cm to about 200 cm, from about 100 cm to about 200 cm, from about 150 cm to about 200 cm, from about 1 cm to about 150 cm, from about 2 cm to about 125 cm, from about 3 cm to about 100 cm, from about 5 cm to about 75 cm, from about 7 cm to about 50 cm, from about 10 cm to about 40 cm, from about 12 cm to about 30 cm, from about 15 cm to about 20 cm, from about 1 cm to about 3 cm, from about 3 cm to about 5 cm, from about 5 cm to about 8 cm, from about 7 cm to about 10 cm, from about 10 cm to about 20 cm, from about 15 cm to about 50 cm, from about 25 cm to about 75 cm, from about 50 cm to about 100 cm, from about 75 cm to about 125 cm, or from about 100 cm to about 150 cm) of the nerve injury site.

A composition described herein (*e.g.,* a composition containing 4-AP and/or one or more derivatives of 4-AP) can be administered to a mammal in need thereof (*e.g.,* a mammal having a wound such as a skin wound or an internal wound) locally or systemically. In some cases, a composition described herein can be administered systemically. For example, a composition described herein can be designed for oral or parenteral (including intraperitoneal, subcutaneous, intramuscular, intravenous, and intradermal) administration to a mammal having a wound. Compositions suitable for oral administration include, without limitation, liquids, tablets, capsules, pills, powders, gels, and granules. Compositions suitable for parenteral administration include, without limitation, aqueous and non-aqueous sterile injection solutions that can contain anti-oxidants, buffers, bacteriostats, and solutes that render the formulation isotonic with the blood of the intended recipient. In some cases, a composition described herein can be formulated for parenteral administration (*e.g.,* intraperitoneal injection or intravenous injection).

An effective amount (*e.g.,* effective dose) of 4-AP and/or one or more derivatives of 4-AP in a thermoresponsive composition described herein (*e.g.,* a thermoresponsive polymer composition containing 4-AP and/or one or more derivatives of 4-AP) or a composition described herein (*e.g.,* a composition containing 4-AP and/or one or more derivatives of 4-AP) can vary depending on the severity of the nerve injury and/or the wound (*e.g.,* a skin wound or an internal wound), the route of administration, the age and general health condition of the subject, excipient usage, the possibility of co-usage with other therapeutic treatments such as use of other agents, and/or the judgment of the treating physician.

An effective amount of 4-AP and/or one or more derivatives of 4-AP in a thermoresponsive composition described herein (*e.g.,* a thermoresponsive polymer composition containing 4-AP and/or one or more derivatives of 4-AP) or a composition described herein (*e.g.,* a composition containing 4-AP and/or one or more derivatives of 4-AP) can be any amount that can treat a nerve injury and/or a wound (*e.g.,* a skin wound or an internal wound) within a mammal without producing significant toxicity to the mammal. An effective amount of 4-AP and/or one or more derivatives of 4-AP in a thermoresponsive composition described herein can be any appropriate amount. In some cases, an effective amount of 4-AP and/or one or more derivatives of 4-AP in a thermoresponsive composition described herein can be from about 0.5 milligrams per kilogram body weight (mg/kg) to about 10 mg/kg (*e.g.,* from about 0.5 mg/kg to about 9 mg/kg, from about 0.5 mg/kg to about 8 mg/kg, from about 0.5 mg/kg to about 7 mg/kg, from about 0.5 mg/kg to about 6 mg/kg, from about 0.5 mg/kg to about 5 mg/kg, from about 0.5 mg/kg to about 4 mg/kg, from about 0.5 mg/kg to about 3 mg/kg, from about 0.5 mg/kg to about 2 mg/kg, from about 0.5 mg/kg to about 1 mg/kg, from about 1 mg/kg to about 10 mg/kg, from about 2 mg/kg to about 10 mg/kg, from about 3 mg/kg to about 10 mg/kg, from about 4 mg/kg to about 10 mg/kg, from about 5 mg/kg to about 10 mg/kg, from about 6 mg/kg to about 10 mg/kg, from about 7 mg/kg to about 10 mg/kg, from about 8 mg/kg to about 10 mg/kg, from about 9 mg/kg to about 10 mg/kg, from about 1 mg/kg to about 9 mg/kg, from about 2 mg/kg to about 8 mg/kg, from about 3 mg/kg to about 7 mg/kg, from about 4 mg/kg to about 6 mg/kg, from about 0.5 mg/kg to about 1 mg/kg, from about 1 mg/kg to about 3 mg/kg, from about 3 mg/kg to about 5 mg/kg, from about 5 mg/kg to about 7 mg/kg, or from about 7 mg/kg to about 9 mg/kg). In some cases, an effective amount of 4-AP and/or one or more derivatives of 4-AP in a thermoresponsive composition described herein can be from about 0.01 nanograms per milliliter serum (ng/mL) to about 100 ng/mL (*e.g.,* from about 0.01 ng/mL to about 90 ng/mL, from about 0.01 ng/mL to about 80 ng/mL, from about 0.01 ng/mL to about 70 ng/mL, from about 0.01 ng/mL to about 60 ng/mL, from about 0.01 ng/mL to about 50 ng/mL, from about 0.01 ng/mL to about 40 ng/mL, from about 0.01 ng/mL to about 30 ng/mL, from about 0.01 ng/mL to about 20 ng/mL, from about 0.01 ng/mL to about 10 ng/mL, from about 0.01 ng/mL to about 1 ng/mL, from about 0.1 ng/mL to about 100 ng/mL, from about 1 ng/mL to about 100 ng/mL, from about 10 ng/mL to about 100 ng/mL, from about 20 ng/mL to about 100 ng/mL, from about 30 ng/mL to about 100 ng/mL, from about 40 ng/mL to about 100 ng/mL, from about 50 ng/mL to about 100 ng/mL, from about 60 ng/mL to about 100 ng/mL, from about 70 ng/mL to about 100 ng/mL, from about 80 ng/mL to about 100 ng/mL, from about 90 ng/mL to about 100 ng/mL, from about 1 ng/mL to about 90 ng/mL, from about 10 ng/mL to about 80 ng/mL, from about 20 ng/mL to about 70 ng/mL, from about 30 ng/mL to about 60 ng/mL, from about 40 ng/mL to about 50 ng/mL, from about 1 ng/mL to about 20 ng/mL, from about 20 ng/mL to about 40 ng/mL, from about 40 ng/mL to about 60 ng/mL, or from about 60 ng/mL to about 80 ng/mL). In some cases, an effective amount of 4-AP and/or one or more derivatives of 4-AP in a composition described herein can be from about 2 mg/kg to about 8 mg/kg (*e.g.,* from about 2 mg/kg to about 7 mg/kg, from about 2 mg/kg to

about 6 mg/kg, from about 2 mg/kg to about 5 mg/kg, from about 2 mg/kg to about 4 mg/kg, from about 3 mg/kg to about 8 mg/kg, from about 4 mg/kg to about 8 mg/kg, from about 5 mg/kg to about 8 mg/kg, from about 6 mg/kg to about 8 mg/kg, from about 3 mg/kg to about 7 mg/kg, from about 4 mg/kg to about 6 mg/kg, from about 3 mg/kg to about 5 mg/kg, or from about 5 mg/kg to about 7 mg/kg). In some cases, an effective amount of 4-AP and/or one or more derivatives of 4-AP in a composition described herein can be from about 0.01 ng/mL to about 100 ng/mL (*e.g.,* from about 0.01 ng/mL to about 90 ng/mL, from about 0.01 ng/mL to about 80 ng/mL, from about 0.01 ng/mL to about 70 ng/mL, from about 0.01 ng/mL to about 60 ng/mL, from about 0.01 ng/mL to about 50 ng/mL, from about 0.01 ng/mL to about 40 ng/mL, from about 0.01 ng/mL to about 30 ng/mL, from about 0.01 ng/mL to about 20 ng/mL, from about 0.01 ng/mL to about 10 ng/mL, from about 0.01 ng/mL to about 1 ng/mL, from about 0.1 ng/mL to about 100 ng/mL, from about 1 ng/mL to about 100 ng/mL, from about 10 ng/mL to about 100 ng/mL, from about 20 ng/mL to about 100 ng/mL, from about 30 ng/mL to about 100 ng/mL, from about 40 ng/mL to about 100 ng/mL, from about 50 ng/mL to about 100 ng/mL, from about 60 ng/mL to about 100 ng/mL, from about 70 ng/mL to about 100 ng/mL, from about 80 ng/mL to about 100 ng/mL, from about 90 ng/mL to about 100 ng/mL, from about 1 ng/mL to about 90 ng/mL, from about 10 ng/mL to about 80 ng/mL, from about 20 ng/mL to about 70 ng/mL, from about 30 ng/mL to about 60 ng/mL, from about 40 ng/mL to about 50 ng/mL, from about 1 ng/mL to about 20 ng/mL, from about 20 ng/mL to about 40 ng/mL, from about 40 ng/mL to about 60 ng/mL, or from about 60 ng/mL to about 80 ng/mL). The effective amount can remain constant or can be adjusted as a sliding scale or variable dose depending on the mammal's response to treatment. Various factors can influence the actual effective amount used for a particular application. For example, the frequency of administration, duration of treatment, use of multiple treatment agents, route of administration, and severity of the injured nerve may require an increase or decrease in the actual effective amount administered.

When a thermoresponsive composition described herein (*e.g.,* a thermoresponsive polymer composition containing 4-AP and/or one or more derivatives of 4-AP) is formulated for controlled-release of 4-AP and/or one or more derivatives of 4-AP from the thermoresponsive composition (*e.g.,* when the thermoresponsive composition is in its gel phase), the thermoresponsive composition described herein can maintain an effective amount of 4-AP and/or one or more derivatives of 4-AP for any appropriate amount of time (*e.g.,* an appropriate amount of time after administering the thermoresponsive composition to a mammal). For example, a thermoresponsive composition described herein can maintain an effective amount of 4-AP and/or one or more derivatives of 4-AP in a mammal for from about 1 minute to about 4 weeks, for from about 3 minutes to about 4 weeks, for from about 5 minutes to about 4 weeks (*e.g.,* for about 3 weeks) after administering the thermoresponsive composition to the mammal (*e.g.,* after the thermoresponsive composition has formed a gel).

The frequency of administration of a thermoresponsive composition described herein (*e.g.,* a thermoresponsive polymer composition containing 4-AP and/or one or more derivatives of 4-AP) or a composition described herein (*e.g.,* a composition containing 4-AP and/or one or more derivatives of 4-AP) can be any frequency that can treat a nerve injury and/or a wound (*e.g.,* a skin wound or an internal wound) within a mammal without producing significant toxicity to the mammal. For example, the frequency of administration can be from about once a week to about once every two months, from about once every two weeks to about once every six weeks, or from about once every three weeks to about once a month (*e.g.,* once every four weeks). The frequency of administration can remain constant or can be variable during the duration of treatment. A course of treatment with a thermoresponsive composition described herein can include rest periods. For example, a thermoresponsive composition described herein can be administered once a month over a six-month period followed by a rest period (*e.g.,* a one or two month rest period), and such a regimen can be repeated multiple times. In some cases, a thermoresponsive composition described herein (*e.g.,* a thermoresponsive polymer composition containing 4-AP and/or one or more derivatives of 4-AP) can be administered no more than one time to treat a nerve injury within a mammal (*e.g.,* a human). As with the effective amount, various factors can influence the actual frequency of administration used for a particular application. For example, the effective amount, duration of treatment, use of multiple treatment agents, route of administration, and severity of the injured nerve and/or the wound may require an increase or decrease in administration frequency.

An effective duration for administering a thermoresponsive composition described herein *(e.g.,* a thermoresponsive polymer composition containing 4-AP and/or one or more derivatives of 4-AP) or a composition described herein *(e.g.,* a composition containing 4-AP and/or one or more derivatives of 4-AP) can be any duration that treats an injured nerve and/or a wound *(e.g.,* a skin wound or an internal wound) within a mammal without producing significant toxicity to the mammal. For example, the effective duration can vary from several days to several weeks, months, or years. In some cases, the effective duration for the treatment of an injured nerve can range in duration from about one month to about 6 months. Multiple factors can influence the actual effective duration used for a particular treatment. For example, an effective duration can vary with the frequency of administration, effective amount, use of multiple treatment agents, route of administration, and severity of the injured nerve and/or the wound being treated.

In some cases, methods described herein also can include administering to a mammal (*e.g.,* a mammal having a nerve injury) one or more additional agents used to treat a nerve injury (*e.g.,* one or more agents in addition to a thermoresponsive composition described herein such as a thermoresponsive composition containing 4-AP and/or one

or more derivatives of 4-AP). The one or more additional agents used to treat an injured nerve can include any appropriate agent(s) used to treat a nerve injury. In some cases, an agent that can be used to treat a nerve injury can be an anticonvulsant. In some cases, an agent that can be used to treat a nerve injury can be a steroid (*e.g.,* a corticosteroid and a glucocorticoid). In some cases, an agent that can be used to treat a nerve injury can be an anti-inflammatory agent (*e.g.,* a non-steroidal anti-inflammatory drug (NSAID)). Examples of agents that can be used to treat a nerve injury include, without limitation, lamotrigine, gabapentin, valproic acid, topiramate, famotodine, phenobarbital, diphenylhydantoin, phenytoin, mephenytoin, ethotoin, mephobarbital, primidone, carbamazepine, ethosuximide, methsuximide, phensuximide, trimethadione, benzodiazepine, phenacemide, acetazolamide, progabide, clonazepam, divalproex sodium, magnesium sulfate injection, metharbital, paramethadione, phenytoin sodium, valproate sodium, clobazam, sulthiame, dilantin, diphenylan, L-5-hydroxytrytophan, methylprednisolone, dexamethasone, prednisone, salicylates (*e.g.,* aspirin), propionic acid derivatives (*e.g.,* ibuprofen or naproxen), acetic acid derivatives (*e.g.,* indomethacin), oxicam derivatives (*e.g.,* piroxicam), and fenamates (*e.g.,* menafemic acid). In cases where a mammal having a nerve injury is treated with a thermoresponsive composition described herein and is treated with one or more additional agents used to treat a nerve injury, the additional agent(s) used to treat a nerve injury can be administered at the same time or independently. For example, the additional agent(s) used to treat a nerve injury can be formulated into a thermoresponsive composition containing 4-AP and/or one or more derivatives of 4-AP to form a single composition. In some cases, a thermoresponsive composition described herein can be administered first, and the one or more additional agents used to treat a nerve injury can be administered second, or vice versa.

In some cases, the severity of the nervy injury present within a mammal and/or the severity of one or more symptoms of the nerve injury being treated can be monitored. Any appropriate method can be used to determine whether or not the severity of the nervy injury present within a mammal and/or the severity of one or more symptoms of the nerve injury is reduced. For example, physical examinations, electromagnetic fields, functional nerve electrical stimulation techniques, electromyography, nerve conduction studies, and/or magnetic resonance imaging can be used to assess the severity of the nervy injury present within a mammal and/or the severity of one or more symptoms of the nerve injury.

In some cases, methods described herein can include administering to a mammal (*e.g.,* a mammal having a skin wound) one or more additional agents used to treat a wound (*e.g.,* one or more agents in addition to a composition described herein such as a composition containing 4-AP and/or one or more derivatives of 4-AP). The one or more additional agents used to treat a wound (*e.g.,* a skin wound or an internal wound) can include any appropriate agent(s) used to treat a wound. In some cases, an agent that can be used to treat a wound can be an antibiotic. In some cases, an agent that can be used to treat a wound can be bacteriostatic. In some cases, an agent that can be used to treat a wound can be an antiseptic. In some cases, an agent that can be used to treat a wound can be an antimycotic. Examples of agents that can be used to treat a wound include, without limitation, penicillin, erythromycin, amoxicillin, methicillin, chlorhexidine gluconate, chloroxylenol, hydrogen peroxide, iodine, and silver (e.g., nanocrystalline silver, silver sulfadiazine, colloidal silver). In cases where a mammal having a wound is treated with a composition described herein and is treated with one or more additional agents used to treat a wound, the additional agent(s) used to treat a wound can be administered at the same time or independently. For example, the additional agent(s) used to treat a wound can be formulated into a composition containing 4-AP and/or one or more derivatives of 4-AP to form a single composition. In some cases, a composition described herein can be administered first, and the one or more additional agents used to treat a wound can be administered second, or vice versa.

In some cases, the healing of the wound (*e.g.,* a skin wound or an internal wound) present within a mammal being treated can be monitored. Any appropriate method can be used to determine the healing of the wound present within a mammal. For example, visual inspection, physical examinations (*e.g.,* for joint tenderness, swelling, redness, and flexibility), and/or imaging tests (*e.g.,* X-rays, MRI, ultrasound, computed tomography (e.g., computed tomography with or without angiography), radiographic angiography, and/or magnetic resonance angiography) can be used to assess the healing of the wound present within a mammal.

The invention will be further described in the following examples, which do not limit the scope of the invention described in the claims.

## EXAMPLES

*Example 1: 4-AP as a diagnostic agent in differentiating Traumatic peripheral nerve injuries (TPNI) based on axonal continuity*

**Materials and Methods:**

*Administration of i.p. 4-AP*

4-Aminopyridine was from Sigma-Aldrich. 4-AP stock was prepared in saline and i.p. dose of 150 μg/kg was

administered in rats. HPLC analysis of rat serum samples was performed at different time points and for different 4-AP doses and it was found that 150 μg/kg 4-AP dose in SD rat leads to tolerable 4-AP concentration in serum (Figure 1).

*HPLC analysis of serum samples*

Circulating level of 4-AP in rat serum sample was evaluated using ABSciex 4000 Q Trap mass spectrometry coupled with a Waters Acquity UPLC separation (UPLC/MS/MS) system in the Mass Spectrometry Core Facility in College of Medicine of Penn State University.

4-AP-d4 was used as an internal standard. The multiple reaction monitoring mode (MRM) was used to analyze and quantify 4-AP and 4-AP-d4, with the transitions of m/z 95>78 for 4-AP and 99>82 for 4-AP-d4. 4-AP was quantified by using a standard curve which was constructed by plotting the ratio of the peak area of 4-AP to the peak area 4-AP-d4 versus 4-AP concentration (0.01 μM -20 μM). All peaks were integrated and quantified by ABSciex Multiquan 2.1 software.

*Formulation and application of (4-AP)-PLGA-PEG at nerve crush site*

Poly(lactide-co-glycolide)-b-Poly(ethylene glycol)-b-Poly(lactide-co-glycolide) 1700-1500-1700Da (LA:GA 15:1 (94%/6% LA/GA) was from PolySciTech.

All of the chemicals were used without further purification. 20% weight per volume solution was made using 1X phosphate-buffered saline (PBS) and PLGA-PEG block copolymer. A known amount of gelling solution was then vortexed with 4-AP to achieve the desired concentration. To locally administer the formulation, (4-AP)-PLGA-PEG was pipetted directly onto the sciatic nerve crush site.

*Animals*

350-400 grams male Sprague-Dawley (SD) rats (Charles river laboratories) were used in this study.

*Muscle tension measurement immediately after sciatic nerve crush/transection injury*

Sciatic nerve innervates triceps surae muscle and thus sciatic nerve conduction can be measured in terms of triceps surae muscle contraction. As shown in this experimental setup (Figure 2) sciatic nerve was electrically stimulated proximal to crush site, and triceps surae muscle tension was measured by force transducer (FT10; Grass Instruments).

Briefly, Sprague-Dawley (SD) rats were anaesthetized with 3-4% isoflurane, the trachea was cannulated, and the lungs were mechanically ventilated (Harvard Apparatus) with oxygen balanced vaporized 2% isoflurane. The sciatic nerve and triceps surae muscles were exposed by blunt dissection and the calcaneal tendon was severed and linked with braided fishing line to a force transducer. A shielded stimulating electrode was placed under sciatic nerve and injury was performed distal to a stimulating electrode. Sciatic nerve underwent a standardized crush injury with blunt forcep (Miltex #18-1107) and jig or laceration injury with fine scissors. Sciatic nerve crush injury was done 3 mm proximal to trifurcation. The triceps surae muscles were contracted for 10 seconds at 40 Hz, 0.1 ms pulse, and a voltage two times above the motor threshold. The muscle tension was measured before injury, after crush or laceration, and 30 minutes after i.p. injection of saline or 150 μg/kg 4-AP, or local (4-AP)-PLGA-PEG treatment.

*Muscle tension measurement 3 days post sciatic nerve crush injury*

SD rats were anaesthetized in an induction chamber with isoflurane utilizing an induction dose of 3-5% balanced with oxygen. The area surrounding the site of surgical incision was shaved with a mechanical clipper. Then the skin was cleaned with 70% ethanol and betadine using sterile gauze. Ophthalmic lubricant was applied to the eyes to prevent corneal abrasion. Isotonic saline was administered subcutaneously to prevent dehydration during the procedure. A heat lamp was utilized to maintain the animal's body temperature. Surgery was done on a sterile blue surgical pad. Anesthesia was maintained with 1-2% isoflurane balanced with oxygen supplied via a nose cone. Surgery was done using instruments sterilized with hot glass beads.

The sciatic nerve was exposed by 2cm longitudinal incision along the posterior border of the thigh from greater trochanter to knee. The biceps femoris muscles were separated by blunt dissection to expose popliteal fossa and sciatic nerve. Sciatic nerve crush injury was done 3 mm proximal to trifurcation. Crush injury was done using blunt forcep (Miltex #18-1107) and jig for 5 seconds. The segment of the crushed nerve corresponds to the 2 mm zone at the tip of the Miltex forcep. After the surgery, the muscle was sutured together with 6-0 monofilament and skin with 2-0 silk suture. Triple antibiotic cream was applied over the area as prophylactic against infection. Isoflurane was discontinued while still under supplemental oxygen with the nose cone until the rat awakens 1 mg/kg buprenorphine was given subcutaneously for

pain management.

Muscle tension analysis in these animals was performed post 3 days of surgery. On the 3rd day rats were anaesthetized in an induction chamber with isoflurane as described above. The trachea was cannulated, and the lungs were mechanically ventilated (Harvard Apparatus). Anesthesia was maintained with 2% isoflurane. The sciatic nerve was re-exposed by cutting the sutures joining skin and muscle. The calcaneal tendon was severed and linked with braided fishing line to a force transducer (FT10; Grass Instruments). A shielded stimulating electrode was placed under the sciatic nerve proximal to crush injury. The triceps surae muscles were contracted for 10 seconds at 40 Hz; 0.1 ms pulse and minimum stimulating voltage (equivalent to the motor threshold). This initial measured muscle tension corresponds to muscle tension 3 days post crush, which was basal muscle tension reading for these experiments. Rats were administered with i.p. saline (0.9%) and muscle tension was measured post 30 minutes. The rats were then treated with i.p. 150 μg/kg 4-AP following which muscle tension was measured 30 minutes post-4-AP treatment. To evaluate the effect of local 4-AP, (Sal)-PLGA-PEG was applied at the site of crush injury on the left sciatic nerve and (4-AP)-PLGA-PEG was applied at the site of crush injury on the right sciatic nerve.

*Data analysis*

All bar graph results are presented as means ± SEM. Data were analyzed using GraphPad PRISM 7 (GraphPad Software, San Diego, CA, USA) and statistical analysis was performed using one-way ANOVA followed by Tukey's post-hoc test for multiple comparisons. A P value < 0.05 was considered to be statistically significant.

**Result**

Functional recovery after traumatic nerve injury depends on axonal continuity. To evaluate the effect of 4-AP in distinguishing axonal continuity in TPNI, two different nerve injuries and two distinct time points for the study were used. The two different nerve injuries were nerve crush injury where axonal continuity was retained and nerve transection where axonal continuity was lost. Evaluation of nerve function after traumatic nerve injury in the clinical setting may be done within weeks or months of injury. Therefore, two different time points were used, immediately after injury and 3 days post-injury.

While studying the effect of 4-AP immediately after nerve crush injury, it was found that sciatic nerve crush injury completely abolished the response to electrical stimulation (2100 g muscle tension before the injury; no muscle tension post crush) (Figure 3A and C). Single-dose of 4-AP 150μg/kg i.p. (900 g muscle tension) and local 4-AP (1700 g muscle tension) both significantly restored muscle response to electrical stimulation (Figure 3A and C). Muscle response was increased by 800% and 1600 % over a crush by systemic and local 4-AP treatment respectively (Figure 3B and D). Similarly, AUC and peak muscle tension were significantly increased by systemic (Figure 3E and F) and local 4-AP treatment (Figure 3G and H).

It was also found that restoration of nerve conduction upon 4-AP treatment does not occur in nerve transection (Figure 4A and B). This suggests that 4-AP improves nerve conduction and thus muscle response upon electrical stimulation only when there is axonal continuity.

To evaluate the effect of 4-AP late after TPNI, the effect of 4-AP was studied 3 days post nerve crush injury. For these experiments, a stimulation voltage far less than that used for maximum contraction was used. This resulted in minimal to no response to electrical stimulation and thus minimal muscle tension 3 days post-injury (Figure 5A and C).

Single-dose of 4-AP 150 μg/kg i.p. (250 g muscle tension) and local 4-AP (500 g muscle tension) both significantly increased muscle response to electrical stimulation (Figure 5A and C) even with lower stimulation voltage. Muscle response was increased by 80% and 250% over a crush by systemic and local 4-AP treatment, respectively (Figure 5B and D). Similarly, AUC and peak muscle tension were significantly increased by systemic (Figure 5E and F) and local 4-AP treatment (Figure 5G and H).

*Example 2: (4-AP)-PLGA-PEG as a Local Treatment for Acute Peripheral Nerve Injury*

**Methods**

*(4-AP)-PLGA-PEG thermogel formulation*

4-AP was incorporated into a PLGA-PEG block copolymer as shown in the table below.

| Component | Function | Details |
|---|---|---|
| **4-Aminopyridine (4-AP)** | Active ingredient | FDA-approved for MS |

(continued)

| Component | Function | Details |
|---|---|---|
| **Poly(lactic-co-glycolic acid) (PLGA)** 1700 Da 15:1 LA:GA | Hydrophobic component | Biodegradable, biocompatible FDA-approved polymer for drug loading |
| **Polyethylene glycol (PEG)** 1500 Da | Hydrophilic component | FDA-approved hydrophilic polymer for injections Easily excreted |

*(4-AP)-PLGA-PEG thermogel properties*

Thermogelation was confirmed *in vitro* (Figure 6), and the formulation structure was validated with proton nuclear magnetic resonance spectroscopy (H-NMR). All the spectra were performed at 600 mHz in a solvent of 1x PBS and 5% $D_2O$. Between 1.6 and 2.7 is usually an H next to sp2 carbon. Signal at 3.67 is usually H next to electronegative atom, in this case O. Signal at approximately 4.7 is from $D_2O$ quench which results in $H_2O$/HOD peak.

*Release of 4-APfrom (4-AP)-PLGA-PEG thermogel*

A known amount of formulation was placed into tubes incubated at 37°C along with 1x PBS to serve as release media. Release media was sampled at various timepoints and concentration was measured using ultraviolet-visible spectrophotometry to study 4-AP release over time.

*Nerve crush injury model*

Anesthetized mice (10-week-old male C57BL6) underwent sciatic nerve crush injury using a pressure-gauge tethered forceps of 30-second duration. Immediately after inducing the crush injury, (4-AP)-PLGA-PEG was placed onto the crush site (Figure 12) at a dose of 1.4 mg/kg. Concentration of 4-AP was optimized to ensure immediate *in vivo* gelation once the formulation was placed onto the nerve site. For pharmacokinetic studies, blood was collected at various timepoints and serum 4-AP levels were measured using high performance liquid chromatography. In some experiments, systemic 4-AP, instead of (4-AP)-PLGA-PEG, was administered at a dose of 2 mg/kg.

**Results**

The ability of a (4-AP)-PLGA-PEG formulation to undergo solution-gel transition was evaluated *in vitro.* At an ambient temperature of less than 25°C, both polymers dissolved in solution and the solution could be loaded with drug (Figure 6). The hydrophobic PLGA segments formed associative crosslinks and the hydrophilic PEG segments allowed the copolymer molecules to stay in solution. At lower temperatures, hydrogen bonding between hydrophilic PEG segments and water molecules dominated the aqueous solution, resulting in dissolution in water. As the temperature increased, the hydrogen bonding became weaker, while hydrophobic forces among the PLGA segments were strengthened, leading to solution-gel transition. Upon warming to 25-30°C a thermogel appearance start to form, and above 30°C, a high viscosity gel formed (Figure 6). During the degradation of a PEG-PLGA-PEG gel, there was a preferential mass loss of PEG-rich components, and the remaining gel became more hydrophobic in an aqueous environment resulting in less water content.

H-NMR verified that 4-AP was successfully incorporated into PLGA-PEG (Figure 7). The NMR spectra of 4-aminopyridine (4-AP) is $^1$H NMR ($D_2O$): $\delta$ 8.02 (d, J=5.4 Hz), 6.69 (d, J=5.4 Hz). PLGA-PEG $^1$H NMR ($D_2O$): 3.68 (s), 1.60-1.50 (m), 1.46-1.38 (m), 1.31 (d, J=7.0 Hz). However, after mixing of 4-AP with PLGA-PEG at room temperature, the $^1$H NMR ($D_2O$): $\delta$ 7.94 (d, J=6.4 Hz), 6.83 (d, J=6.4 Hz), 3.68 (s), 1.46-1.38 (m), 1.29 (dd, J=6.8 Hz, 0.8Hz). These data reveal the chemical shifts of the mixture at both aromatic protons of 4-AP from $\delta$ 8.02 to $\delta$7.94 and $\delta$6.69 to $\delta$6.83 respectively. It was also observed that PLGA-PEG aliphatic region was changed from $\delta$1.31 to $\delta$1.29 after mixing. These chemical shifts changes indicated that the chemical environment have changed for 4-AP when mixed with PLGA-PEG.

The ability of 4-AP to be released from (4-AP)-PLGA-PEG thermogel was evaluated (Figure 8). Cumulative *in vitro* release of 4-AP showed supratherapeutic release in the first day with sustained release thereafter (n=3). Cumulative release was proportional to amount of 4-AP loaded. Moreover, the formulation continued to release 4-AP for over 3 weeks *in vitro* as seen in Figure 8.

Additional properties of the (4-AP)-PLGA-PEG thermogel were also evaluated *in vitro.* A dynamic temperature

sweep at 10 rad/s and 3% strain from 10 to 40°C, with a heating rate of 0.5°C/minute was performed to evaluate the effect of 4-AP concentration on gelation temperature. The gelation temperature increased with drug concentration, and no gelation was observed at 10 μg/μL (Figure 9A and 9B). A dynamic time sweep at 10 rad/s and 3% strain from 25 to 37°C, with a heating rate of 0.5°C/minute was performed to evaluate the effect of 4-AP concentration on gelation time. The gelation time increased with drug concentration (Figure 10). A dynamic time sweep at 10 rad/s and 3% strain from 37 to 25°C to test reversibility was performed to evaluate whether the solution-gel transition could be reversed. It was observed that for solutions where gelation occurred (e.g., concentrations less than 10 μg/μL), gelation was fully reversible (Figure 11).

The formulation was tested in 10 week old C57BL6 mice having a nerve crush injury. (4-AP)-PLGA-PEG forms a solid gel when placed directly onto the sciatic nerve crush site. As seen in Figure 12, the gel was still present and directly on the nerve site after a week although it has begun to degrade.

Pharmacokinetic studies were also performed and are shown in Figure 13. An initial burst release of 4-AP was seen within the first day in which serum 4-AP levels peaked at 1 hour at about 75.8 ng/mL (Figure 13). Local (4-AP)-PLGA-PEG continued to release 4-AP into serum for over 2 weeks, with serum levels dropping to about 8.22 ng/mL within 1.5 - 3 hours (Figure 13), and with 4-AP levels being nearly undetectable at Day 21 (n=9) (Figure 13). Serum levels never crossed the human tolerable limit of 100 ng/mL.

Motor function and sensory function were evaluated after (4-AP)-PLGA-PEG was applied to a moderate sciatic nerve crush injury.

Figure 14 contains graphs showing that (4-AP)-PLGA-PEG enhances motor and sensory function after moderate sciatic nerve crush injury. Mice which received 4-AP-PLGA-PEG had better sciatic function at days 3, 5, 7, and 14 after injury. The treatment surpassed that of daily systemic dosing of 4-AP at the maximum dose. Similarly, grip strength was significantly improved in the thermogel group at days 5, 7, 14, 21, and 28 as compared to saline control.

These results demonstrate that (4-AP)-PLGA-PEG exhibits controlled release of 4-AP both *in vitro* and *in vivo* for up to 3 weeks. Both *in vitro* and *in vivo,* (4-AP)-PLGA-PEG demonstrated a burst release of 4-AP within the first day, with 4-AP peaking in serum at 1 hour. After the initial 4-AP burst release, 4-AP was released as a low maintenance dose for approximately 3 weeks that was within the human tolerable limit. Thus, the thermoresponsive composition can deliver safe systemic amounts of 4-AP, while delivering supratherapeutic doses locally to the injury site.

*Example 3: 4-AP as a Systemic Treatment for Wound Healing*

Dorsal skin excision wounds (5-mm diameter) were made on 7-8-week-old wild-type C57BL/6 mice (Figure 15). Mice were systemically administered saline (control) or 80 μg of 4-AP daily for 14 days. All mice were subjected to functional wound healing and tissue regeneration image-based assays at days 0, 3, 7, 10, and 14. The wound size of each mouse was measured and compared to zero day wound diameter from digital images. The percentage of wound closure was calculated by using the formula:

$$\text{Wound closure (\%)} = (\text{area of original wound} - \text{area of actual wound})/\text{area of original wound} \times 100.$$

It was observed that systemic administration of 4-AP accelerates the wound healing process compared to saline treated mice (Figure 16, Figure 17, Figure 18, and Figure 19).

These results demonstrate that 4-AP can be used as a therapeutic agent for the treatment of cutaneous wounds.

*Example 4: (4-Aminopyridine)-PLGA-PEG as a Thermosensitive and Locally Injectable Treatment for Acute Peripheral Nerve Injury*

This study demonstrates the applicability of a locally applicable 4-AP delivery system, its pharmacokinetic characteristics, and the effects on the recovery of neuromuscular function following sciatic nerve crush injury in mice. A PLGA/PEG triblock copolymer containing 4-AP was synthesized (Figure 20), and its physiochemical and thermogelling properties were examined.

**Materials and Methods**

*Animals*

Ten-week-old male C57BL/6J mice (Jackson Laboratories, Bar Harbor, Maine) weighing 25 ± 3 grams were

used.

*Block Copolymer Synthesis*

Poly(lactide-co-glycolide)-b-Poly(ethylene glycol)-b-Poly(lactide-co-glycolide) (1700-1500-1700Da, LA:GA 15:1, 94%/6% LA/GA, PolySciTech) and 4-aminopyridine (Sigma-Aldrich) were used without further purification. 4-AP was incorporated in PLGA-PEG-PLGA triblock copolymer solution (PBS, pH 7.4, polymer concentration: 20 wt%), stirred at 4°C until it was completely dissolved, and characterized via proton nuclear magnetic resonance ($^1$H NMR) to determine the composition. $^1$H NMR experiments were carried out with Bruker Avance II 600 MHz instrument equipped with TCI cryo-probe. Standard pre-saturation pulse program from Bruker pulse sequence library was used to suppress residual $D_2O$ solvent. The samples were prepared at room temperature, and experiments were performed at 298°K.

*Rheological Characterization of Copolymer Aqueous Solutions*

The small amplitude oscillatory shear experiments were performed in a Discovery Hybrid Rheometer (DHR-3) from TA Instruments. The rheometer was equipped with a 60 mm diameter stainless steel cone with a truncation gap of 28 μm and cone angle 1°. The cone was installed in the top part of the rheometer which also hosted the motor and transducer for both torque and normal force. The bottom plate was constituted by a Peltier element used to control the temperature with an accuracy of ± 0.1°C.

1.2 mL of solution was typically poured onto the bottom plate at 20°C to minimize evaporation. The loading was followed by a trimming stage where the excess sample was removed before leading the cone to the measuring gap (μm). Once in the measuring position, the solution was surrounded by an organic non-interacting oil to avoid water evaporation. The oil viscosity was much lower than the aqueous solution so that the torque signal was almost unaffected by the presence of the oil. Rapid experiments with no oil at room temperature corroborated the results obtained with oil.

The linear viscoelastic limits were probed by means of shear strain amplitude sweep experiments at 10 rad/second and at the temperature of interest. The range of temperatures explored was 10-40°C. A shear strain of 0.03 strain units ensured the linear viscoelastic regime for the whole temperature window. Temperature sweep tests were all performed at 10 rad/second and 0.03 strain units, from 10°C to 40°C with a heating rate of 0.5°C/minute. The thermal expansion of the measuring systems and the resulting change of gap was automatically taken into account by the instrument. This ensured a constant measuring gap regardless of the temperature used in the experiment. The time evolution of the shear response of the solutions was monitored by time sweep experiments. Even in this case, the frequency was 10 rad/second and the shear strain 0.03 strain units. Frequency sweep experiments were performed at 0.03 strain units, well within the linear viscoelastic regime, at 25°C and in the range of frequencies 100 - 0.1 rad/second.

The monitored rheological functions were: the storage modulus G' (elastic contribution to the material response), the loss modulus G" (the viscous contribution to the material response), and the ratio G"/G', the loss factor tan(δ), with δ being the phase shift between the sinusoidal input exerted by the instrument and the output, the material response. tan(δ) was used as a parameter to detect gelation.

*In Vitro Drug Release*

Polymer solutions containing varying concentrations of 4-AP (2 μg/μL, 5 μg/μL, and 10 μg/μL) were transferred to 1.5 mL test tubes, and the samples were incubated in a water bath at 37°C to convert them to physical hydrogels. Next, PBS (pH 7.4, 37 °C) was added to each test tube as release media, and the samples were left in the water bath at 37°C for 30 days. At designated timepoints, release media was extracted from the tubes and replaced with the same amount of fresh PBS to maintain the sink condition. 4-AP amount released into the media was measured with ultraviolet-visible spectrophotometry (Thermo Scientific NanoDrop One Spectrophotometer) to determine cumulative drug release over time.

*In Vivo Drug Release in Mice*

Mice (n=9) were given (4-AP)-PLGA-PEG at a body weight-adjusted dose of 1.4 mg/kg. Serum was retro-orbitally collected at various timepoints, and serum 4-AP concentration was determined using high-performance liquid chromatography (HPLC) to ensure that the serum levels in mice are within the human tolerable limit of 100 ng/mL. Circulating levels of 4-AP in mouse serum samples were determined using ABSciex 4000 Q Trap mass spectrometry (MS) coupled with a Waters Acquity ultra performance liquid chromatography (UPLC) separation system (UPLC/MS/MS). 4-AP-d4 was used as an internal standard. The multiple reaction monitoring mode was used to analyze and quantify 4-AP and 4-AP-d4, with the transitions of m/z 95>78 for 4-AP and 99>82 for 4-AP-d4. 4-AP was quantified by using a standard curve, which was constructed by plotting the ratio of the peak area of 4-AP to the peak area 4-AP-d4 versus

4-AP concentration (0.01 μM - 20 μM). All peaks were integrated and quantified by ABSciex Multiquan 2.1 software. To assess *in vivo* biodegradation of (4-AP)-PLGA-PEG, the sciatic nerves were surgically exposed at weekly timepoints to observe location and mass of the gel.

*Local (4-AP)-PLGA-PEG Injection under Ultrasound Guidance*

To verify if (4-AP)-PLGA-PEG could be locally and non-invasively administered to a targeted nerve injury site, ultrasonic guidance was used for local (4-AP)-PLGA-PEG injection. Briefly, mice (n=5) were anesthetized with 5% isoflurane in an induction chamber and fur over the hindlimb was shaved using an animal clipper. Skin was prepped in an aseptic fashion, and the mice were placed on the mouse-handling stage in a prone position. A Vevo 3100 (Visual Sonics, Canada) micro-ultrasound machine with a 40 MHz probe was used to image longitudinal sections of the proximal hindlimb. The probe was manually used to scan the hindlimb proximally and distally to ensure identification of the sciatic nerve. A 20 G needle was manually inserted into the hindlimb to first confirm its presence and then positioned over the sciatic nerve. 20 μL (4-AP)-PLGA-PEG was then injected onto the sciatic nerve. Following injection, the sciatic nerve was surgically exposed to confirm placement and adherence of the gel on the nerve, and the mouse was sacrificed.

*Mouse Model of Sciatic Nerve Crush Injury*

Sciatic nerve crush injury was performed with pressure-gauge-tethered forceps as described elsewhere (see, e.g., Elfar et al., J. Bone Joint Surg. Am., 90: 1644-1653 (2008)). Briefly, after intraperitoneal (IP) ketamine (100 mg/kg)/xylazine (10 mg/kg) anesthesia, the right hindlimb was shaved and prepared with alcohol swabs and povidone-iodine (Betadine). Under a binocular microscope (Model PZMIII, World Precision Instruments), a lateral skin incision (~2.5 cm) was made along the length of the femur, and the sciatic nerve (SN) was bluntly exposed through the iliotibial band. Crush injury was performed ~3 mm proximal to the SN trifurcation using calibrated forceps (5 mm tip width; 18-1107, Miltex Instruments) for 30 second duration at a pressure of 4.4 MPa. Skin was closed via surgical staples, and post-operative slow release buprenorphine (0.05 mg/kg) was given subcutaneously as an analgesic. The experimental animals (n=5/group) were randomly assigned to Sham (normal saline, 0.1 mL/mouse), SN crush injury with saline (normal saline, 0.1 mL/mouse), SN crush injury with systemic 4-AP (4-AP, 10 mg/kg), SN crush injury with PLGA-PEG vehicle (PLGA-PEG, ~20 μL on sciatic nerve injury site), and SN crush injury with (4-AP)-PLGA-PEG ((4-AP)-PLGA-PEG, 1.4 mg/kg, ~20 μL on sciatic nerve injury site) groups. Systemic 4-AP was given intraperitoneally (IP) once daily for 28 days. Local groups received (4-AP)-PLGA-PEG immediately after crush injury. Post-injury functional recovery was assessed by walking track analysis (WTA), sensory nerve test (SNT), and grip strength test on days 1, 3, 7, 14, 21, and 28. The animals were euthanized on post-injury day 28 to harvest sciatic nerves (flash-frozen in liquid nitrogen) for immunohistochemical analysis.

*Sciatic Function Index (SFI)*

To evaluate *in vivo* global motor function recovery, sciatic function index (SFI) was determined by WTA as described elsewhere (see, e.g., Elfar et al., J. Bone Joint Surg. Am., 90: 1644-1653 (2008)). Briefly, mice were trained to walk freely along a 77 cm by 7 cm corridor lined with paper, and individual footprints of the hindlimbs were obtained before surgery as baseline and on post-surgery days 1, 3, 7, 14, 21, and 28. At least three measurable footprints for each hindlimb were obtained. Two blinded observers measured three footprints per hindlimb with digital calipers. SFI was calculated using three parameters: (1) toe spread (TS, first to the fifth toe), (2) total print length (PL), and (3) intermediate toe spread (IT, second to the fourth toe) and the following formula: $SFI = -38.3 \{(EPL-NPL)/NPL\} + 109.5 \{(ETS-NTS)/NTS\} + 13.3\{(EIT-NIT)/NIT\} - 8.8$, where E is for experimental (injured) and N is for normal (contralateral uninjured) sides.

*Hindlimb Grip Strength Test*

A grip strength meter (BIO-GS3; Bioseb-In Vivo Research Instruments) was used to measure hindlimb grip strength. Briefly, the mice were restrained by holding the scruff and base of the tail. Mice were allowed to hold the grid and were gently pulled along the length of the sensor grid until the grip was released. Force value was recorded 5 times per animal to calculate the average grip strength. Attention was paid to minimize paw injury and habit formation during each trial.

*Von Frey Test*

Mice were placed in a transparent polycarbonate chamber (~10 × 10 cm) with a metallic mesh floor approximately 25 cm above the bench. Animals were acclimatized prior to testing. SNT was performed using Von Frey filament unit (NC12775-08, Touch Test® Sensory Evaluators). Briefly, the filament pressure (1 g force) was applied to the plantar surface of the hindlimb through the mesh floor, and the animal withdrawing its paw was considered a positive response. The withdrawal reflex of the hindlimb was recorded five times per animal to calculate the percent response.

*Sciatic Nerve Processing and Immunohistochemical Analysis*

Sciatic nerve processing and immunohistochemical staining were performed. SNs were harvested on day 28 post-injury from the ipsilateral hindlimbs of mice. Nerves were fixed in 4% paraformaldehyde (PFA) solutions overnight, washed with 70% alcohol, and embedded in paraffin. A microtome (Model RM2235, Leica) was used to cut serial 5 μm longitudinal sections from the paraffin blocks. Tissue sections were deparaffinized, serially rehydrated with xylene and ethanol, and antigen retrieval was performed using 10 mM sodium citrate buffer (pH 6.0). Permeabilization and nonspecific binding blocking were done using 1% Triton X-100 and 5% goat serum, respectively. Primary antibody staining was performed with anti-NF-H (1:1000; NB300-135, Novus biologicals) and anti-MPZ (1:1000; PZ0, Aves Labs) followed by secondary antibody incubation with Alexa Fluor 488 (1:1000; A11008, Invitrogen) and Alexa Fluor 647 (1:1000; A21449; Invitrogen). Staining without primary antibodies served as a control for non-specific fluorescence. Nuclei were counter-stained with ProLong™ Gold antifade reagent with DAPI (P36935; Invitrogen), and sections were observed under a fluorescent microscope (ZEISS Apotome 2). Stained nerve tissues were analyzed, and immunofluorescence intensity was quantified using NIH-ImageJ software.

*Statistical Analysis*

All the results were expressed as mean ± standard error (SE). For group comparison, the statistical differences of mean values were analyzed by least significant difference (LSD) t-tests, one-way, and two-way analysis of variance (ANOVA). A p value of $< 0.05$ was considered as significant.

**RESULTS**

*Characterization of 4-AP Triblock Copolymer*

Figure 21 shows the NMR spectra of 4-AP is $^1$H NMR ($D_2O$): δ 8.02 (d, J=5.4 Hz), 6.69 (d, J=5.4 Hz). PLGA-PEG $^1$H NMR ($D_2O$): 3.68 (s), 1.60-1.50 (m), 1.46-1.38 (m), 1.31 (d, J=7.0 Hz). After mixing of 4-AP with PLGA-PEG at room temperature, the $^1$H NMR ($D_2O$): δ 7.94 (d, J=6.4 Hz), 6.83 (d, J=6.4 Hz), 3.68 (s), 1.46-1.38 (m), 1.29 (dd, J=6.8 Hz, 0.8Hz). The spectra revealed that the chemical shifts of the mixture showed significant change at both aromatic protons of 4-AP from δ8.02 to δ7.94 and δ6.69 to δ6.83, respectively. In addition, the PLGA-PEG aliphatic region was changed from δ1.31 to δ1.29 after mixing. These findings of chemical shift changes indicate that the chemical environment changes for 4-AP when mixed with PLGA-PEG.

*Thermogellability of Copolymer Aqueous Solutions*

Both PLGA-PEG-PLGA and PLGA-PEG-PLGA polymers with 4-AP were soluble in PBS at room temperature and underwent solution-to-gel (sol-gel) transitions with increasing temperature. Figure 22A shows the change in modulus of PLGA-PEG-PLGA and PLGA-PEG-PLGA polymers with varying concentrations of 4-AP as a function of temperature. At low or room temperature, the loss modulus G" was greater than the storage modulus G' reflecting a solution-free flowing phase. An abrupt increase in modulus was observed along with the formation of physical hydrogels as the temperature increased. The sol-gel transition temperatures (Tgel), the crossover point of G" and G', of PLGA-PEG and 2 μg/μL (4-AP)-PLGA-PEG were 31.3°C and 32°C, respectively (Figure 22A). The gel window of the polymer system covered body temperature, indicating that the thermogel is suitable for biomedical applications. Tgel increased with increasing 4-AP concentration (Figure 22A). Figures 22C and 22D show the change in moduli as a function of time. Sol-gel transition occurred in 19 seconds (Figure 22C), and this transition was fully reversible and occurred in 1228 seconds (Figure 22D).

*In Vitro Release of 4-AP*

The *in vitro* release profiles of 4-AP from PLGA-PEG-PLGA were evaluated at varying concentrations (2 μg/μL, 5 μg/μL, and 10 μg/μL). Figure 23 shows the cumulative amounts of 4-AP released over 28 days. The release of 4-AP from PLGA-PEG exhibited a burst release within the first day followed by an ultra-low maintenance dose released until day 28. Cumulative release was proportional to total loaded amount of 4-AP.

*In Vivo Release of 4-AP*

Figure 24 shows serum 4-AP levels after administration of (4-AP)-PLGA-PEG. 4-AP serum levels peaked at one hour after administration and never exceeded the human tolerable limit of 100 ng/mL known to cause adverse side effects in humans (Figure 24A). Serum 4-AP levels were nearly undetectable after 21 days of administration (Figure 24B), indicating that (4-AP)-PLGA-PEG releases systemically detectable amounts of 4-AP at a sustained rate for approximately 21 days *in vivo.*

*Ultrasound-Guided Injection of (4-AP)-PLGA-PEG and In Vivo Biodegradation*

Under ultrasound guidance, it was possible to inject (4-AP)-PLGA-PEG locally to the region of sciatic nerve (Figure 25). Based on its relative density to local tissue, (4-AP)-PLGA-PEG could be visualized on the sciatic nerve after injection (Figure 25D). To determine whether the gel was correctly administered to the sciatic nerve, the mouse hindlimb was exposed to observe the gel location and integrity after injection. As shown in Figure 26, the gel remained directly on the sciatic nerve injury site for over 21 days although its overall mass decreased due to controlled polymeric degradation.

*In Vivo Efficacy of (4-AP)-PLGA-PEG*

Systemic 4-AP administration can improve motor functional recovery after sciatic nerve crush injury (see, e.g., Tseng et al., EMBO Mol. Med., 8: 1409-1420 (2016)), but the effects of a novel locally applicable 4-AP thermogel on the injury site as well as its effects on the functional outcomes after sciatic nerve injury were unknown. The findings presented herein demonstrate that (4-AP)-PLGA-PEG can improve post-injury functional recovery (SFI) on days 1, 3, 7, and 14 (Figure 27A,*$p < 0.05$) compared to saline, PLGA-PEG, and systemic 4-AP groups. (4-AP)-PLGA-PEG also improved grip strength on post-injury days 3, 7, 14, 21, and 28 post-injury compared to all other treatment groups (Figure 27B,*$p < 0.05$). Since grip strength examines neuromuscular function, this suggests that (4-AP)-PLGA-PEG treatment improves volitional muscle strength in proportion to improved global motor function indicated by SFI following crush injury.

Similar to motor functional results, (4-AP)-PLGA-PEG treatment also significantly improved withdrawal reflex (percent response to filament) as compared to the saline group (Figure 27C,*$P < 0.05$) on post-injury days 1, 3, 7, 14 and 21. These findings demonstrate that (4-AP)-PLGA-PEG can improve sensory nerve function after crush injury. Thus, local treatment at a dose well below the systemic dose resulted in reproducible, clinically relevant improvements in global and sensory function recovery after sciatic nerve injury.

*Effects of (4-AP)-PLGA-PEG on Peripheral Nerve Remyelination*

To determine the effect of 4-AP thermogel on nerve remyelination, transverse sectioned nerves were stained for neurofilament-H (NF-H), a structural component of large myelinated axons, and myelin protein zero (MPZ), the most abundant protein in myelin, using immunohistochemistry. Significant increases in myelin were observed by immunofluorescence for both NF-H and MPZ in (4-AP)-PLGA-PEG-treated nerves compared to saline-treated nerves on post-injury day 28 (Figure 28B and Figure 28C). (4-AP)-PLGA-PEG-treated nerves contained approximately 2.5 fold more MPZ and 7.3 fold more NF-H protein in the lesion area than seen in nerves from vehicle-treated animals.

Taken together, these results demonstrate that 4-AP can be used as a local therapeutic agent and can promote motor and sensory function recovery of the limb with better preservation of the axonal myelination. These findings suggest that (4-AP)-PLGA-PEG can be used as a long-acting therapeutic for traumatic peripheral nerve injury.

## OTHER EMBODIMENTS

It is to be understood that while the invention has been described in conjunction with the detailed description thereof, the foregoing description is intended to illustrate and not limit the scope of the invention, which is defined by the scope of the claims. Other aspects, advantages, and modifications are within the scope of the claims.

Although the present invention is defined in the claims, it should be understood that the present invention can also (alternatively) be defined in accordance with the following embodiments:

1. A thermoresponsive composition comprising (a) a thermoresponsive polymer and (b) 4-aminopyridine (4-AP) or one or more derivatives of 4-AP.

2. The thermoresponsive composition of embodiment 1, wherein said thermoresponsive composition is a liquid when below a physiological temperature of a mammal and is a gel when at or above said physiological temperature

of said mammal.

3. The thermoresponsive composition of embodiment 2, wherein said thermoresponsive composition is a liquid at about 10°C to about 32°C.

4. The thermoresponsive composition of embodiment 2, wherein said thermoresponsive composition is a gel at about 32°C to about 37°C.

5. The thermoresponsive composition of any one of embodiments 1-4, wherein said thermoresponsive polymer is a copolymer.

6. The thermoresponsive composition of embodiment 5, wherein said copolymer comprises poly lactic-co-glycolic acid (PLGA) and polyethylene glycol (PEG).

7. The thermoresponsive composition of embodiment 6, wherein said PLGA has a molecular weight of about 200 to about 1900.

8. The thermoresponsive composition of embodiment 6, wherein said PEG has a molecular weight of about 1500 to about 3000.

9. The thermoresponsive composition of embodiment 6, wherein said copolymer comprises from about 3:5 PLGA:PEGto about 9:1 of PLGA:PEG.

10. The thermoresponsive composition of any one of embodiments 1-9, wherein said thermoresponsive composition comprises 4-AP.

11. The thermoresponsive composition of any one of embodiments 1-9, wherein said thermoresponsive composition comprises a derivative of 4-AP, and wherein said derivative of 4-AP is selected from the group consisting of 3,4-diaminopyridine, 3-hydroxy-4-aminopyridine, N-(4-pyridyl)-t-butyl carbamate, N-(4-pyridyl) ethyl carbamate, N-(4-pyridyl) methyl carbamate, and N-(4-pyridyl) isopropyl carbamate.

12. The thermoresponsive composition of any one of embodiments 1-11, wherein said thermoresponsive composition comprises from about 10 nM to about 1 $\mu$M of said 4-AP or said derivative of 4-AP.

13. A method for treating a nerve injury within a mammal, wherein said method comprises administering a thermoresponsive composition comprising (a) a thermoresponsive polymer and (b) 4-AP or one or more derivatives of 4-AP onto, into, around, and/or near said nerve injury within said mammal.

14. The method of embodiment 13, wherein said mammal is a human.

15. The method of any one of embodiments 13-14, wherein said nerve injury is a crush injury.

16. The method of any one of embodiments 13-15, wherein said nerve injury is in a sciatic nerve.

17. The method of any one of embodiments 13-16, wherein said thermoresponsive composition is a liquid when below a physiological temperature of said mammal and is a gel when at or above said physiological temperature of said mammal such that said thermoresponsive composition, once administered onto, into, around, and/or near said nerve injury forms a gel *in situ* within said mammal.

18. The method of embodiment 17, wherein said gel releases about 0.5 mg/kg to about 10 mg/kg of said 4-AP or one or more derivatives of 4-AP.

19. The method of any one of embodiments 13-18, wherein said gel releases said 4-AP or one or more derivatives of 4-AP for about 60 seconds to about 4 weeks.

20. A method for assessing a nerve injury within a mammal, wherein said method comprises:

(a) administering a thermoresponsive composition comprising (i) a thermoresponsive polymer and (ii) 4-AP or

one or more derivatives of 4-AP onto, into, around, and/or near said nerve injury within said mammal;
(b) administering an electrical stimulation onto, into, around, and/or near said nerve injury within said mammal;
(d) detecting the presence of absence of nerve conduction of said nerve injury;
(d) classifying said nerve injury as having axonal continuity based at least in part on the presence of said nerve conduction; and
(e) classifying said nerve injury as lacking axonal continuity based at least in part on the absence of said nerve conduction.

21. The method of embodiment 20, wherein said mammal is a human.

22. The method of any one of embodiments 20-21, wherein said nerve injury is in a motor nerve.

23. A method for treating a wound within a mammal, wherein said method comprises administering a composition comprising 4-AP or one or more derivatives of 4-AP to said mammal.

24. The method of embodiment 23, wherein said mammal is a human.

25. The method of any one of embodiments 23-24, wherein said wound is a cutaneous wound.

26. The method of any one of embodiments 23-24, wherein said wound is selected from the group consisting of after-shave wounds, abrasion wounds, diabetic wounds, bed sores, surgical wounds, anastamotic leaks, tendon gaps, muscle defects, multi-tissue disruptions, and ulcerations.

27. The method of any one of embodiments 23-26, wherein said administration is a systemic administration.

28. The method of embodiment 27, wherein said systemic administration comprises intravenous injection.

## Claims

**1.** A composition for use in a method for treating a wound within a mammal, wherein said composition comprises 4-AP or one or more derivatives of 4-AP, wherein said method comprises administering the composition to said mammal.

**2.** The composition for use of claim 1, wherein said mammal is a human.

**3.** The composition for use of claim 1 or claim 2, wherein said wound is a cutaneous wound.

**4.** The composition for use of claim 1 or claim 2, wherein said wound is selected from the group consisting of after-shave wounds, abrasion wounds, diabetic wounds, bed sores, surgical wounds, anastamotic leaks, tendon gaps, muscle defects, multi-tissue disruptions, and ulcerations.

**5.** The composition of use of claim 4, wherein the wound is an ulceration selected from the group consisting of scrotal ulcerations, buccal sores, and gastrointestinal tract ulcerations.

**6.** The composition for use of any one of claims 1-5, wherein administering the composition comprises administering the composition orally, parenterally, intraperitoneally subcutaneously, intramuscularly, or intradermally.

**7.** The composition for use of claim 6, wherein administering the composition comprises administering the composition orally, and optionally wherein the composition is a liquid, tablet, capsule, pill, powder, gel, or granule.

**8.** The composition for use of claim 6, wherein administering the composition comprises administering the composition parentally, and optionally wherein the composition comprises aqueous or non-aqueous sterile injection solutions, wherein the aqueous or non-aqueous sterile injection solutions comprise anti-oxidants, buffers, bacteriostats, or solutes.

**9.** The composition for use of any one of claims 1-5, wherein said administration is a systemic administration.

**10.** The composition for use of claim 9, wherein said systemic administration comprises intravenous injection.

**11.** The composition for use of any one of claims 1-10, wherein the composition is administered within two weeks of the mammal sustaining the wound, and optionally wherein the composition is administered within one hour to ten days of the mammal sustaining the wound.

**12.** The composition for use of any one of claims 1-11, wherein the composition is administered within two weeks of the mammal being identified as having the wound.

**13.** The composition for use of any one of claims 1-12, wherein the duration of administration can range in duration from one month to six months.

**14.** The composition for use of any one of claims 1-13 wherein the method further comprises administering an additional agent, wherein the additional agent is an antibiotic, bacteriostatic, antiseptic, or antimycotic.

**15.** The composition for use of claim 14, wherein the agent comprises penicillin, erythromycin, amoxicillin, methicillin, chlorhexidine gluconate, chloroxylenol, hydrogen peroxide, iodine, or silver.

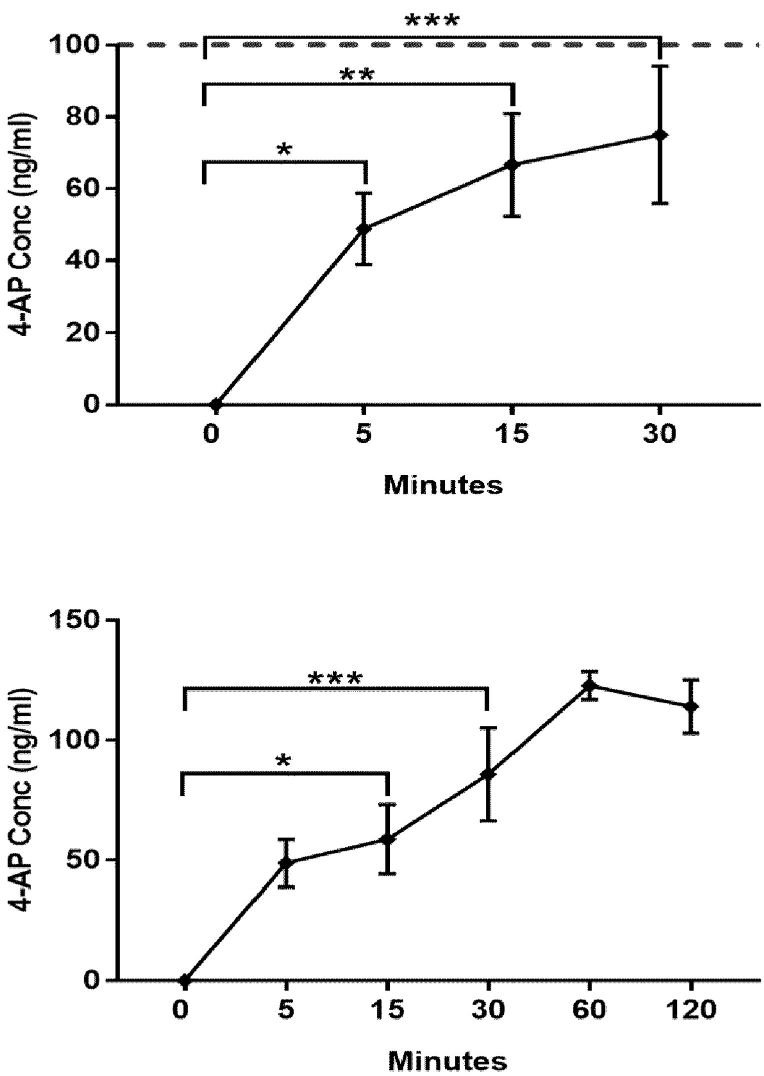
***
**
*
100
80
60
40
20
0
4-AP Conc (ng/ml)
0
5
15
30
Minutes
150
100
50
0
4-AP Conc (ng/ml)
***
*
0
5
15
30
60
120
Minutes

FIG. 1

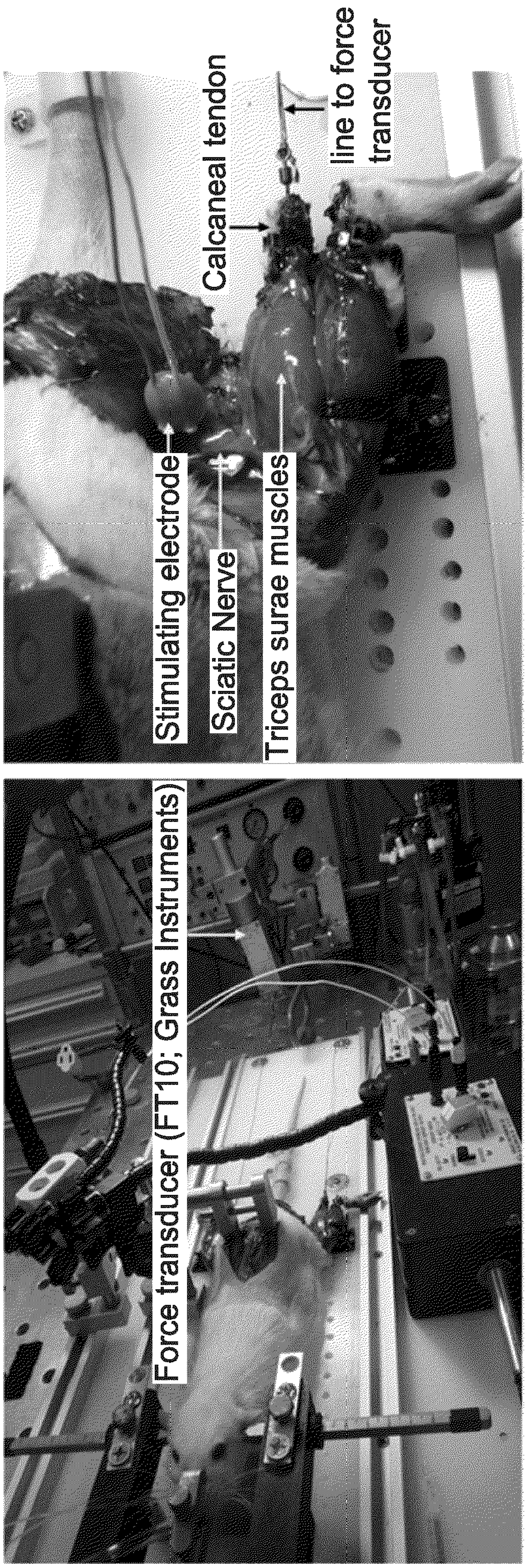
Force transducer (FT10; Grass Instruments)
Stimulating electrode
Sciatic Nerve
Triceps surae muscles
Calcaneal tendon
line to force transducer

FIG. 2

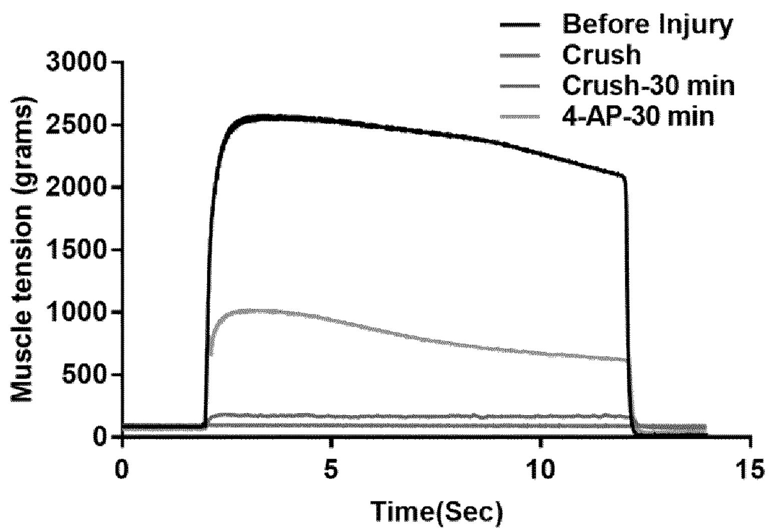
Before Injury
Crush
Crush-30 min
4-AP-30 min
Muscle tension (grams)
3000
2500
2000
1500
1000
500
0
0
5
10
15
Time(Sec)

FIG. 3A

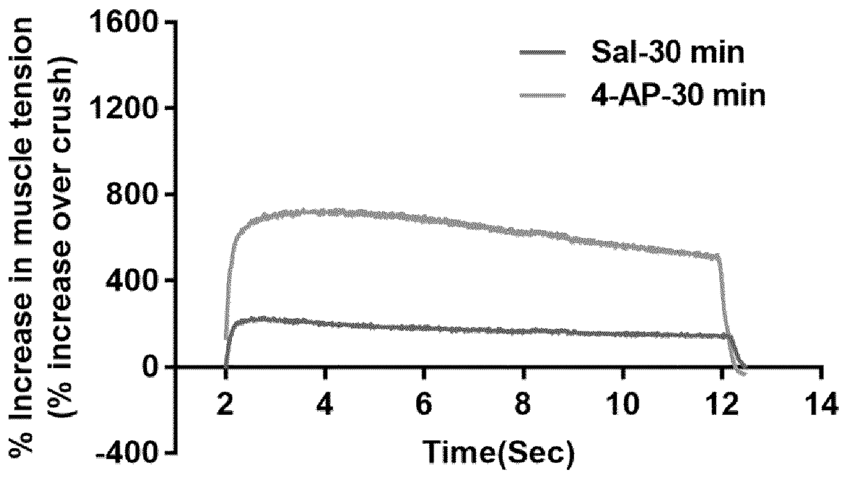
% Increase in muscle tension
(% increase over crush)
Sal-30 min
4-AP-30 min
1600
1200
800
400
0
-400
2
4
6
8
10
12
14
Time(Sec)

FIG. 3B

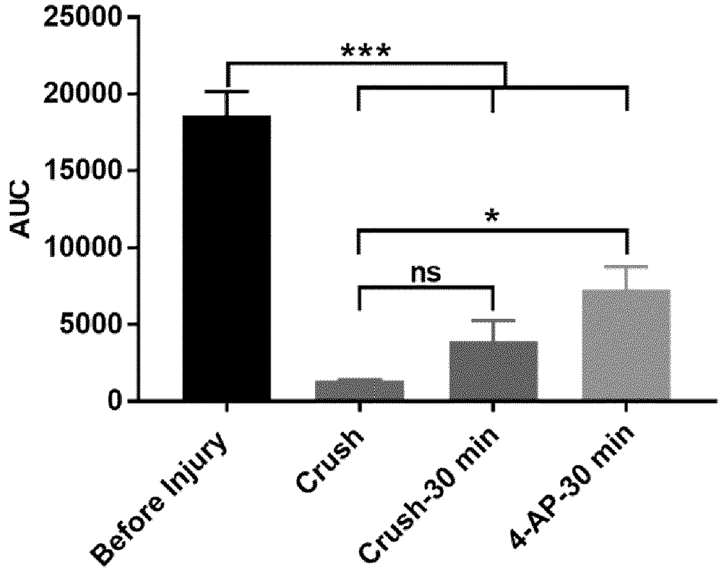
25000
20000
15000
10000
5000
0
AUC
***
*
ns
Before Injury
Crush
Crush-30 min
4-AP-30 min

FIG. 3C

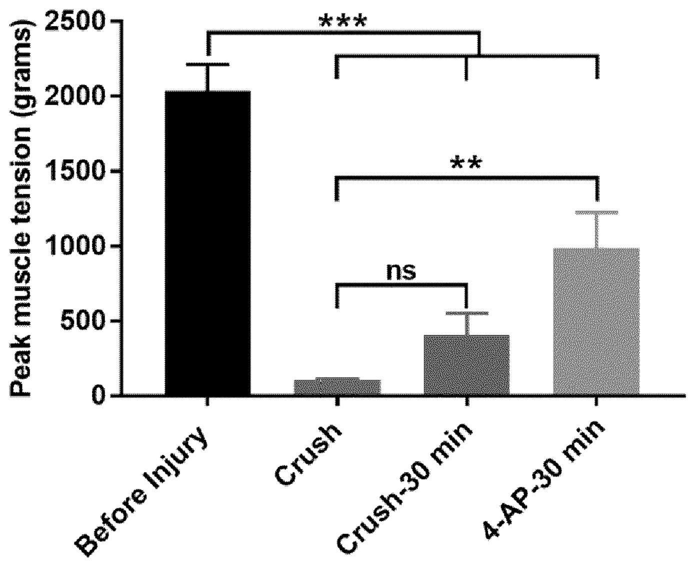
2500
2000
1500
1000
500
0
Peak muscle tension (grams)
***
**
ns
Before Injury
Crush
Crush-30 min
4-AP-30 min

FIG. 3D

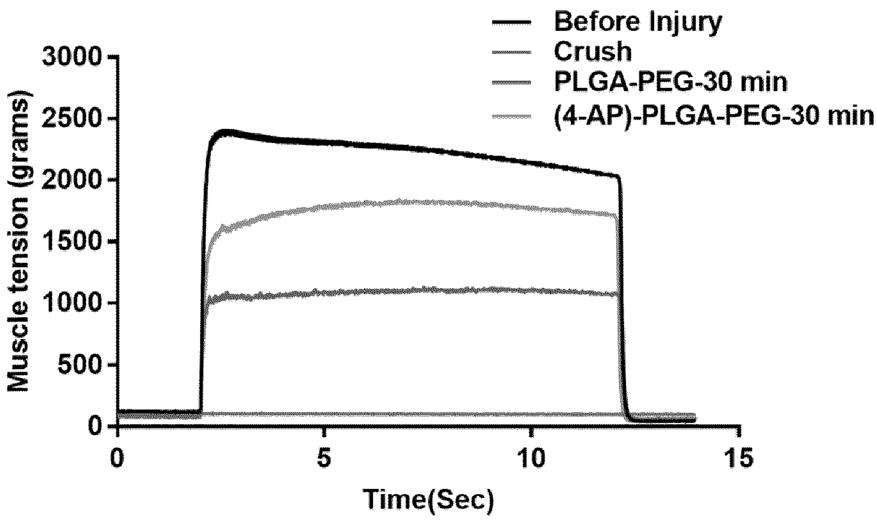
Before Injury
Crush
PLGA-PEG-30 min
(4-AP)-PLGA-PEG-30 min
Muscle tension (grams)
3000
2500
2000
1500
1000
500
0
0
5
10
15
Time(Sec)

FIG. 3E

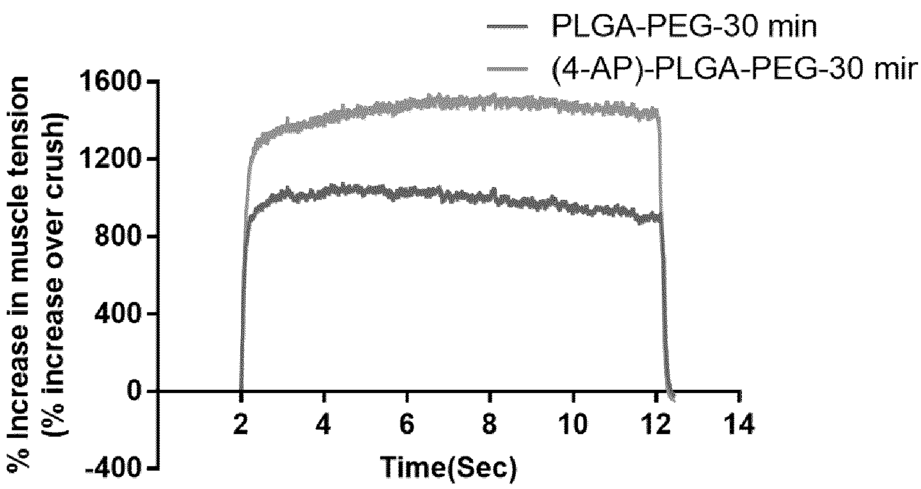
PLGA-PEG-30 min
(4-AP)-PLGA-PEG-30 mir
% Increase in muscle tension
(% increase over crush)
1600
1200
800
400
0
-400
2
4
6
8
10
12
14
Time(Sec)

FIG. 3F

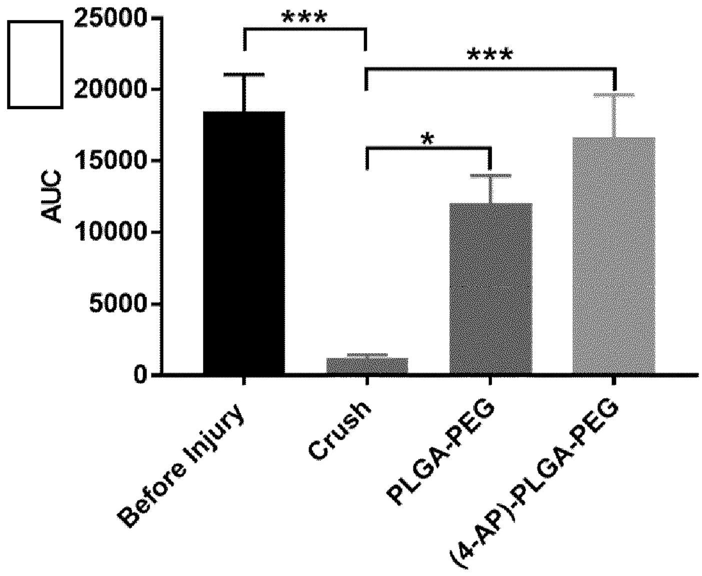
***
***
*
AUC
25000
20000
15000
10000
5000
0
Before Injury
Crush
PLGA-PEG
(4-AP)-PLGA-PEG

FIG. 3G

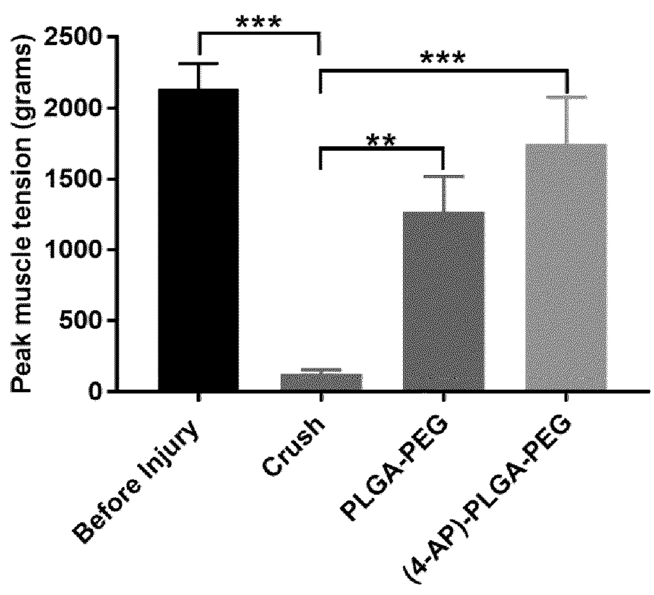
***
***
**
Peak muscle tension (grams)
2500
2000
1500
1000
500
0
Before Injury
Crush
PLGA-PEG
(4-AP)-PLGA-PEG

FIG. 3H

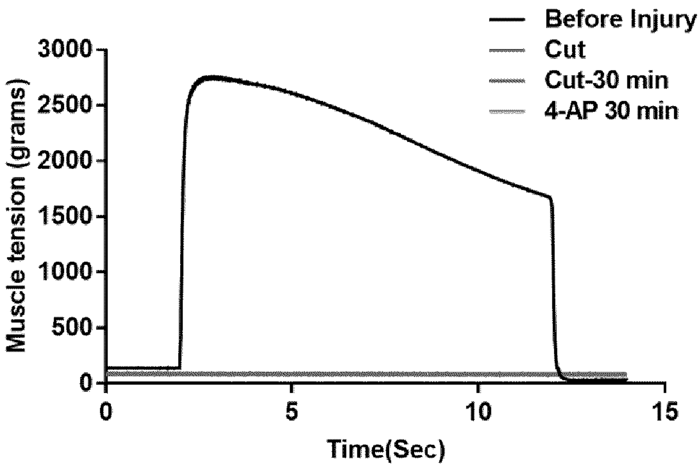
Before Injury
Cut
Cut-30 min
4-AP 30 min
Muscle tension (grams)
3000
2500
2000
1500
1000
500
0
0
5
10
15
Time(Sec)

FIG. 4A

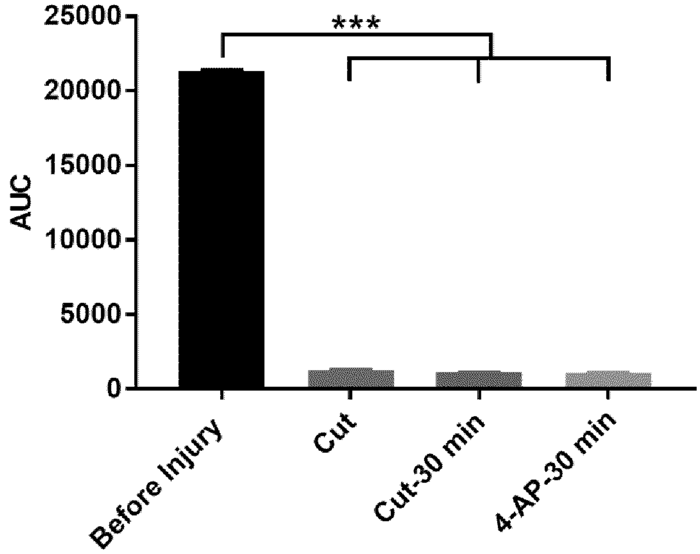
AUC
25000
20000
15000
10000
5000
0
***
Before Injury
Cut
Cut-30 min
4-AP-30 min

FIG. 4B

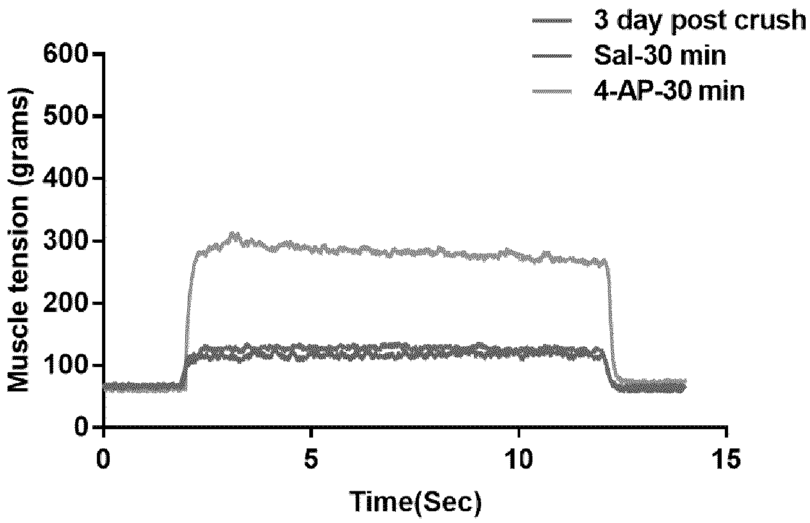
3 day post crush
Sal-30 min
4-AP-30 min
Muscle tension (grams)
600
500
400
300
200
100
0
0
5
10
15
Time(Sec)

FIG. 5A

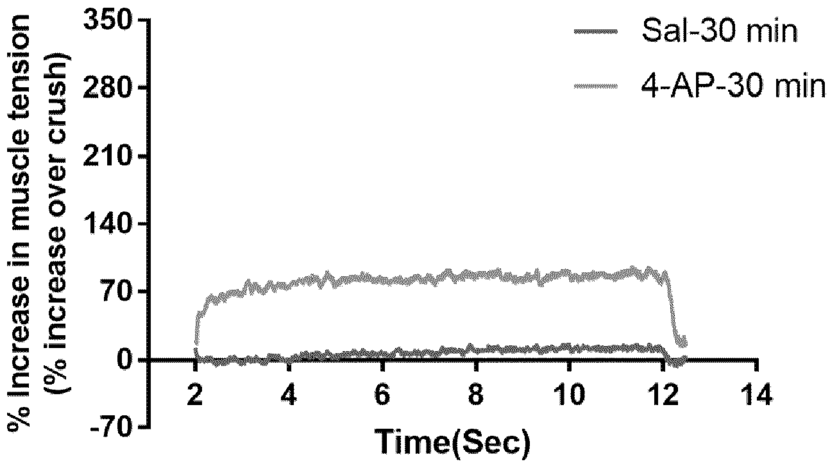
Sal-30 min
4-AP-30 min
% Increase in muscle tension
(% increase over crush)
350
280
210
140
70
0
-70
2
4
6
8
10
12
14
Time(Sec)

FIG. 5B

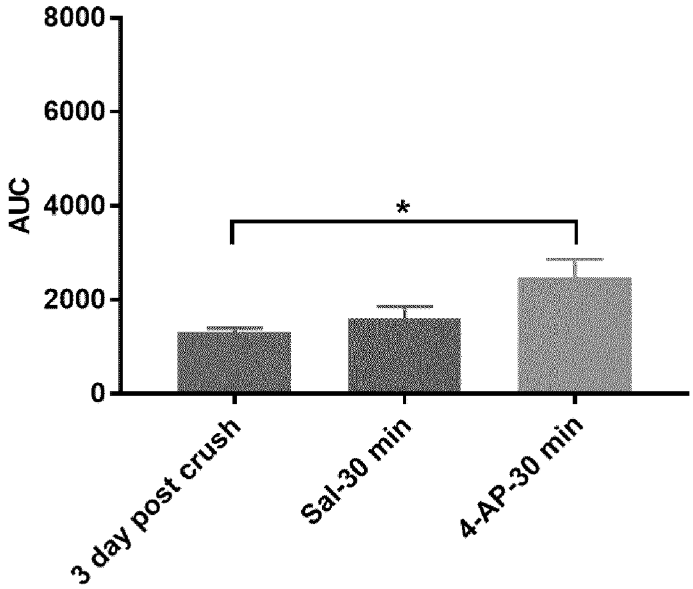
8000
6000
4000
2000
0
AUC
*
3 day post crush
Sal-30 min
4-AP-30 min

FIG. 5C

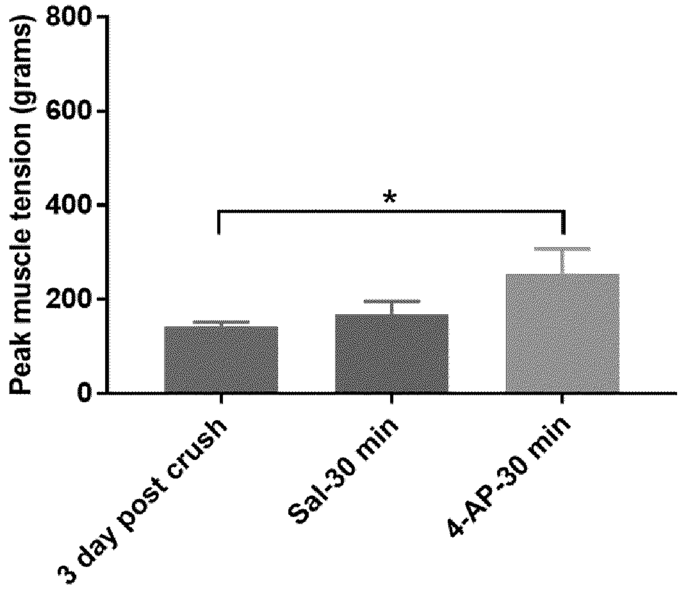
800
600
400
200
0
Peak muscle tension (grams)
*
3 day post crush
Sal-30 min
4-AP-30 min

FIG. 5D

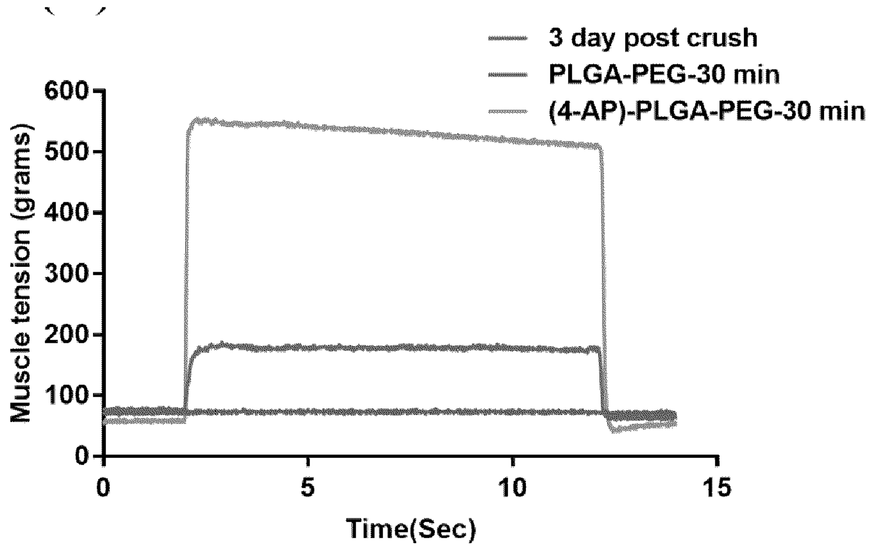
3 day post crush
PLGA-PEG-30 min
(4-AP)-PLGA-PEG-30 min
Muscle tension (grams)
600
500
400
300
200
100
0
0
5
10
15
Time(Sec)

FIG. 5E

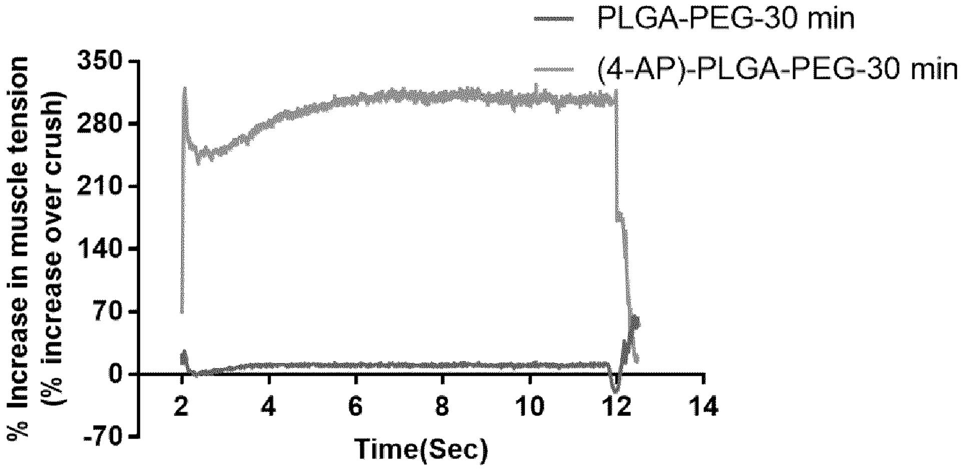
PLGA-PEG-30 min
(4-AP)-PLGA-PEG-30 min
% Increase in muscle tension
(% increase over crush)
350
280
210
140
70
0
-70
2
4
6
8
10
12
14
Time(Sec)

FIG. 5F

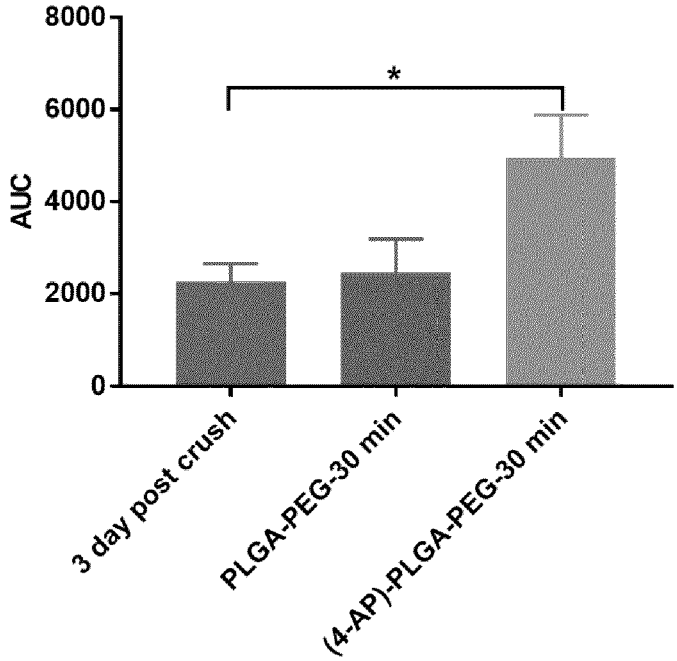
8000
6000
4000
2000
0
AUC
*
3 day post crush
PLGA-PEG-30 min
(4-AP)-PLGA-PEG-30 min

FIG. 5G

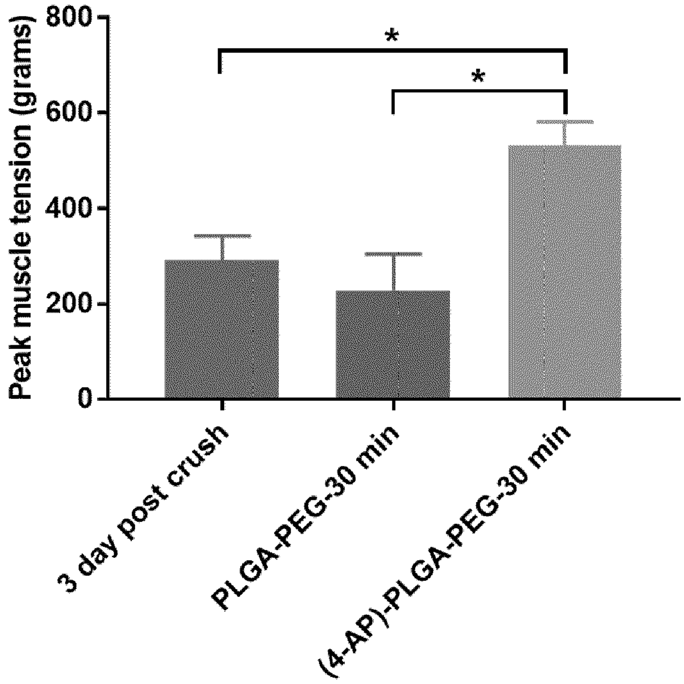
800
600
400
200
0
Peak muscle tension (grams)
*
*
3 day post crush
PLGA-PEG-30 min
(4-AP)-PLGA-PEG-30 min

FIG. 5H

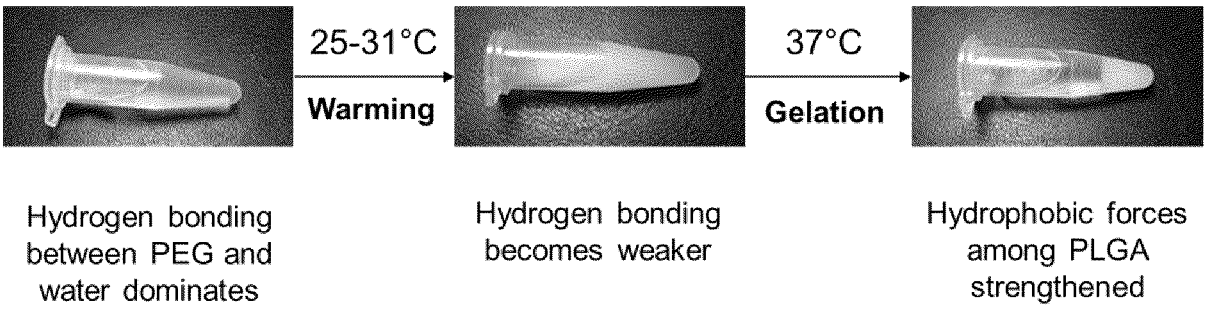
25-31°C
Warming
37°C
Gelation
Hydrogen bonding between PEG and water dominates
Hydrogen bonding becomes weaker
Hydrophobic forces among PLGA strengthened

FIG. 6

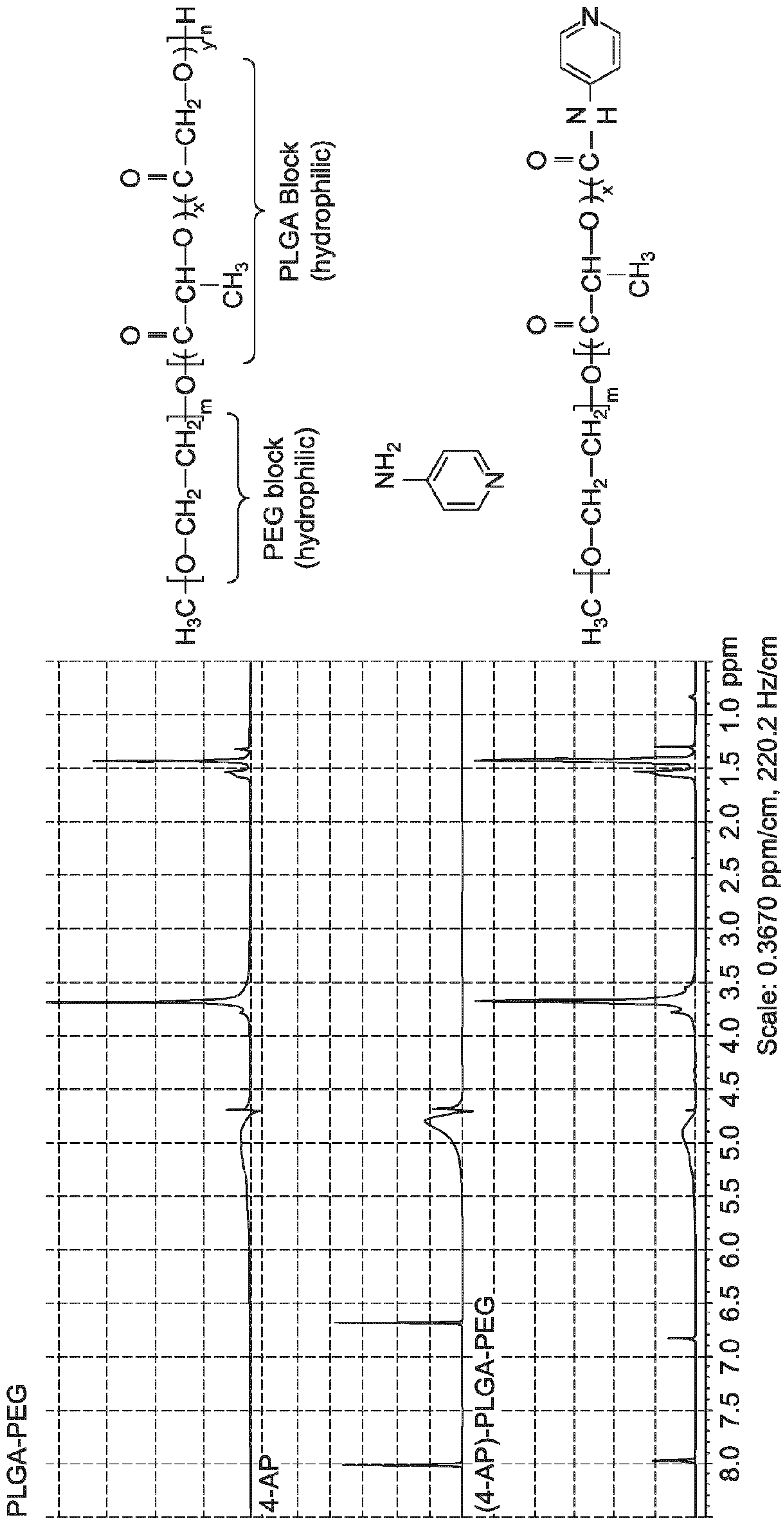
PEG block
(hydrophilic)
PLGA Block
(hydrophilic)
PLGA-PEG
4-AP
(4-AP)-PLGA-PEG
8.0 7.5 7.0 6.5 6.0 5.5 5.0 4.5 4.0 3.5 3.0 2.5 2.0 1.5 1.0 ppm
Scale: 0.3670 ppm/cm, 220.2 Hz/cm

FIG. 7

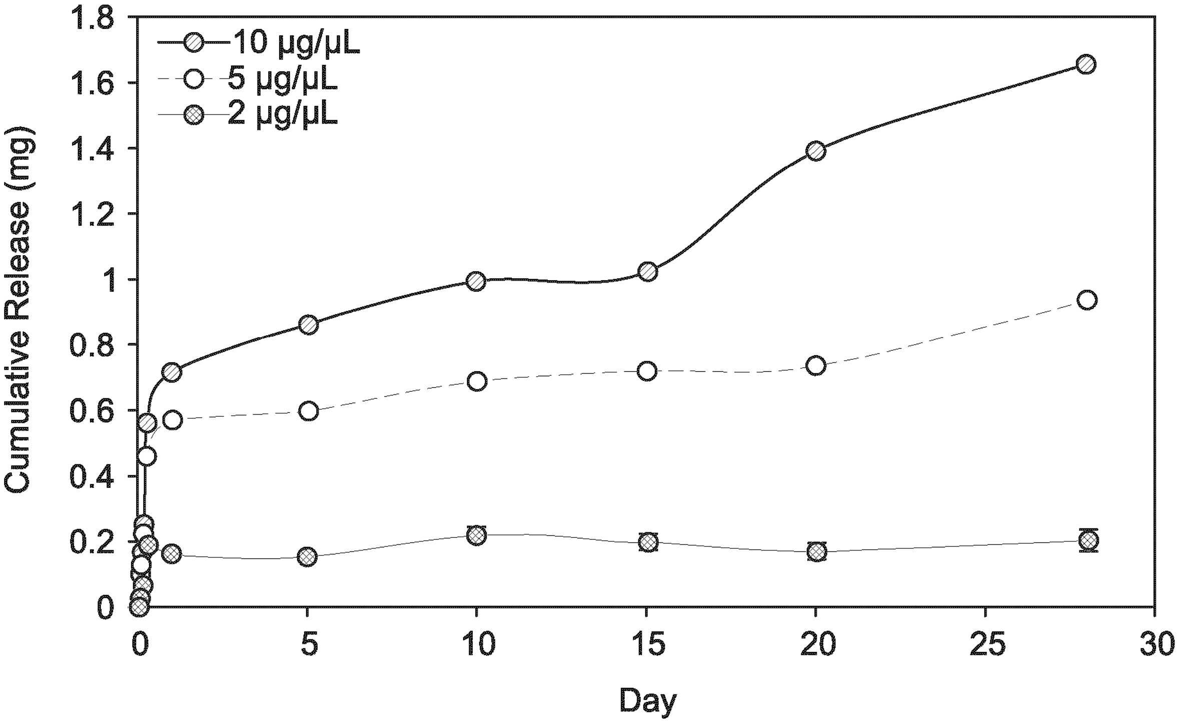
10 µg/µL
5 µg/µL
2 µg/µL
Cumulative Release (mg)
1.8
1.6
1.4
1.2
1
0.8
0.6
0.4
0.2
0
0
5
10
15
20
25
30
Day

FIG. 8

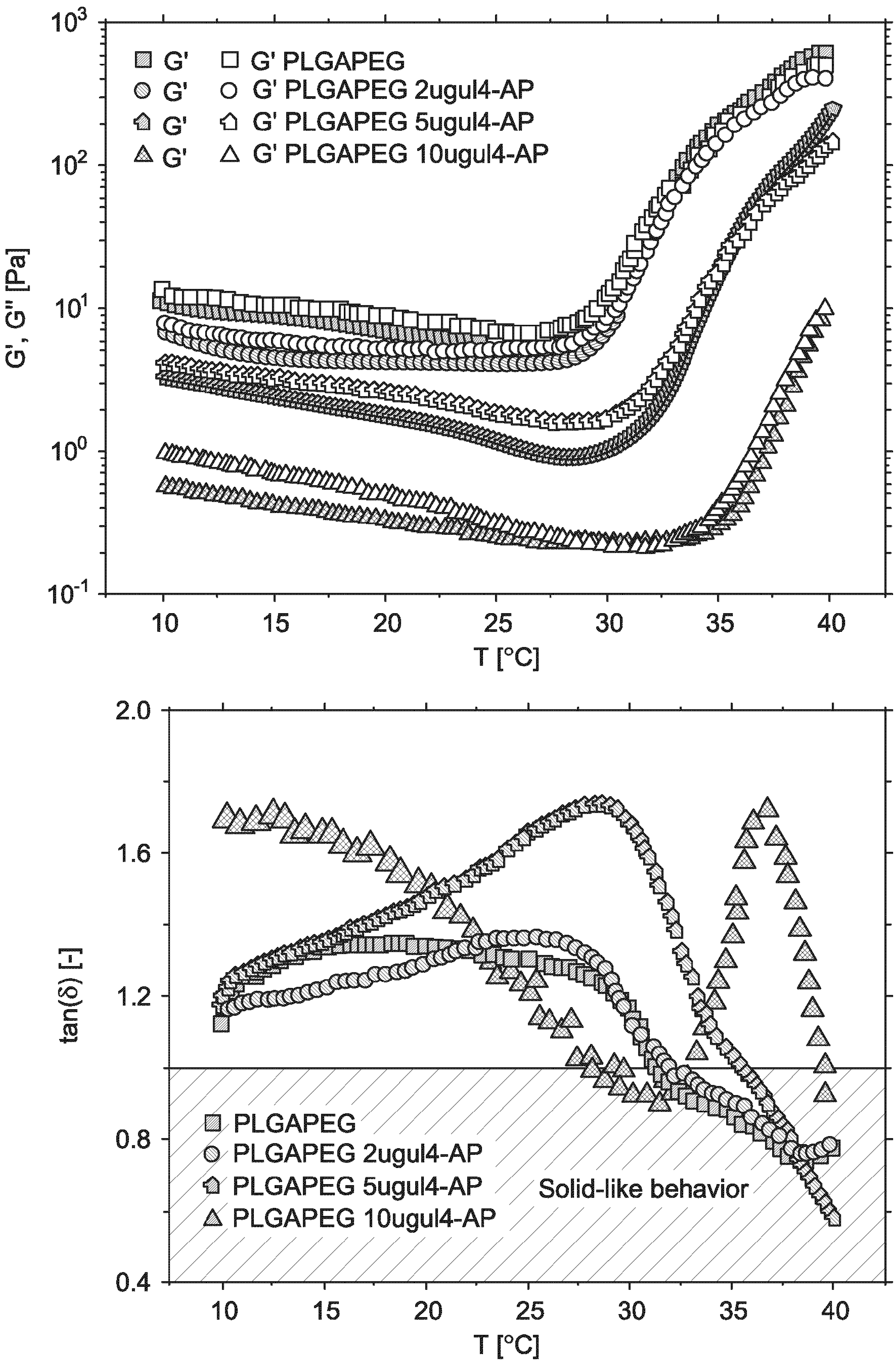
G'
G' PLGAPEG
G'
G' PLGAPEG 2ugul4-AP
G'
G' PLGAPEG 5ugul4-AP
G'
G' PLGAPEG 10ugul4-AP
G', G'' [Pa]
T [°C]
tan(δ) [-]
PLGAPEG
PLGAPEG 2ugul4-AP
PLGAPEG 5ugul4-AP
PLGAPEG 10ugul4-AP
Solid-like behavior
T [°C]

FIG. 9

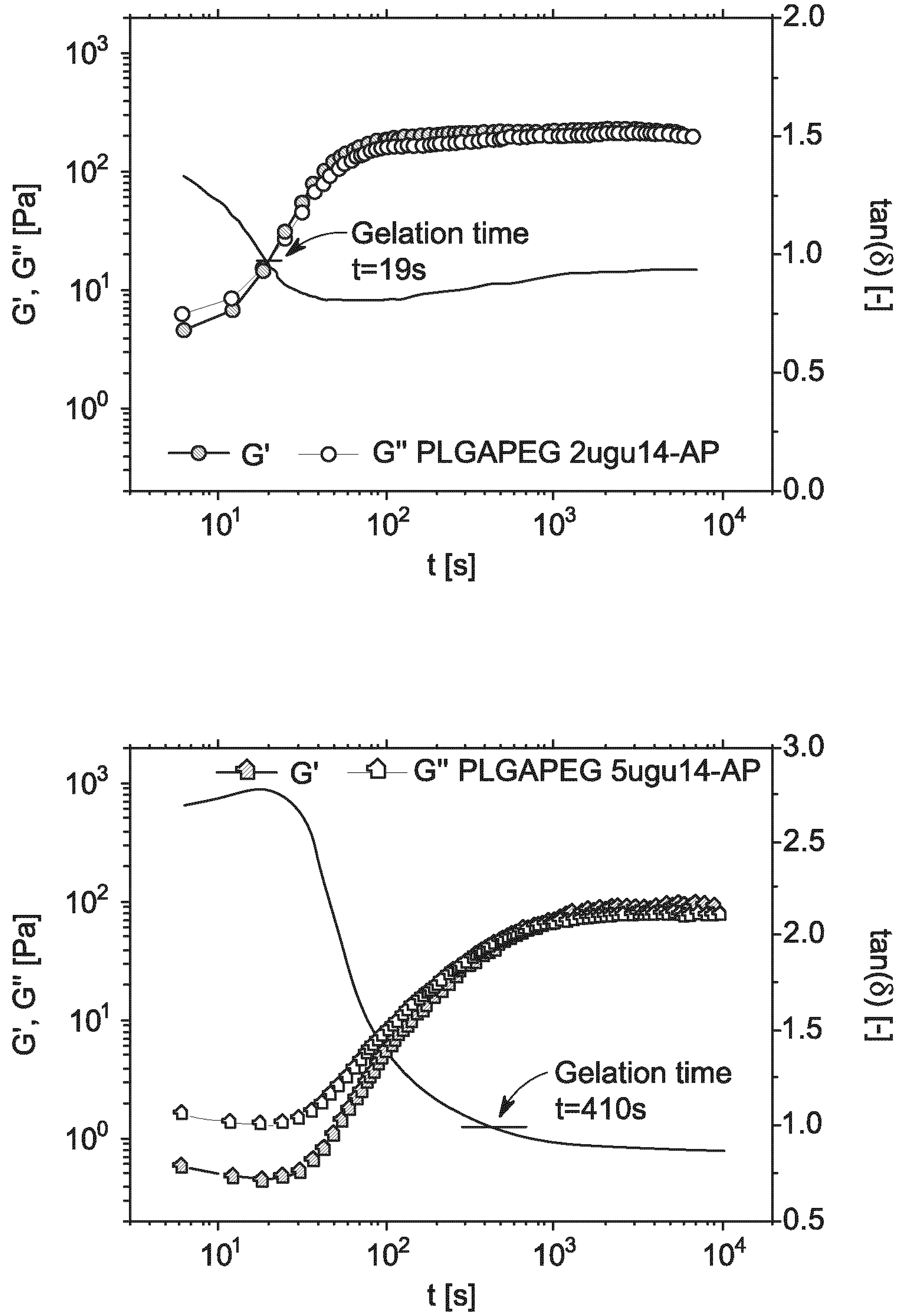
G', G'' [Pa]
tan(δ) [-]
t [s]
Gelation time
t=19s
G' G'' PLGAPEG 2ugu14-AP
Gelation time
t=410s
G' G'' PLGAPEG 5ugu14-AP

FIG. 10

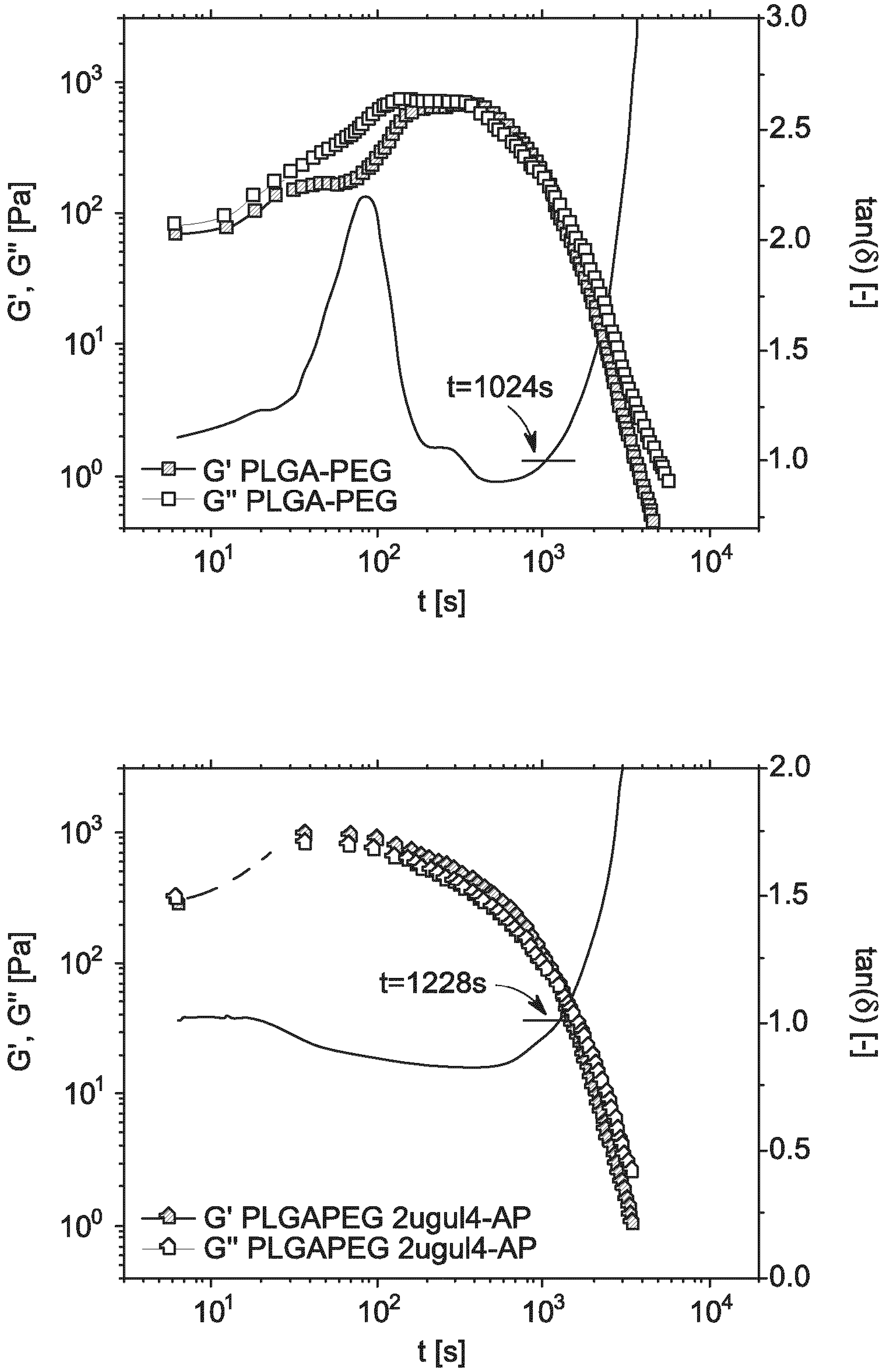
G', G'' [Pa]
tan(δ) [-]
t [s]
t=1024s
G' PLGA-PEG
G'' PLGA-PEG
t=1228s
G' PLGAPEG 2ugul4-AP
G'' PLGAPEG 2ugul4-AP

FIG. 11

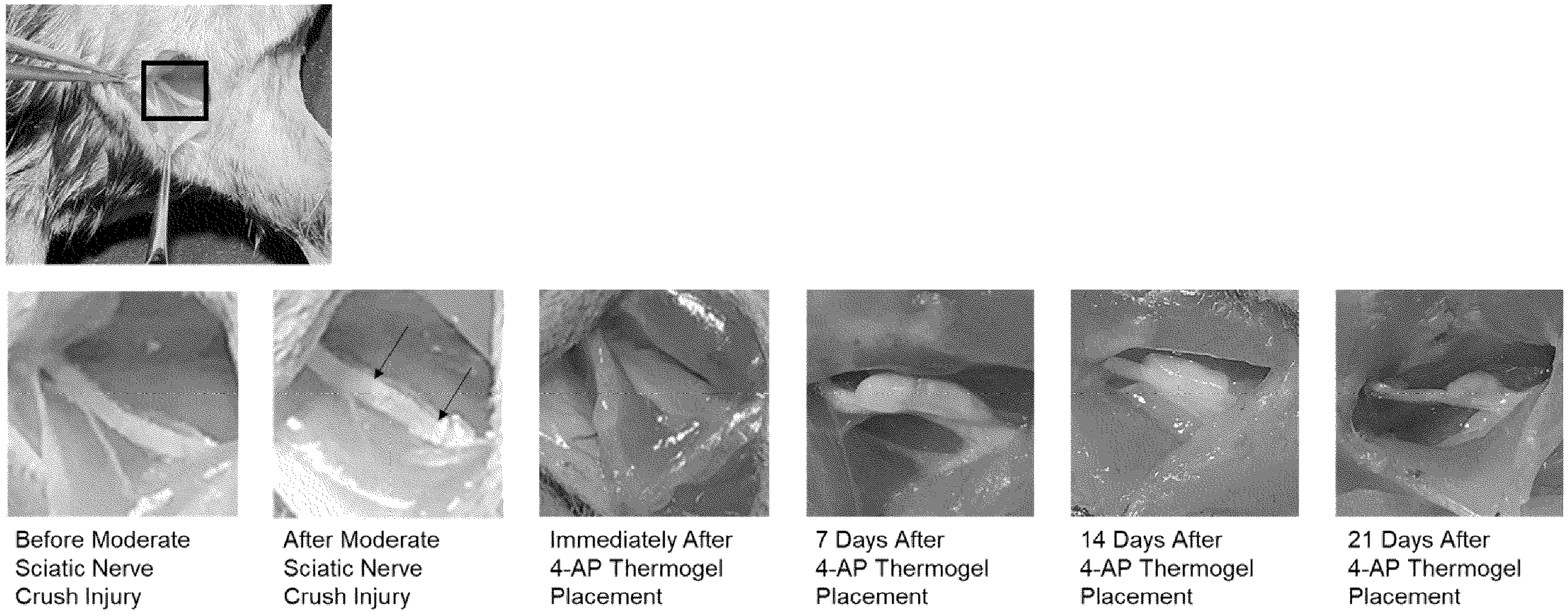
Before Moderate
Sciatic Nerve
Crush Injury
After Moderate
Sciatic Nerve
Crush Injury
Immediately After
4-AP Thermogel
Placement
7 Days After
4-AP Thermogel
Placement
14 Days After
4-AP Thermogel
Placement
21 Days After
4-AP Thermogel
Placement

FIG. 12

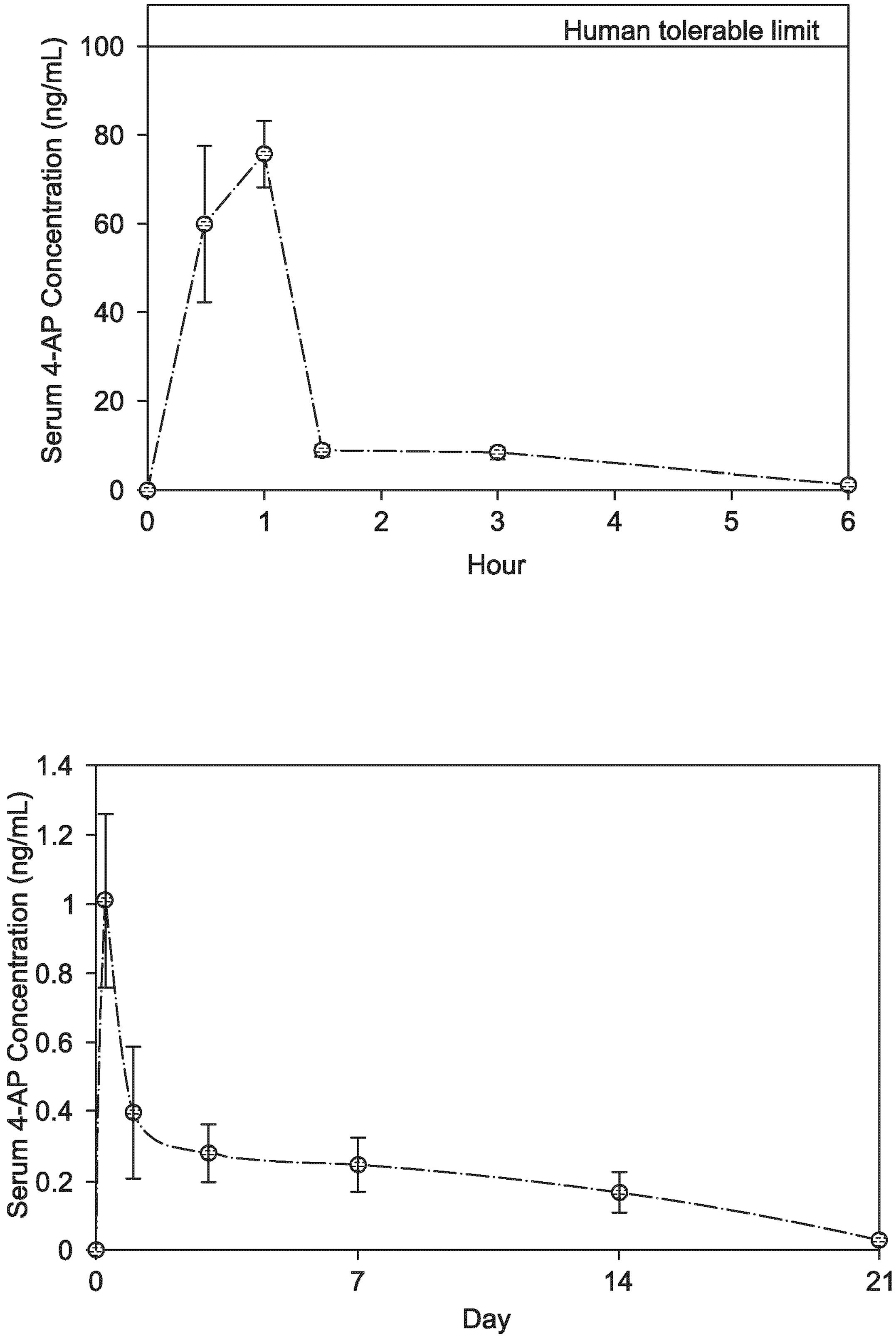
Human tolerable limit
100
80
60
40
20
0
Serum 4-AP Concentration (ng/mL)
0
1
2
3
4
5
6
Hour
1.4
1.2
1
0.8
0.6
0.4
0.2
0
Serum 4-AP Concentration (ng/mL)
0
7
14
21
Day

FIG. 13

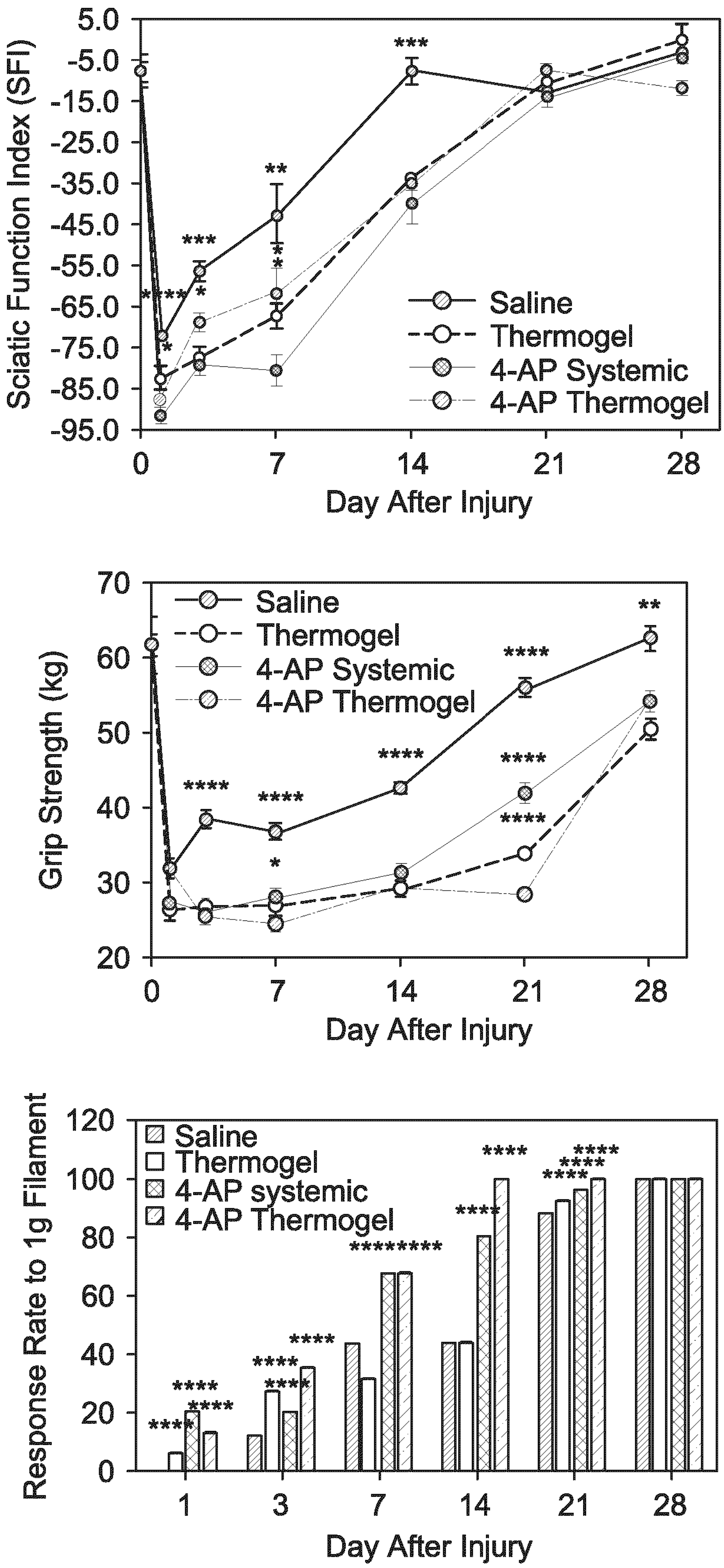
Sciatic Function Index (SFI)
5.0
-5.0
-15.0
-25.0
-35.0
-45.0
-55.0
-65.0
-75.0
-85.0
-95.0
0
7
14
21
28
Day After Injury
Saline
Thermogel
4-AP Systemic
4-AP Thermogel
Grip Strength (kg)
70
60
50
40
30
20
Response Rate to 1g Filament
120
100
80
60
40
20
0
1
3
4-AP systemic

FIG. 14

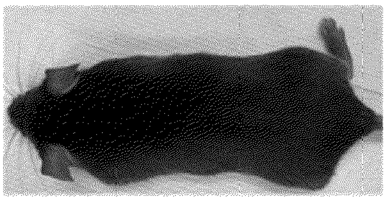

FIG. 15A

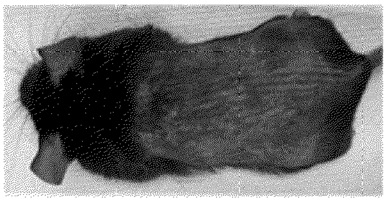

FIG. 15B

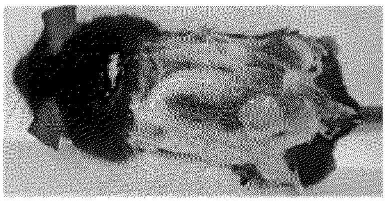

FIG. 15C

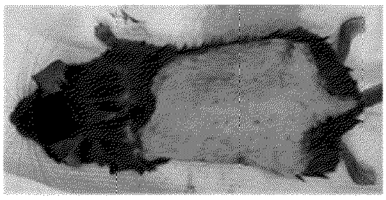

FIG. 15D

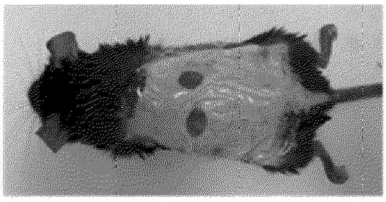

FIG. 15E

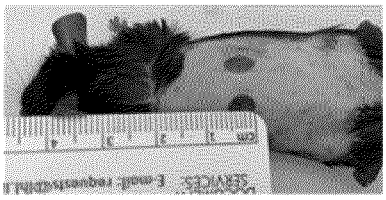

FIG. 15F

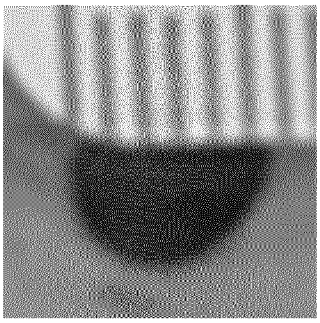

FIG. 16A

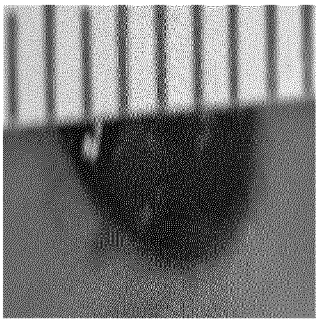

FIG. 16B

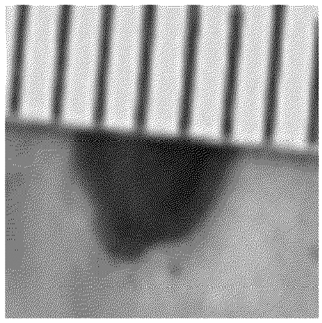

FIG. 16C

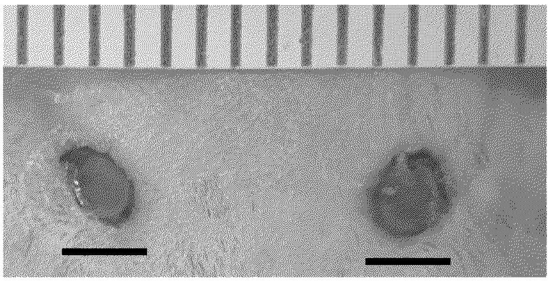

FIG. 17A

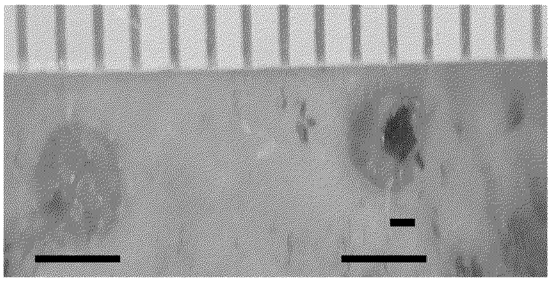

FIG. 17B

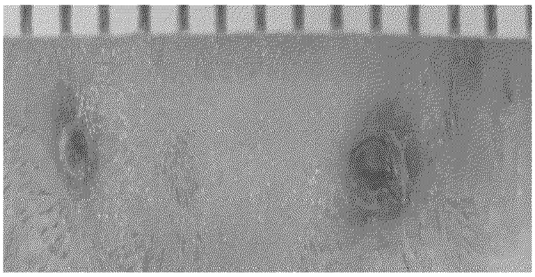

FIG. 18A

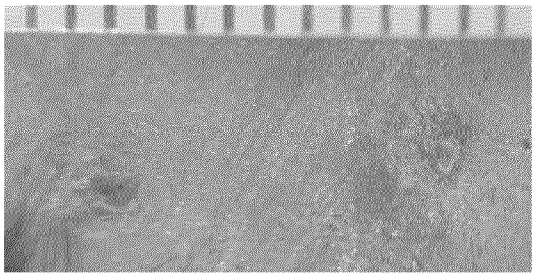

FIG. 18B

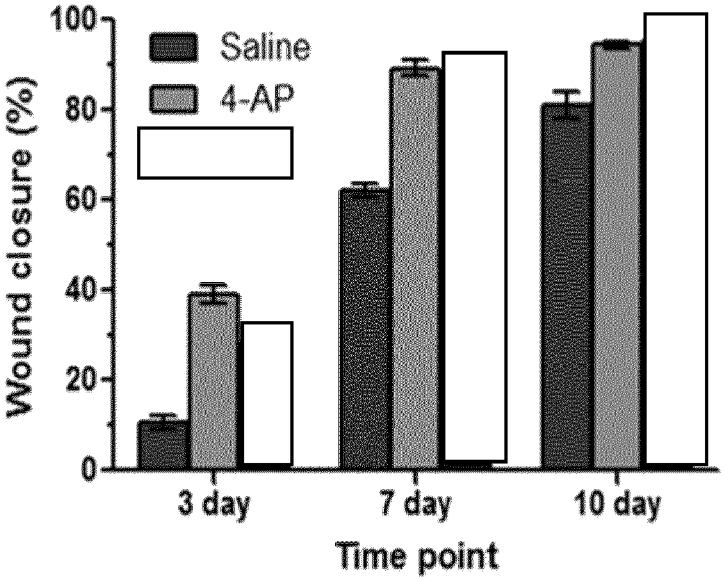

100
80
60
40
20
0
Wound closure (%)
Saline
4-AP
3 day
7 day
10 day
Time point


FIG. 19

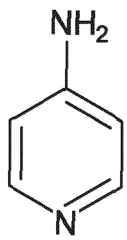

4-aminopyridine (4-AP)

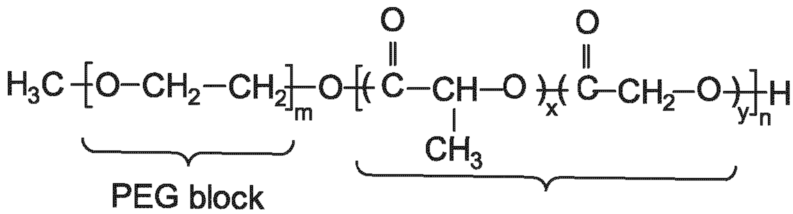

PEG block
(hydrophilic)

PLGA Block
(hydrophobic)

FIG. 20A

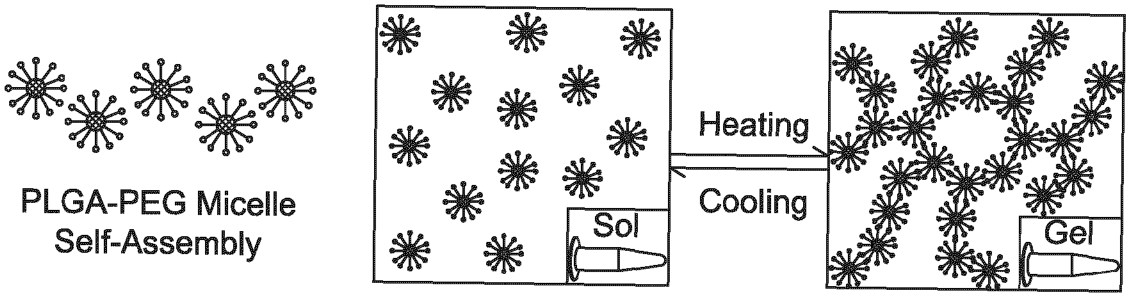


FIG. 20B

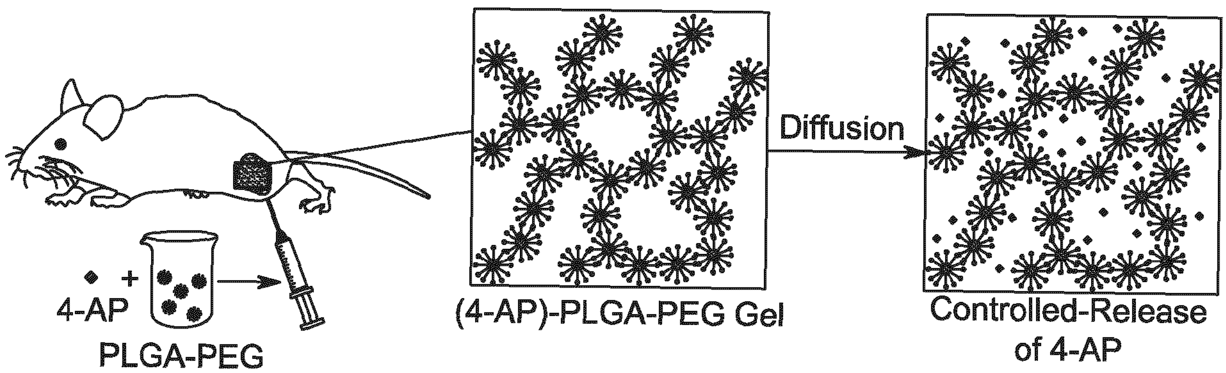


FIG. 20C

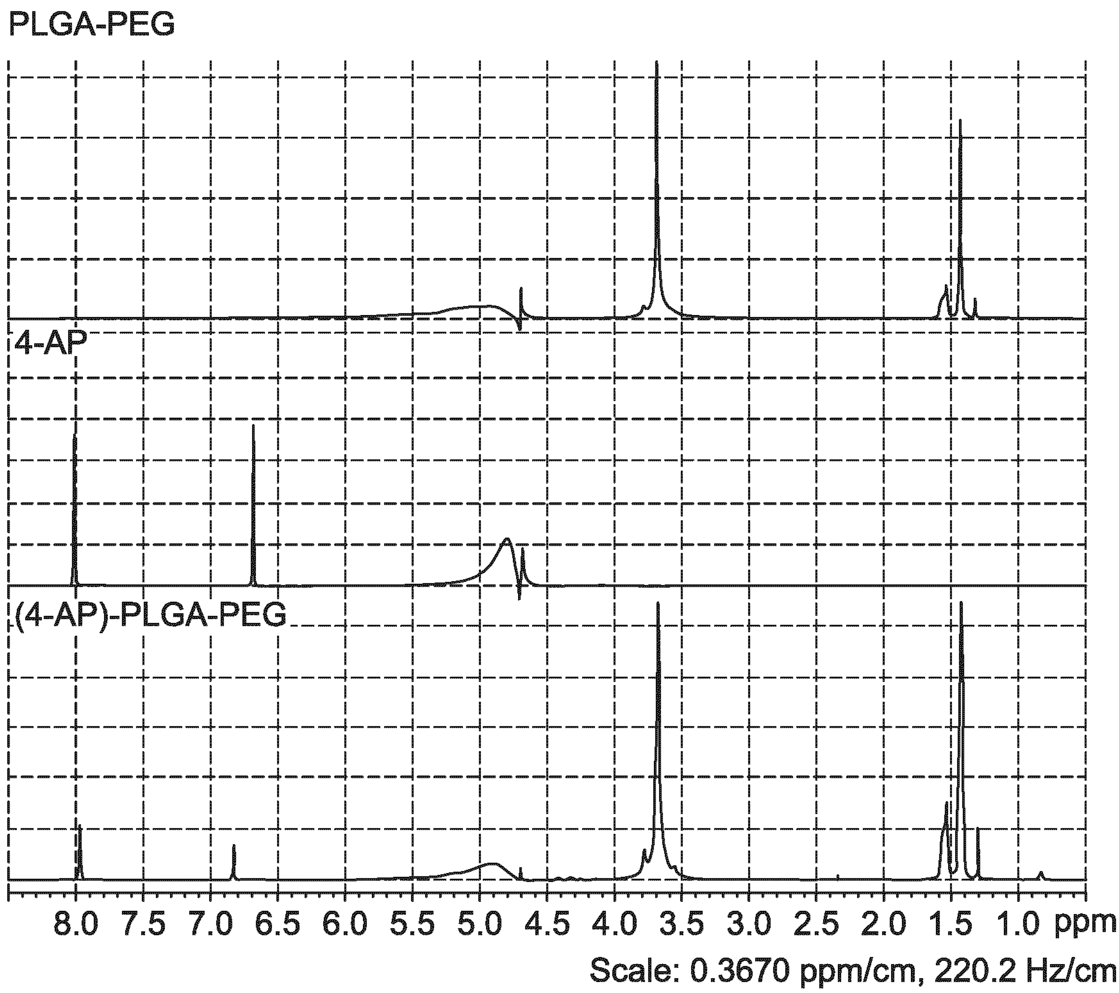
PLGA-PEG
4-AP
(4-AP)-PLGA-PEG
8.0 7.5 7.0 6.5 6.0 5.5 5.0 4.5 4.0 3.5 3.0 2.5 2.0 1.5 1.0 ppm
Scale: 0.3670 ppm/cm, 220.2 Hz/cm

FIG. 21

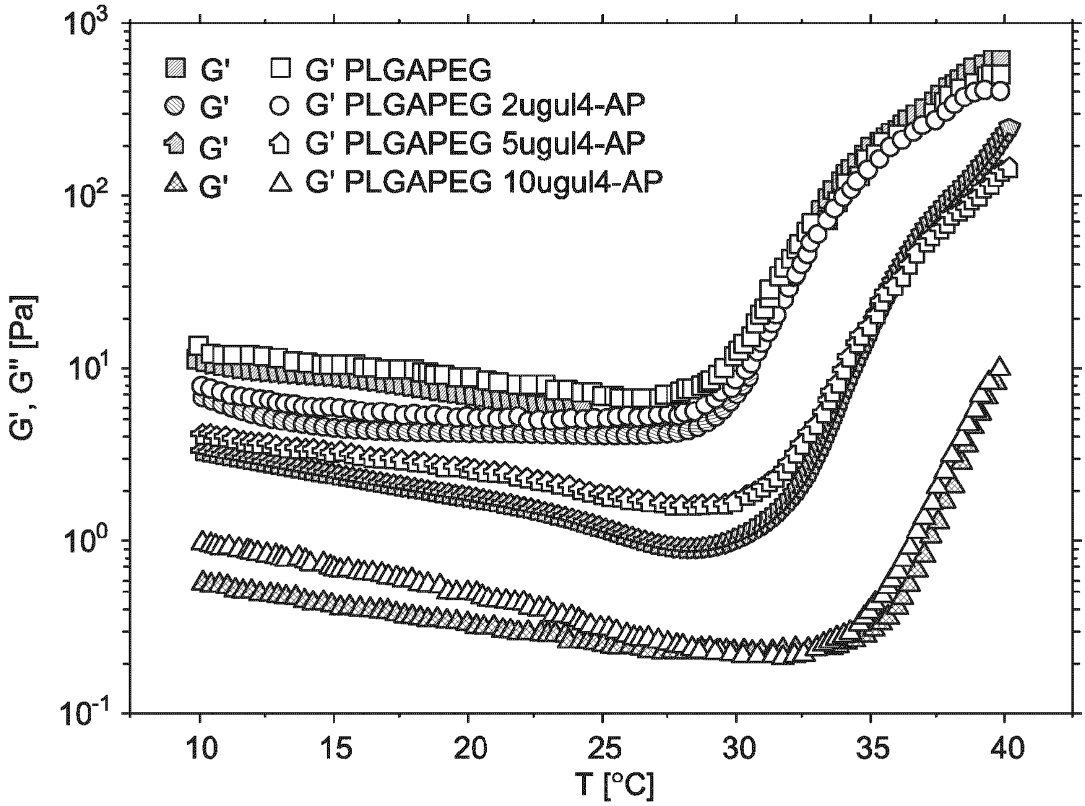


FIG. 22A

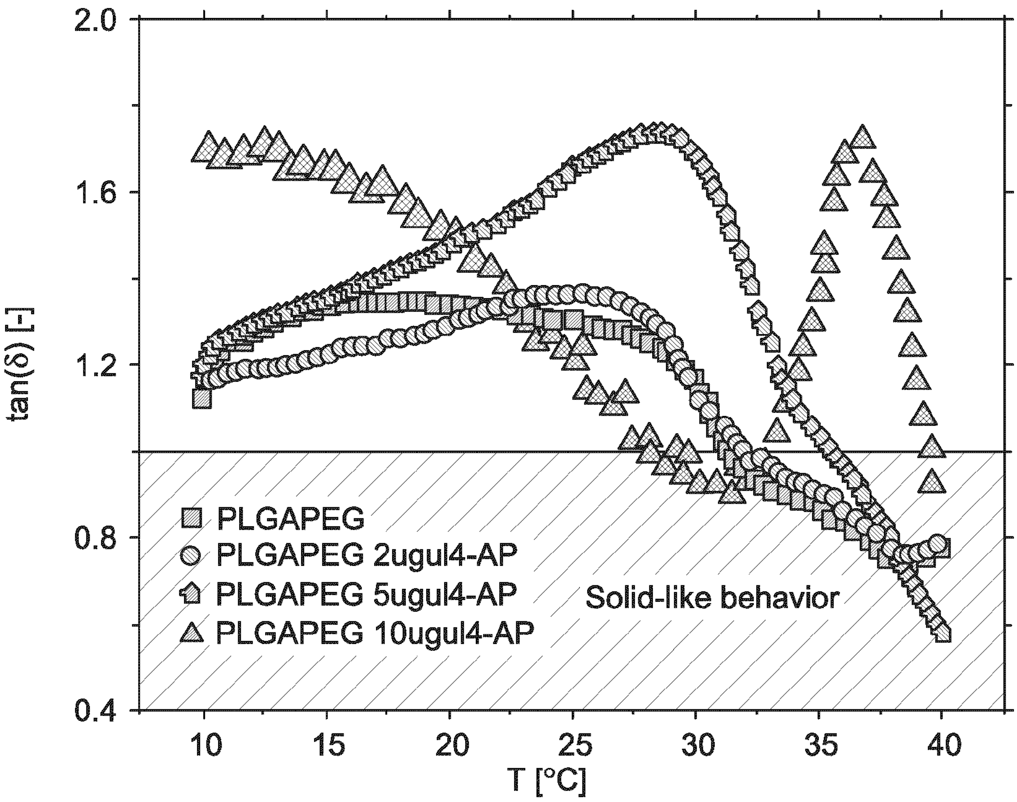


FIG. 22B

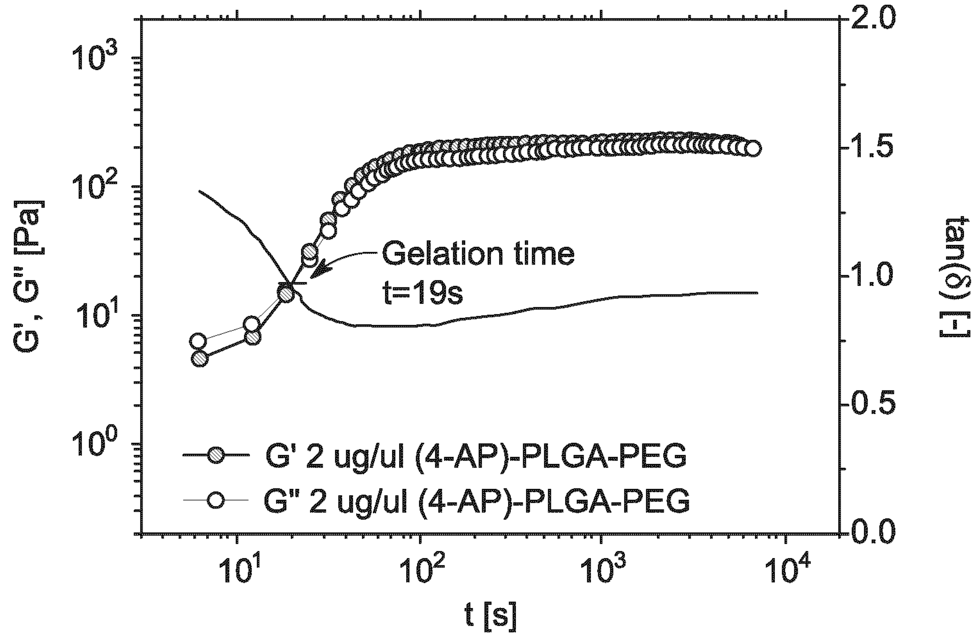


FIG. 22C

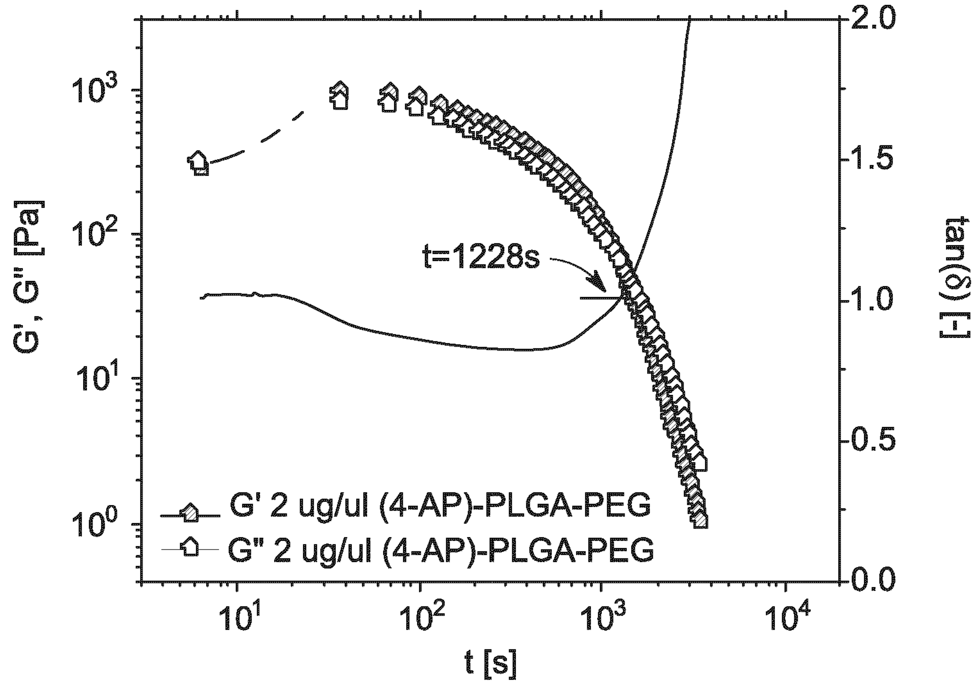


FIG. 22D

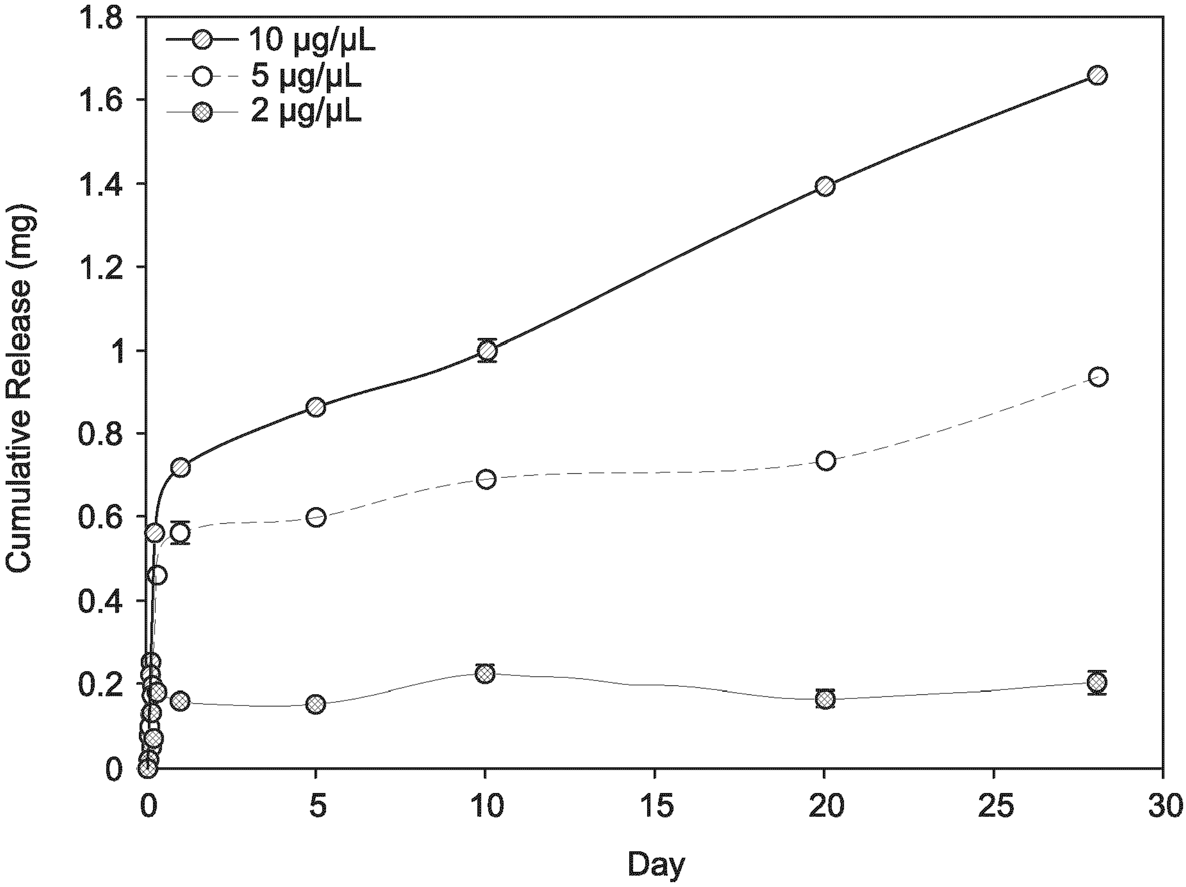

10 µg/µL
5 µg/µL
2 µg/µL
Cumulative Release (mg)
1.8
1.6
1.4
1.2
1
0.8
0.6
0.4
0.2
0
0
5
10
15
20
25
30
Day


FIG. 23

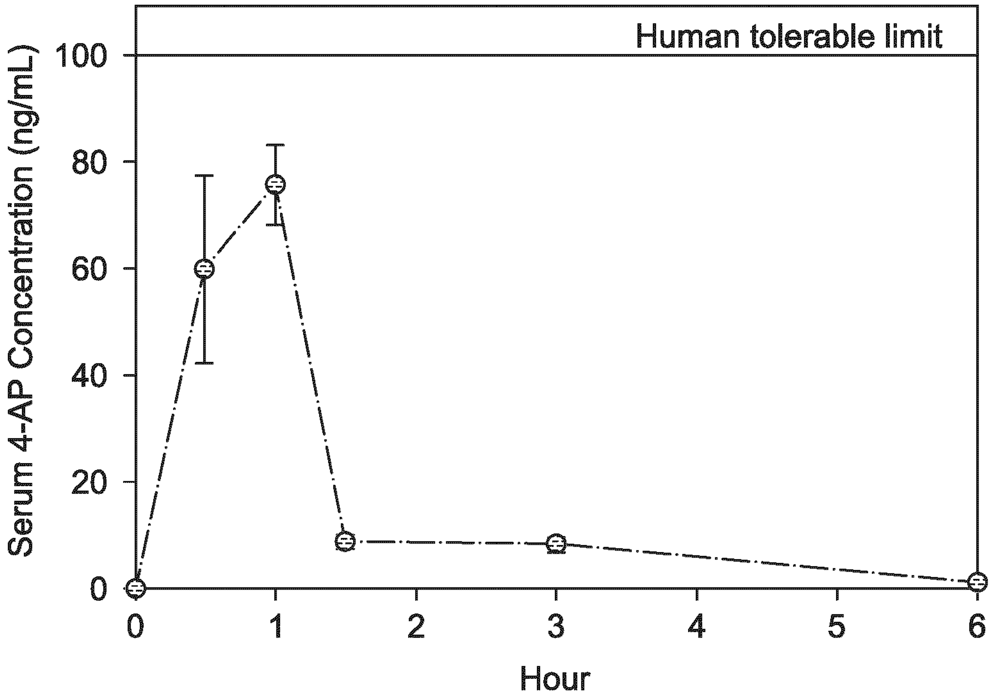


FIG. 24A

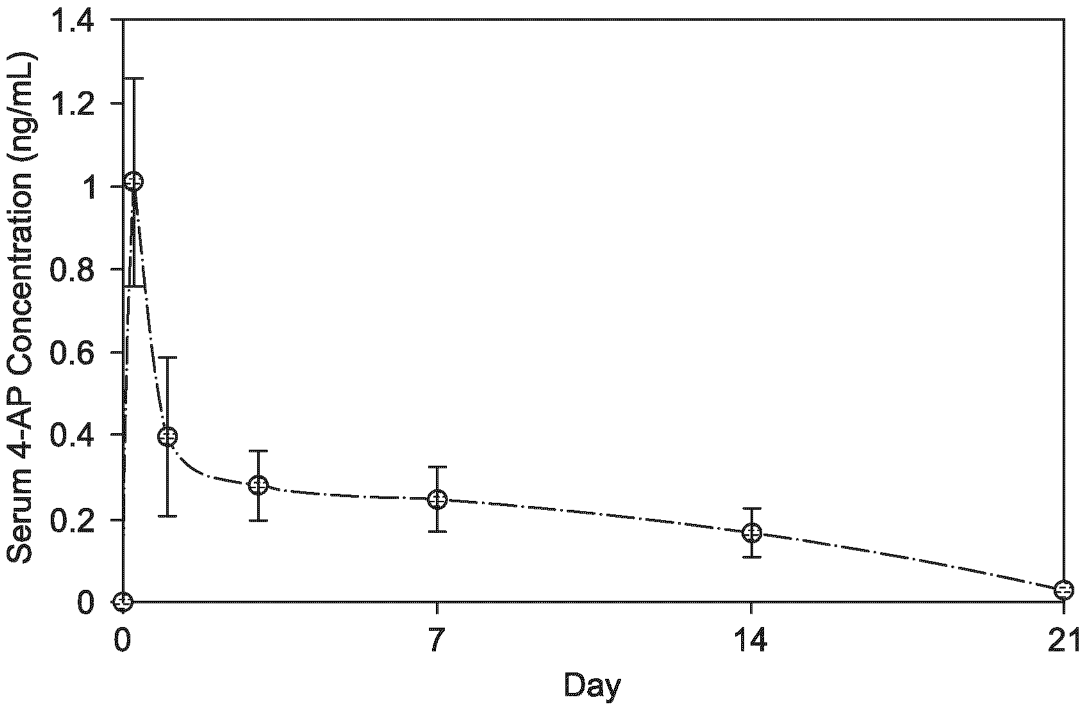


FIG. 24B

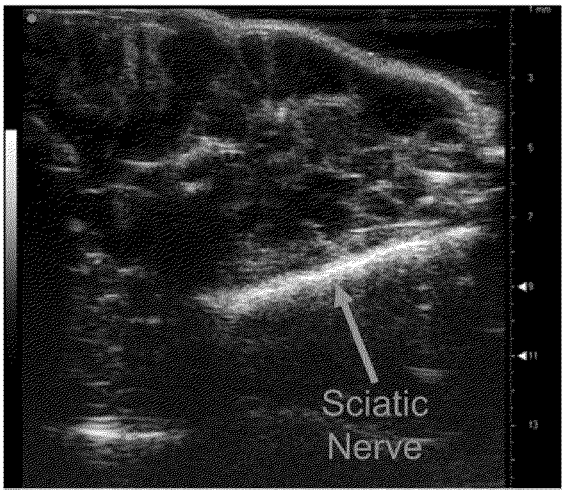


FIG. 25A

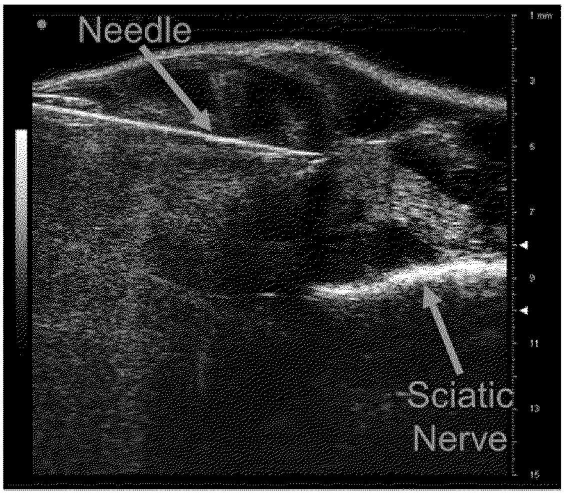


FIG. 25B

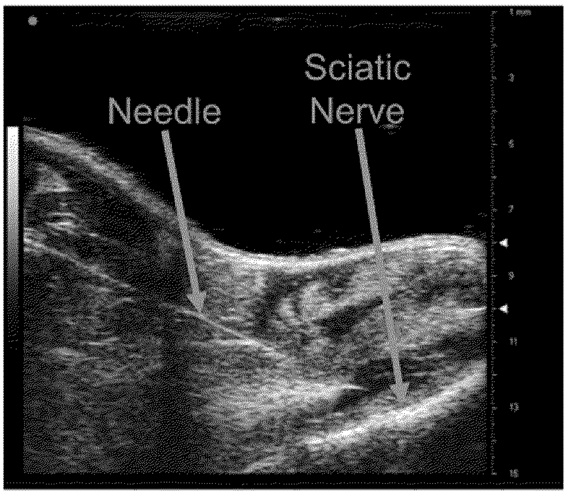


FIG. 25C

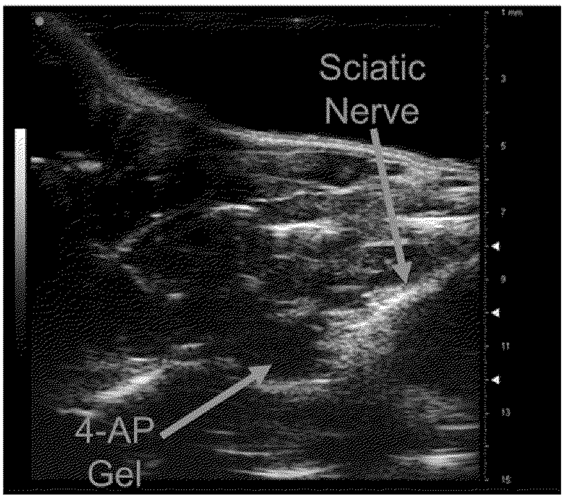


FIG. 25D

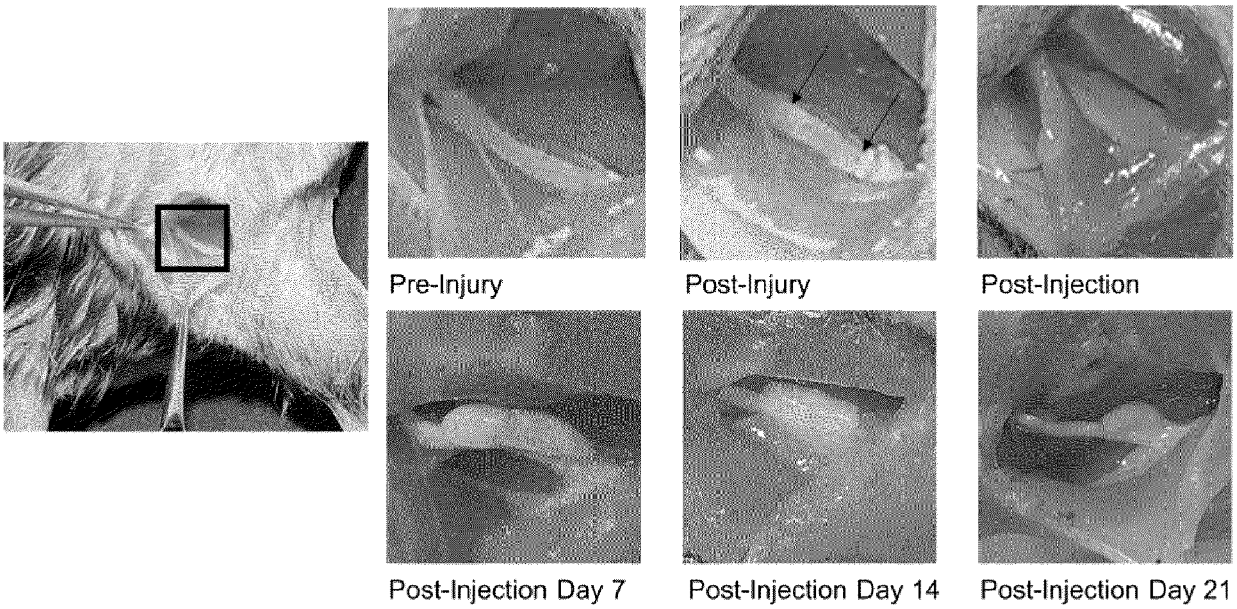
Pre-Injury
Post-Injury
Post-Injection
Post-Injection Day 7
Post-Injection Day 14
Post-Injection Day 21

FIG. 26

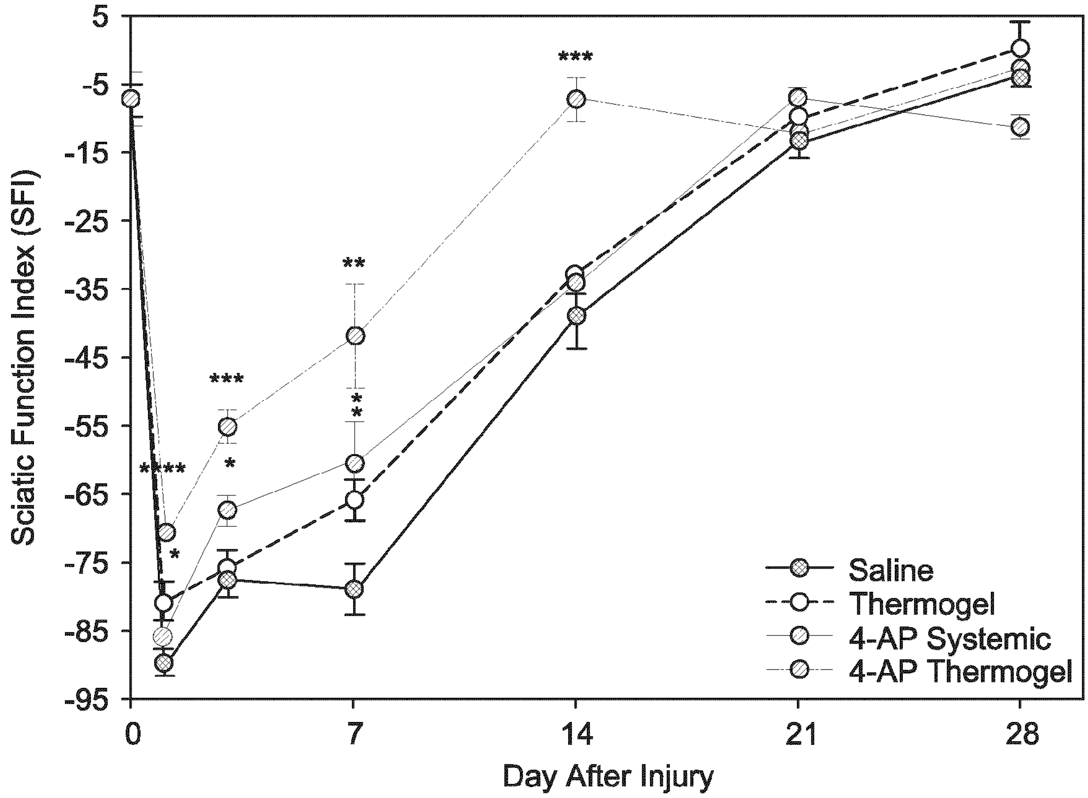


FIG. 27A

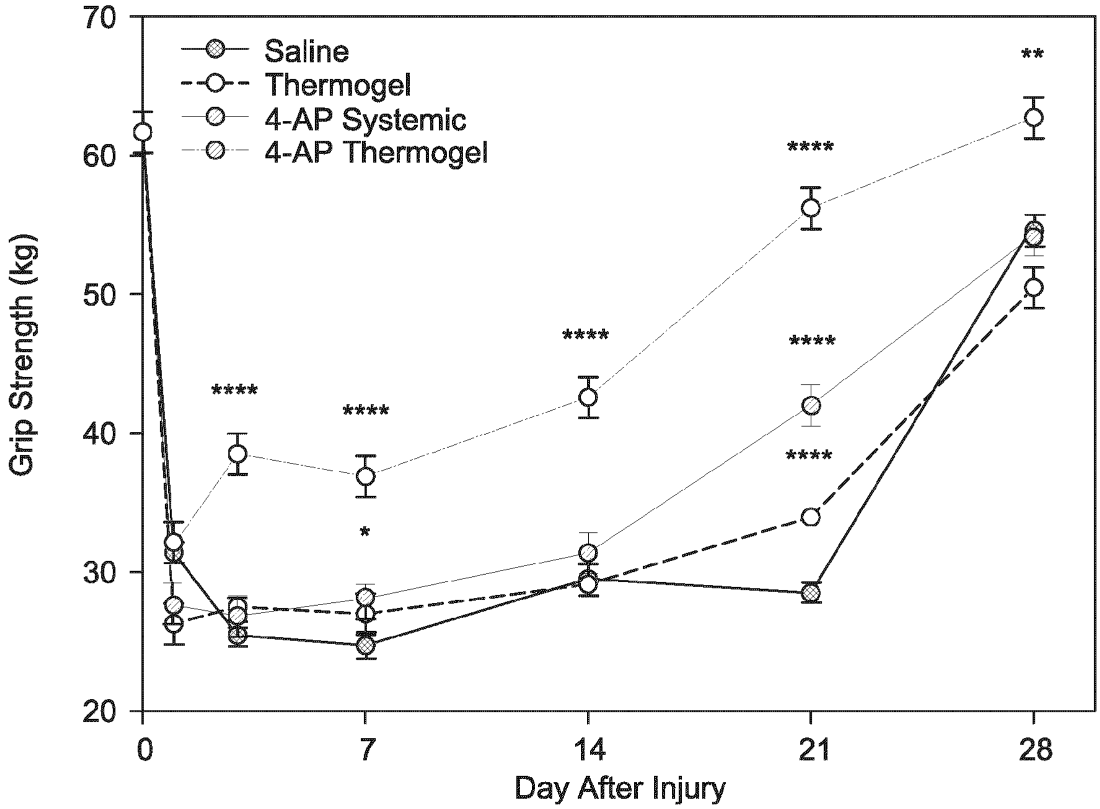


FIG. 27B

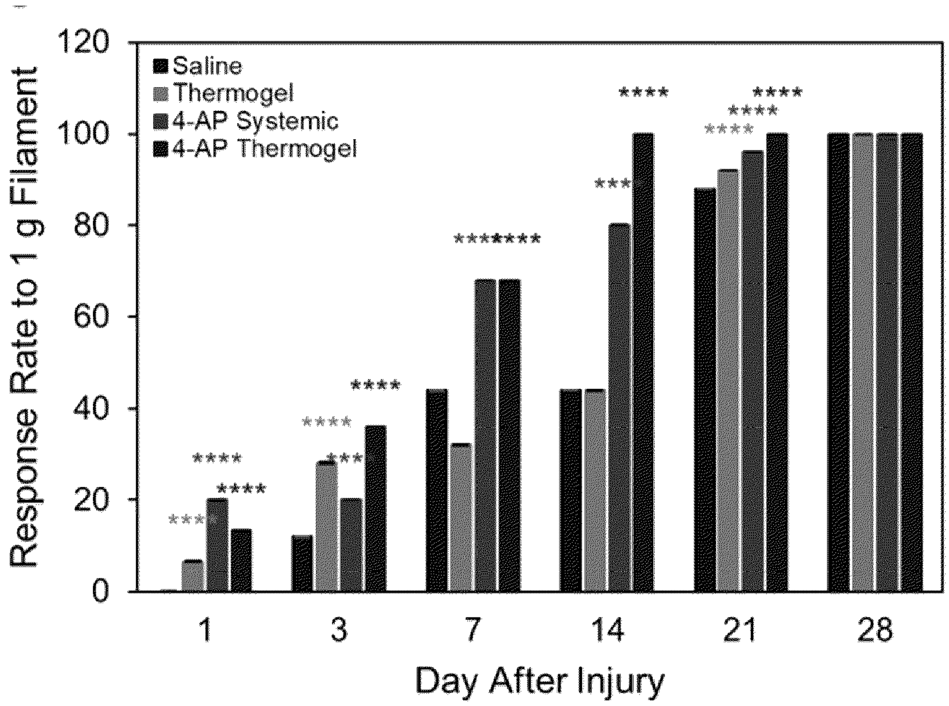

120
100
80
60
40
20
0
Response Rate to 1 g Filament
Saline
Thermogel
4-AP Systemic
4-AP Thermogel
****
1
3
7
14
21
28
Day After Injury


FIG. 27C

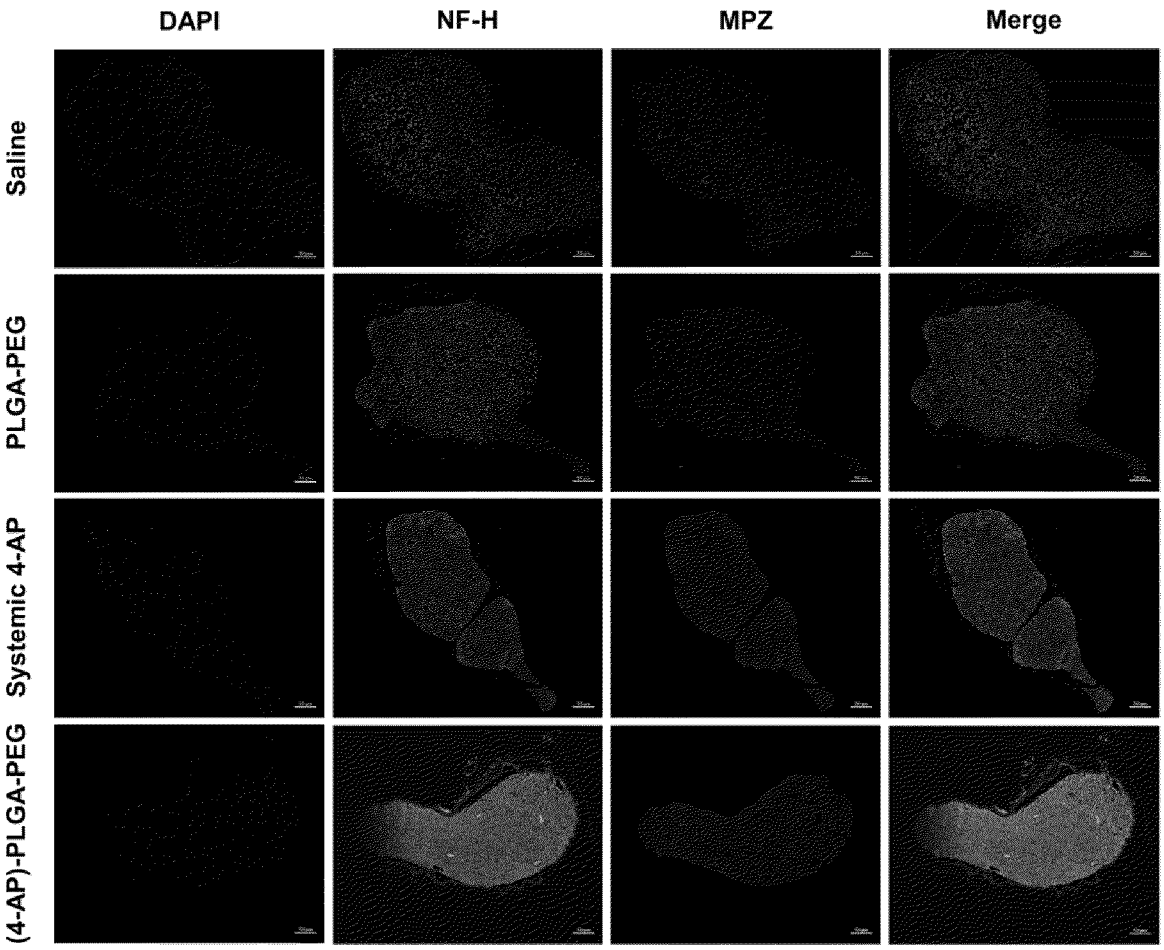


FIG. 28A

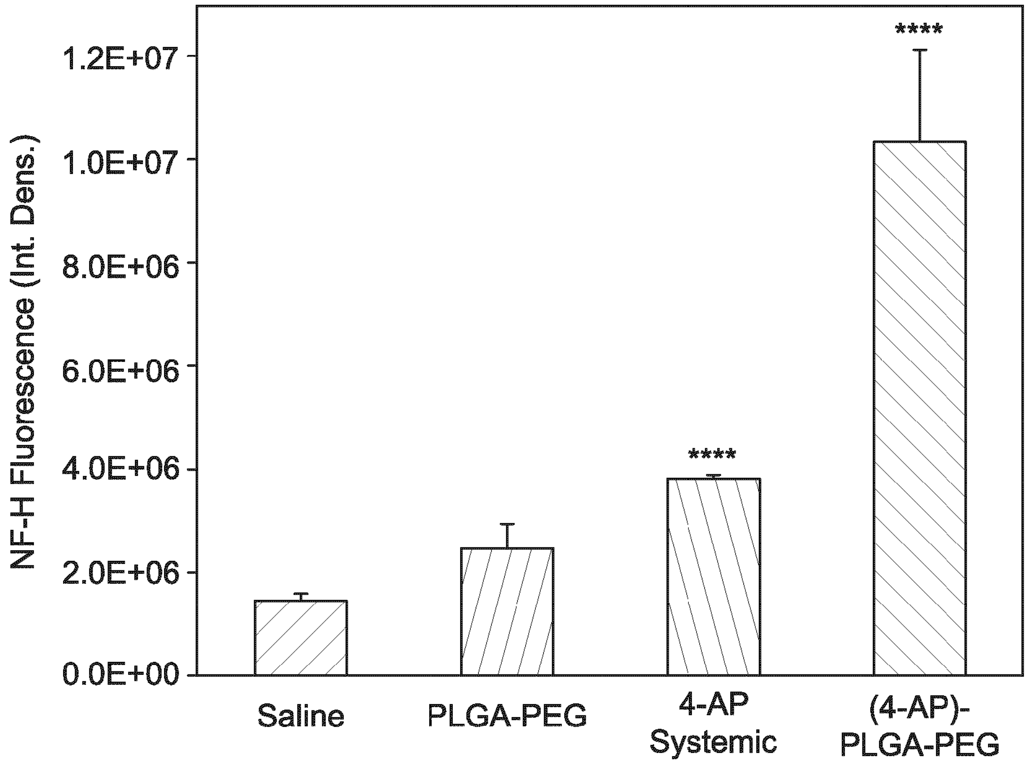


FIG. 28B

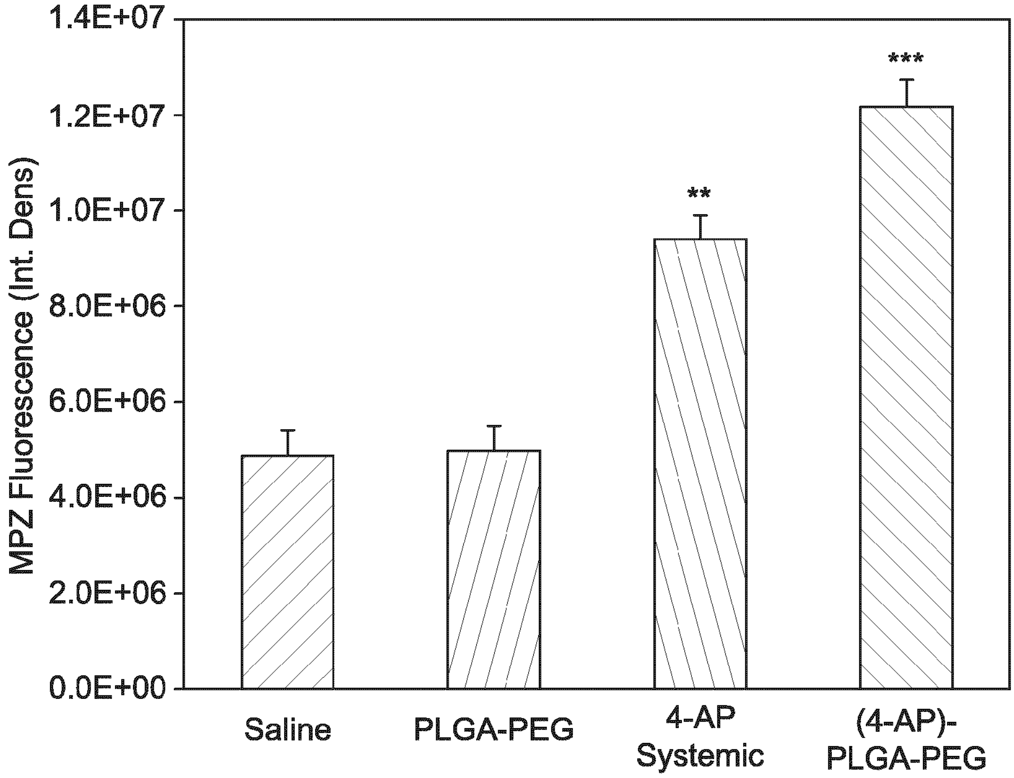


FIG. 28C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62963992
- US 63005890
- US 20180271847
- US 9993429 B

### Non-patent literature cited in the description


- **ELFAR et al.** *J. Bone Joint Surg. Am.,* 2008, vol. 90, 1644-1653
- **TSENG et al.** *, EMBO Mol. Med.,* 2016, vol. 8, 1409-1420